(19) Europäisches Patentamt
European Patent Office
Office européen des brevets

(11) EP 2 596 835 B1

(12) **EUROPÄISCHE PATENTSCHRIFT**

(45) Veröffentlichungstag und Bekanntmachung des Hinweises auf die Patenterteilung:
**30.04.2014 Patentblatt 2014/18**

(51) Int Cl.:
***A61N 5/10*** $^{(2006.01)}$

(21) Anmeldenummer: **12188753.3**

(22) Anmeldetag: **17.10.2012**

(54) **Verfahren zum Berechnen von lokalen Teilstrahlendosen in einer Strahlentherapieanlage und entsprechende Strahlentherapieanlage.**

Method for operating a radiotherapy facility and radiotherapy facility

Procédé de fonctionnement d’une installation de radiothérapie et installation de radiothérapie

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.11.2011 DE 102011086930**

(43) Veröffentlichungstag der Anmeldung:
**29.05.2013 Patentblatt 2013/22**

(73) Patentinhaber: **Siemens Aktiengesellschaft 80333 München (DE)**

(72) Erfinder: **Scholz, Christian 68775 Ketsch (DE)**

(56) Entgegenhaltungen:
**US-A1- 2008 240 348**

- **WU Q., ET AL.: "Dynamic splitting of large intensity-modulated fields", PHYS. MED. BIOL., Bd. 45, 2000, Seiten 1731-1740, XP003000995,**
- **E.B. HUG ET AL.: INT. J. RADIAT. ONKOL. BIOL. PHYS, Bd. 47, 2000, Seiten 979-984, XP055057225,**
- **HARISH K. MALHOTRA ET AL: "Technical and dosimetric considerations in IMRT treatment planning for large target volumes", JOURNAL OF APPLIED CLINICAL MEDICAL PHYSICS, Bd. 6, Nr. 4, 21. November 2005 (2005-11-21), Seiten 77-87, XP055057228, ISSN: 1526-9914, DOI: 10.1120/jacmp.2026.25360**
- **DANNY Z CHEN ET AL: "A New Algorithm for a Field Splitting Problem in Intensity-Modulated Radiation Therapy", ALGORITHMICA, SPRINGER-VERLAG, NE, Bd. 61, Nr. 3, 16. Juli 2010 (2010-07-16), Seiten 656-673, XP019939398, ISSN: 1432-0541, DOI: 10.1007/S00453-010-9429-6**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 596 835 B1

## Beschreibung

Strahlentherapie, welche auch als Radiotherapie (RT) bezeichnet wird, ist ein therapeutischer Ansatz auf der Grundlage einer ionisierenden Strahlung, um beispielsweise Krebs zu behandeln. Die Strahlentherapie kann jedoch auch zur Behandlung anderer Erkrankungen verwendet werden. Bei der Strahlentherapie wird versucht, eine ausreichende therapeutische Strahlendosis zu einem erkrankten Gewebe zuzuführen, während umgebendes gesundes Gewebe ausgespart wird. Der therapeutische Effekt beruht auf einer ionisierenden Wirkung der Strahlung auf erkranktes Gewebe.

Die Dosisapplikation mittels Bestrahlung erfolgt zum Beispiel mittels intensitätsmodulierter Photonentherapie (IMRT), Protonen oder Kohlenstoffionen. Voraussetzung dafür ist eine auf dreidimensionaler Diagnostik, zum Beispiel Computertomographie oder Magnetresonanztomographie, beruhende Bestrahlungsplanung. In der Bestrahlungsplanung werden die Bestrahlungsparameter so festgelegt, dass im Tumorvolumen bzw. Zielvolumen die nötige Gesamtstrahlendosis appliziert und umliegendes, gesundes Gewebe bestmöglich geschont wird. Allgemein kann die Anwendung von Bestrahlung mittels Photonenstrahlen in einem sogenannten "Step-and-Shoot"-Ansatz erfolgen, indem der Strahl ausgeschaltet wird, während sich Lamellen eines Lamellenkollimators sich bewegen, um durch die Bewegung einen nachfolgenden Strahlabschnitt zu definieren. Ein weiterer Ansatz ist die sogenannte dynamische Technik, in der der Strahl angeschaltet bleibt, während sich die Lamellen bewegen. Für Protonen oder Schwerionen werden entweder sog. "scanned-beam"-Techniken verwendet, bei denen der Strahl über ein Zielvolumen gerastert wird, oder passive Feldformungstechniken, d.h. es erfolgt der Einsatz von Kompensatoren und Energie- bzw. Reichweiten-Modulatoren.

Die Planung einer Bestrahlung eines Zielvolumens, z. B. eines Tumors, erfolgt im Rahmen eines Behandlungsplans. Ein Behandlungsplan umfasst z. B. die Anzahl und Orientierung verschiedener Strahlen, welche zur Applikation einer bestimmten verschriebenen Dosis, d.h. einer Gesamtstrahlendosis, in dem Zielvolumen benötigt werden. In jedem Volumenelement kann ein Strahl dann eine Teilstrahlendosis applizieren. Es existieren verschiedene Techniken, um die applizierte Dosis für jedes Volumenelement zu variieren.

Aus dosimetrischen Gründen kann es notwendig sein, mehr als einen Strahl zu verwenden, um die Gesamtstrahlendosis, die z. B. in Bezug auf die Art des Tumors berechnet wurde, zu applizieren. Zum Beispiel kann das Erstellen eines Behandlungsplans die Definition von gefährdeten Organen (OAR) umfassen, welche von der Applikation einer Strahlendosis verschont werden sollten, da die OARs besonders empfindlich auf Bestrahlung reagieren und große unerwünschte Nebenwirkungen im Falle einer Bestrahlung möglich sind. Die Bestrahlung kann auch die Bestrahlung von mehreren Zielvolumen umfassen. Für jedes Zielvolumen erfolgt dann in einem iterativen Optimierungsprozess die Berechnung von optimalen Bestrahlungsparametern (Anzahl, Art, Intensitätsverteilung und Energie verschiedener Strahlen, etc.). Einzelne dieser Parameter können auch vorgegeben werden, um die Komplexität des Problems zu reduzieren.

In bestimmten klinischen Fällen werden weiterführende Mittel benötigt, um die Behandlung des Patienten in einer Strahlentherapie zu ermöglichen. Ein solcher Fall ist, dass die Projektion eines Zielvolumens in die Richtung eines Strahls das maximale Bestrahlungsgesichtsfeld eines Strahls überschreitet. Zum Beispiel kann das maximale Gesichtsfeld eines Strahls durch hardwareseitige Limitationen definiert sein. Dann ist es notwendig, einen Strahl in zwei oder mehrere Strahlen aufzuspalten. Dieser Vorgang wird typischerweise als Strahlspaltung ("Beam-Split") bezeichnet, wobei das Spalten insbesondere dadurch erreicht werden kann, dass eine Spalt-Ebene ("Split-Plane") definiert wird, um zu unterscheiden, auf welcher Seite der Spalt-Ebene ein bestimmter Strahl eine Teilstrahlendosis appliziert. In der Literatur sind Verfahren bekannt, welche das Aufspalten eines Strahls erlauben. Zum Beispiel offenbart Q. Wu et al. in Phys. Med. Biol. 45 (2000) 1731 - 1740 ein Verfahren zum Aufspalten von Strahlen.

Ein weiteres klinisches Gebiet, in dem weiterführende Mittel zur Behandlung des Patienten mittels Strahlentherapie benötigt werden, ist die dosimetrische Optimierung. Wenn ein dosimetrischer Vorteil erwartet wird, kann es vorteilhaft sein, das Zielvolumen in unterschiedliche Untervolumen zu spalten. Hierbei muss die Spaltung nicht aufgrund von Hardware-Limitationen erfolgen, wie das in Bezug auf die Spalt-Ebenen oben beschrieben wurde. Die verschiedenen Untervolumen werden dann von unterschiedlichen Strahlen bzw. jeweils mehreren Strahlen bestrahlt. Insbesondere in der gescannten Partikel-Strahlentherapie ("Scan particle beam therapy") wird eine solche Aufspaltung des Zielvolumens in Untervolumen verwendet. Dieses Anwendungsgebiet wird Strahlen-Patch ("BeamPatch") genannt und die entsprechenden Steuerungsebenen werden Patch-Ebenen genannt. Zum Beispiel offenbart E.B. Hug et al. in Int. J. Radiat. Onkol. Biol. Phys. 47 (2000) 979 ein Verfahren der gescannten Protonenstrahltechnik, in dem ein einzelner Strahl mittels gescannter Magnete in die erwünschte Position bewegt wird. Es werden Vorteile gezeigt, die aus der Verwendung zweier durch eine Patch-Ebene miteinander in Beziehung gesetzten Protonenstrahlen für eine Klasse von entsprechenden klinischen Fällen erwachsen. Insbesondere wird gezeigt, dass dadurch ein OAR von der Applikation einer Dosis ausgenommen werden kann.

Die oben genannten Verfahren haben den Nachteil, dass es entweder möglich ist, Spalt-Ebenen anzuwenden oder Patch-Ebenen anzuwenden.

Deshalb ist es wünschenswert, ein Verfahren zum Berechnen von lokalen Teilstrahlendosen in einer Strahlentherapieanlage bereitzustellen, welches eine erhöhte Flexibilität in der Behandlungsplanung aufweist.

Diese Aufgabe wird von den Merkmalen der unabhängigen Patentansprüche gelöst. In den abhängigen Ansprüchen sind bevorzugte Ausführungsformen beschrieben.

Gemäß einem Aspekt wird Verfahren zum Berechnen von lokalen Teilstrahlendosen in einer Strahlentherapieanlage zum Applizieren einer Gesamtstrahlendosis in einem Zielvolumen mit mehreren Strahlen bereitgestellt, wobei das Verfahren umfasst: Festlegen mindestens einer ersten Steuerungsebene zur Steuerung der Dosierung der Strahlen, wobei jede der mindestens einen ersten Steuerungsebene das Zielvolumen in zwei Untervolumen aufteilt, jeweils für die Vorderseite und die Rückseite jeder der mindestens einen ersten Steuerungsebene: Zuordnen mindestens eines Strahls, und für jede erste Steuerungsebene: Festlegen einer Untervolumen-Gesamtstrahlendosis für jeweils jedes der zwei Untervolumen als ein Bruchteil der Gesamtstrahlendosis. Das Verfahren umfasst weiterhin das Festlegen mindestens einer zweiten Steuerungsebene zur Steuerung der Positionierung der Strahlen, wobei jede der mindestens einen zweiten Steuerungsebene das Zielvolumen in zwei Untervolumen aufteilt, Zuordnen mindestens eines Strahls zu jeder der mindestens einen zweiten Steuerungsebene. Hierbei wird ein zu einer zweiten Steuerungsebene zugeordneter Strahl durch die jeweilige zweite Steuerungsebene derart zweigeteilt wird, dass die lokale Teilstrahlendosis der so erhaltenen zwei Strahlen jeweils in unterschiedlichen, durch die jeweilige zweite Steuerungsebene definierten, Untervolumen ungleich Null ist. Das Verfahren umfasst weiterhin für mindestens eine Seite einer ersten Steuerungsebene: isoliertes Berechnen der entsprechenden lokalen Teilstrahlendosen aller dieser ersten Steuerungsebene zugeordneten Strahlen, sodass die Summe der lokalen Teilstrahlendosen derjenigen Strahlen, die der dem jeweiligen Untervolumen zugewandten Seite der jeweiligen ersten Steuerungsebene zugeordnet sind, die jeweilige Untervolumen-Gesamtstrahlendosis ergibt und die Summe der lokalen Teilstrahlendosen der restlichen dieser ersten Steuerungsebene zugeordneten Strahlen die Differenz zwischen der jeweiligen Untervolumen-Gesamtstrahlendosis und Gesamtstrahlendosis ergibt.

Der Begriff Gesamtstrahlendosis bezeichnet hierbei diejenige Dosis, z. B. in Einheiten von Gray oder Sievert definiert, welche innerhalb des Zielvolumens durch einen oder mehrere Strahlen appliziert wird. Die Gesamtstrahlendosis sollte möglichst nah an einer verschriebenen Dosis liegen, die in einem Bestrahlungsplan festgelegt wird. Zum Beispiel ist es möglich, eine bestimmte Gesamtstrahlendosis nicht nur durch einen Strahl innerhalb des Zielvolumens zu applizieren, sondern durch mehrere Strahlen. Dies kann dosimetrische Vorteile beinhalten, wie später näher erläutert werden wird.

Das Zielvolumen kann z. B. einen Tumor bezeichnen, welcher durch gezieltes Applizieren einer Gesamtstrahlendosis zerstört werden soll. Hierzu werden Strahlen verwendet, wobei Strahlen z. B. Röntgenstrahlen, Elektronenstrahlen, Schwerionenstrahlen oder auch Protonenstrahlen bezeichnen können. Verschiedene Strahlenarten sind möglich, wobei dem Fachmann jeweils Vor- und Nachteile der verschiedenen Strahlenarten bekannt sind. Insbesondere weisen unterschiedliche Strahlen unterschiedliche Abhängigkeiten der Eindringtiefe von der Energie auf sowie verschiedene biologische Wirksamkeiten.

Insbesondere existiert ein Zusammenhang zwischen der Energie der Partikel (Photonen, Elektronen, Protonen, Schwerionen) und der applizierten Gesamtstrahlendosis. Weiterhin ist ein Strahl typischerweise durch einen Quellpunkt ("Source spot") und einen Zielpunkt ("Target spot") gekennzeichnet. Der Quellpunkt wird typischerweise durch eine Strahlerzeugungsvorrichtung der Strahlentherapieanlage definiert. Durch die Vorgabe einer Strahlrichtung einer Strahlenenergie kann die Strahlrichtung definiert werden. Die Mitte eines Gesichtsfelds eines Strahls wird als Isozentrum bezeichnet.

Der Begriff Steuerungsebene bezeichnet geometrische Ebenen, welche von einem Benutzer, z. B. in ein dreidimensionales Bild eines Körperbereiches eines Patienten zur Steuerung der Applizierung von Strahlendosis z.B. innerhalb eines dreidimensionalen Abbilds des Zielbereichs positioniert werden können. Es ist möglich, dass Steuerungsebenen eine Dicke aufweisen. Die Verwendung von Steuerungsebenen erleichtert es dem Benutzer, bestimmte Eingabeparameter in Bezug auf die Strahlen festzulegen. Zum Beispiel können Parameter, die sich auf die Teilstrahlendosen beziehen, einfach festgelegt werden. Weiterhin können Parameter, welche sich auf die Positionierung der Strahlen beziehen, einfach festgelegt werden. Es sollte klar sein, dass die Verwendung von Patch-Ebenen und Spalt-Ebenen nicht notwendigerweise die Orientierung des Strahls, der mit einer solchen Ebene verknüpft ist, vorgibt. Zum Beispiel können mehrere Strahlen mit einer Seite einer Patch-Ebene verknüpft sein, welche unterschiedliche Orientierungen, das bedeutet unterschiedliche Strahlursprünge und Strahlrichtungen, aufweisen.

Erste und zweite Steuerungsebenen werden z. B. von einem Benutzer verwendet, um sowohl die Dosierung als auch die Positionierung der Strahlung zu steuern. Erste Steuerungsebenen werden auch als Patch-Ebenen in der Strahlentherapie bezeichnet. Erste Steuerungsebenen oder Patch-Ebenen können z. B. einen dosimetrischen Vorteil bewirken. Zum Beispiel kann durch die Verwendung von Patch-Ebenen erreicht werden, dass die Gesamtstrahlendosis durch zwei Strahlen innerhalb des Zielvolumens appliziert wird, wobei die zwei Strahlen unterschiedliche Quellpunkte haben, so dass die beiden Strahlen auf unterschiedlichen Wegen zu dem Tumor gelangen. Dies hat den Vorteil, dass umliegendes Gewebe oder OARs, welche möglichst geringe Strahlendosen erhalten sollen, geschont werden.

Hierbei teilt eine Patch-Ebene das Zielvolumen in zwei Untervolumina. Ein Strahl kann dann, z. B. von einem Benutzer, mit einer Seite der Patch-Ebene verknüpft werden. Es ist möglich, dass zwei Strahlen, die jeweils mit der Vorderseite oder der Rückseite einer Patch-Ebene verknüpft sind, dasselbe Isozentrum oder denselben Strahlursprung aufweisen. Der Benutzer kann gleichzeitig einen Bruchteil der Gesamtstrahlendosis festlegen, welche dieser Strahl in dem Untervolumen applizieren soll, das der Seite, mit der der Strahl verknüpft ist, zugewandt ist. Zum Beispiel kann der Bruchteil 70% betragen, was bedeutet, dass der mit der entsprechenden Seite der Patch-Ebene verknüpfte Strahl, in diesem Untervolumen 70% der Gesamtstrahlendosis appliziert. Sind mehrere Strahlen mit der gleichen Seite einer Patch-Ebene verknüpft, so teilen sich diese Strahlen den gegebenen Bruchteil der Gesamtstrahlendosis, das bedeutet, dass die Summe aller Teilstrahlendosen dem vorgegebenen Bruchteil der Gesamtstrahlendosis bzw. die Untervolumen-Gesamtstrahlendosis ergibt.

Die Untervolumen-Gesamtstrahlendosis kann kleiner sein als die Gesamtstrahlendosis. Nichtsdestotrotz ist es notwendig, dass an jedem Punkt innerhalb des Zielvolumens, auch innerhalb eines Untervolumens, die Gesamtstrahlendosis appliziert wird. Die Differenz zwischen der Gesamtstrahlendosis und der Untervolumen-Gesamtstrahlendosis wird hierbei von denjenigen Strahlen innerhalb eines Untervolumens appliziert, die mit der entgegengesetzten Seite der Patch-Ebene verknüpft sind. In dem oben genannten numerischen Beispiel, bei dem die Untervolumen-Gesamtstrahlendosis einen Bruchteil von 70% der Gesamtstrahlendosis beträgt, wären dies 30% der Gesamtstrahlendosis.

Weiterhin können zweite Steuerungsebenen dann verwendet bzw. derart festgelegt werden, dass Strahlen, welche eine Teilstrahlendosis in einem Zielvolumen applizieren, welches größer ist als das dem Strahl maximal zugängliche Volumen, durch eine zweite Steuerungsebene zweigeteilt werden. Solche Ebenen werden auch als Spalt-Ebenen oder "Split-Planes" bezeichnet. Zum Beispiel kann die maximale Größe eines Querschnitts durch ein Zielvolumen, welches von einem Strahl abgetastet werden kann, begrenzt sein. Dies ist typischerweise für Photonenstrahlen der Fall, wenn Lamellenkollimatoren verwendet werden, welche ein begrenztes Gesichtsfeld aufweisen oder für gescannte Teilchenstrahlen, bei denen das Gesichtsfeld durch die maximale kontrollierbare Ablenkung des Teilchenstrahls vom Zentralstrahl bestimmt ist.

Jedoch ist die Größe eines Zielvolumens nicht durch die technischen Randbedingungen definiert, sondern z. B. durch die Größe eines Tumors und möglicherweise zuzüglich von Randbereichen, um die Streuung der Strahlen in der Optimierung zu kompensieren und damit ein besseres Optimierungsergebnis zu erzielen. Wird es notwendig, ein Zielvolumen zu bestrahlen, welches größer ist als die maximal einem Strahl zugängliche Größe, so können Spalt-Ebenen verwendet werden, um z.B. einen Strahl (ein sogenannter "compound-Strahl") zweizuteilen. Es werden dann zwei Strahlen erhalten, welche jeweils ein kleineres Gesichtsfeld aufweisen. Hierzu ist anzumerken, dass ein "compound-Strahl" kein tatsächlich zur Bestrahlung genutzter Strahl ist, sondern lediglich ein zur Berechnung bei der Strahlungsplanung verwendeter sogenannter virtueller Strahl ist.

Des Weiteren kann auch ein Strahlpositionierungspuffer im Rahmen des Zielvolumens berücksichtigt werden, der es erlaubt, die Einzelstrahlen und/oder Spalt-Ebenen zu verschieben.

Zum Beispiel indem das Zielvolumen zwischen dem Applizieren des ersten der beiden Strahlen und dem Applizieren des zweiten der beiden Strahlen bewegt wird oder indem die Strahlungsquelle bewegt wird, kann erreicht werden, dass beide Strahlen in dem entsprechenden Zielvolumen eine Teilstrahlendosis applizieren können.

Durch die Kombination von Spalt-Ebenen mit Patch-Ebenen wird es möglich, in bestimmten klinischen Anwendungsfällen besonders gute Ergebnisse in der Applikation von Strahlendosen in Zielvolumen zu erreichen. Die Bestrahlung entlang der Wirbelsäule ist ein Beispiel. Zum Beispiel kann es notwendig sein, dass das Rückenmark von der Applikation einer Strahlendosis ausgenommen wird. Solche Bereiche, die gegenüber der Applikation von Strahlendosis geschützt werden müssen, werden als gefährdetes Organ (OAR) bezeichnet und können in der Behandlungsplanung definiert werden. Durch die Kombination von zwei im Wesentlichen entgegengesetzten Strahlen, welche mit unterschiedlichen Seiten einer ersten Steuerungsebene, d.h. Patch-Ebene, verknüpft sind, kann erreicht werden, dass das gefährdete Organ in Form des Rückenmarks von der Applikation einer Strahlendosis ausgenommen wird. Gleichzeitig kann es aber notwendig sein, die Bestrahlung entlang eines ausgedehnten Bereichs entlang der Wirbelsäule durchzuführen. Wenn dieses Zielvolumen die maximale Größe des Gesichtsfelds eines Strahls überschreitet, ist es notwendig, zweite Steuerungsebenen, d.h. Spalt-Ebenen, zu verwenden, um die entgegengesetzten Strahlen in mehrere Unterstrahlen aufzuteilen.

Gemäß dem gegenwärtigen Aspekt ist es möglich, eine solche Applikation von Strahlen unter Kombination von ersten und zweiten Steuerungsebenen durchzuführen. Dies erhöht die Flexibilität in der Erstellung eines Bestrahlungsplans. Insbesondere ist es weiterhin möglich, den Bestrahlungsplan derart zu erstellen, dass die ungewollte Bestrahlung bzw. die Bestrahlung von nichtbeteiligten Gebieten minimiert wird.

Gleichzeitig ist es wichtig, dass die Berechnung der Teilstrahlendosis so erfolgt, dass verschiedene Zielgrößen eingehalten werden. Indem die Berechnung getrennt für einzelne Seiten einer ersten Steuerungsebene, das heißt getrennt und isoliert für die Untervolumina, welche einer Seite einer Patch-Ebene zugewandt sind, erfolgt, kann sichergestellt werden, dass die Summe aller lokalen Teilstrahlendosen an jedem Punkt innerhalb des Zielvolumens die Gesamtstrahlendosis ergibt. Dies ist zielführend, um eine sichere und zugleich wirksame Behandlung des Patienten zu ermöglichen. Insbesondere ermöglicht das Verfahren gemäß dem gegenwärtig diskutierten Aspekt das Anwenden von mehreren Strahlen, welche jeweils einer Patch-Ebene und/oder einer Spalt-Ebene zugeordnet sein können, wobei die Berechnung der Teilstrahlendosen derart intuitiv auf einen Parameterwechsel eines Benutzer reagiert, dass die Teilstrahlendosen nicht durch andere Parameter gestört werden. Auch Hardwarebeschränkungen wie ein maximales Gesichtsfeld eines Strahls können in der Berechnung der Teilstrahlendosen berücksichtigt werden.

Insbesondere kann ein Strahl jeweils eindeutig einer Seite einer ersten Steuerungsebene zugeordnet sein. Dies bedeutet, dass ein Strahl nur einer Seite einer ersten Steuerungsebene, das heißt einer Patch-Ebene, zugeordnet sein kann und nicht z. B. zwei Seiten unterschiedlicher Patch-Ebenen zugeordnet sein kann oder auch nicht sowohl der Vorderseite oder der Rückseite einer Patch-Ebene zugeordnet sein kann. Diese eindeutige Zuordnung von Strahlen zu einer Seite einer Patch-Ebene erlaubt es, Zweideutigkeiten in der Erstellung eines Behandlungsplans oder der Berechnung von Teilstrahlendosen zu vermeiden.

Weiterhin können die lokalen Teilstrahlendosen eines einer Seite einer ersten Steuerungsebene zugeordneten Strahls in den durch diese erste Steuerungsebene definierten Untervolumen unterschiedlich sein. Dies bedeutet, dass, wenn ein Strahl z. B. der Vorderseite einer Patch-Ebene zugeordnet ist, der Strahl in dem der Vorderseite der Patch-Ebene zugewandten Untervolumen eine bestimmte Teilstrahlendosis appliziert. Beispielhaft soll diese Dosis etwa 10 Gray betragen. Dann kann es vorteilhaft sein, wenn der Strahl in dem anderen durch diese Patch-Ebene definierten Untervolumen, das bedeutet dem Untervolumen, welches der Rückseite der Patch-Ebene zugewandt ist, eine Teilstrahlendosis appliziert, welche von 10 Gray unterschiedlich ist, z. B. nur 2 Gray beträgt.

Typischerweise kann dies dadurch erreicht werden, dass in der Steuerung der Erzeugung des Strahls bestimmte Strahlenergien derart vorgegeben werden, dass je nach Eindringtiefe des Strahls in das Gewebe, das heißt je nach Position des Strahls in Bezug auf die Patch-Ebene, unterschiedliche Teilstrahlendosen appliziert werden.

Der dosimetrische Effekt der dadurch entsteht, dass ein Strahl in unterschiedlichen Untervolumina einer Patch-Ebene unterschiedliche Teilstrahlendosen appliziert, liegt darin, dass es möglich ist, die Strahlenbelastung von gesundem Gewebe, welches zwischen z. B. der Hautoberfläche und dem Zielvolumen liegt, zu minimieren.

Es ist auch möglich, dass die ersten Steuerungsebenen eine endliche Dicke aufweisen, wobei die Strahlen, welche auf unterschiedlichen Seiten der jeweiligen ersten Steuerungsebene unterschiedliche lokale Teilstrahlendosen aufweisen, durch einen örtlichen Verlauf der lokalen Teilstrahlendosis innerhalb der ersten Steuerungsebene einen graduellen Übergang der lokalen Teilstrahlendosis gewährleisten.

Zum Beispiel kann die Position einer ersten Steuerungsebene durch den Mittelpunkt der ersten Steuerungsebene definiert sein, wenn die erste Steuerungsebene, das heißt die Patch-Ebene, eine gewisse Dicke aufweist. Die Vorderseite bzw. die Rückseite einer ersten Steuerungsebene ist dann gegenüber dem Mittelpunkt, das heißt der Position der ersten Steuerungsebene, versetzt.

Diesbezüglich kann es erwünscht sein, dass, wenn ein Strahl in unterschiedlichen Untervolumina unterschiedliche Teilstrahlendosen appliziert, der Übergang nicht instantan als Funktion des Ortes geschieht, sondern graduell. Wiederum ist damit ein dosimetrischer Vorteil verbunden. Es kann nämlich möglich sein, lokale Überhöhungen bzw. Abschwächungen der applizierten Dosis, sogenannte "Hot-Spots" und "Cold-Spots", zu vermeiden.

Um dies zu erreichen kann es vorteilhaft sein, wenn ein gradueller Übergang der lokalen Teilstrahlendosis zwischen der Vorder- und der Rückseite einer ersten Steuerungsebene bzw. Patch-Ebene mit der ein Strahl verknüpft ist, zu gewährleisten. Dann ist die lokale Teilstrahlendosis in jeweils dem der Vorderseite zugewandten Untervolumen und dem der Rückseite zugewandten Untervolumen konstant und variiert innerhalb der Patch-Ebene. Das Berechnen der lokalen Teilstrahlendosis für ein Untervolumen, welches einer Seite einer Patch-Ebene zugewandt ist, bedeutet dann, dass die lokale Teilstrahlendosis sowohl außerhalb der Patch-Ebene berechnet wird (wo sie z. B. konstant sein kann), als auch innerhalb der Patch-Ebene berechnet wird (wo sie sich graduell verändern kann).

Weiterhin ist es möglich, dass für jeden Strahl ein Strahlengewicht festgelegt wird, wobei die Strahlengewichte die relativen Verhältnisse der Teilstrahlendosen verschiedener Strahlen zueinander festlegen.

Es kann z. B. mittels Strahlengewichten erreicht werden, dass bestimmte Strahlen einen größeren Anteil einer bestimmten Untervolumen-Gesamtstrahlendosis oder der Gesamtstrahlendosis applizieren, als andere Strahlen. Mittels Strahlengewichten können die von verschiedenen Strahlen applizierten Teilstrahlendosen gegeneinander relativ gewichtet werden.

Zum Beispiel kann mittels eines Wichtungsfaktors festgelegt werden, dass ein bestimmter erster Strahl fünfmal so viel Teilstrahlendosis appliziert, wie ein bestimmter zweiter Strahl. Der Wichtungsfaktor für den ersten Strahl kann z. B. 5 betragen, während der Wichtungsfaktor für den zweiten Strahl z. B. 1 betragen kann. Es ist aber auch möglich, dass der Wichtungsfaktor für den ersten Strahl 20 beträgt und der Wichtungsfaktor für den zweiten Strahl 4 beträgt. In jedem Fall ist das Verhältnis der beiden Wichtungsfaktoren 5.

Durch das verwenden von Wichtungsfaktoren kann wiederum ein dosimetrischer Effekt erzielt werden. Es kann vorteilhaft sein, wenn ein bestimmter Strahl, welcher einen größeren Abstand zu einem OAR aufweist als ein weiterer Strahl, eine größere Teilstrahlendosis aufweist als der weitere Strahl.

Dann kann die in dem OAR applizierte Dosis minimiert werden.

Insbesondere kann durch das Verfahren gemäß dem gegenwärtig diskutierten Aspekt weiterhin sichergestellt werden, dass die Strahlwichtungsfaktoren der verschiedenen Strahlen nicht die Berechnung der Teilstrahlendosen innerhalb eines durch eine Patch-Ebene erzeugten Untervolumens beeinflussen. Dies ist der Fall, da die Berechnung der Teilstrahlendosen immer nur isoliert für ein Untervolumen, welches einer Seite einer Patch-Ebene zugewandt ist, erfolgt. Werden dann z. B. die Strahlengewichte eines Strahls, welcher mit einer anderen Seite desselben oder einer anderen Patch-Ebene verknüpft ist, im Rahmen der Behandlungsplanung verändert, so hat dies keinen Einfluss auf die Untervolumen-Gesamtstrahlendosis für welche die Teilstrahlendosis eines bestimmten Strahles berechnet wird. Dies vereinfacht die Berechnung und erlaubt es, die Teilstrahlendosen robust und unter Berücksichtigung der Strahlwichtungsfaktoren zu berechnen. Weiterhin können die Teilstrahlendosen flexibel angepasst werden.

Weiterhin kann das Verfahren gemäß dem gegenwärtig diskutierten Aspekt innerhalb des Zielvolumens für eine bestimmte Seite einer bestimmten ersten Steuerungsebene das Festlegen von ersten Lokalwichtungsfaktoren für jeden Abstand senkrecht zu der bestimmten ersten Steuerungsebene umfassen, wobei die ersten Lokalwichtungsfaktoren den Bruchteil der jeweiligen Untervolumen-Gesamtstrahlendosis an der Gesamtstrahlendosis definieren. Das Verfahren gemäß dem gegenwärtig diskutierten Aspekt kann weiterhin für jeden der bestimmten Seite der bestimmten ersten Steuerungsebene zugeordneten Strahl das Festlegen von zweiten Lokalwichtungsfaktoren für jeden Abstand senkrecht zu einer zweiten Steuerungsebene umfassen, zu denen der jeweilige Strahl zugeordnet ist, wobei die zweiten Lokalwichtungsfaktoren die Strahlengewichte der Strahlen in Abhängigkeit von der Position zur zweiten Steuerungsebene modifizieren. Weiterhin kann das Verfahren gemäß dem gegenwärtig diskutierten Aspekt für jeden der bestimmten Seite der bestimmten ersten Steuerungsebene zugeordneten Strahl das Berechnen der lokalen Teilstrahlendosis für jedes Untervolumen für den jeweiligen Strahl umfassen, basierend auf Elementen, die aus der folgenden Gruppe ausgewählt werden: Strahlwichtungsfaktoren, erste Lokalwichtungsfaktoren, zweite Lokalwichtungsfaktoren, Gesamtstrahlendosis.

Die Verwendung von ersten und zweiten Lokalwichtungsfaktoren kann die Berechnung der Teilstrahlendosen weiter vereinfachen. Zum Beispiel ist es mittels der ersten Lokalwichtungsfaktoren möglich, ortsaufgelöste lokale Teilstrahlendosen derart zu berechnen, dass z. B. ein gradueller Übergang der lokalen Teilstrahlendosen zwischen einer Vorderseite und einer Rückseite einer Patch-Ebene gewährleistet werden kann. Hierzu werden die ersten Lokalwichtungsfaktoren in Abhängigkeit von der Position in Bezug auf z. B. den Mittelpunkt der ersten Steuerungsebene definiert, z.B. als Lot auf die durch den Mittelpunkt definierte Ebene. Es ist dann möglich, eine bestimmte Berechnung jeweils für jeden Wert eines ersten Lokalwichtungsfaktors durchzuführen.

Die Berechnung der Teilstrahlendosen kann auch dadurch vereinfacht werden, dass zweite Lokalwichtungsfaktoren eine Wichtung der Strahlwichtungsfaktoren ermöglichen. Zum Beispiel ist es möglich, dass die zweiten Lokalwichtungsfaktoren die Strahlwichtungsfaktoren zwischen 0% und 100% ihres Wertes modifizieren. Eine solche Modifikation der Strahlwichtungsfaktoren kann z. B. multiplikativ erfolgen. Insbesondere ist es auch in Bezug auf die zweiten Lokalwichtungsfaktoren möglich, einen graduellen Übergang der Teilstrahlendosen in Bezug auf eine zweite Steuerungsebene (Spalt-Ebene) einfach zu berechnen.

Zum Beispiel können die ersten und zweiten Steuerungsebenen eine Dicke aufweisen und die ersten und zweiten Lokalwichtungsfaktoren jeweils innerhalb der entsprechenden Steuerungsebene als Funktion der Position variieren. Derart kann es möglich sein, eine stetige Änderung der Teilstrahlendosen zu erzeugen. Es ist aber auch möglich, z.B. eine treppenförmige örtliche Änderung der Teilstrahlendosen zu erreichen. Wie schon in Bezug auf die ersten Lokalwichtungsfaktoren erläutert, kann die Berechnung der Teilstrahlendosen ortsaufgelöst dann für jeden Wert der zweiten Lokalwichtungsfaktoren erfolgen. Insbesondere kann das Berechnen gemäß folgender Formel für eine Seite einer ersten Steuerungsebene erfolgen:

$$D_i = \frac{w_i \prod_k c_{sp,i,k}}{\sum_{j=1}^{n} w_j \prod_l c_{sp,j,l}} c_{pp} D_{pr} \text{ ,}$$

wobei hier die Strahlwichtungsfaktoren, $c_{pp}$ den entsprechenden ortsaufgelösten ersten Lokalwichtungsfaktor, $c_{pp}D_{pr}$ außerhalb einer Patch-Ebene die Untervolumen-Gesamtstrahlendosis bezeichnet, $D_{pr}$ die Gesamtstrahlendosis und $c_{sp}$ die ortsaufgelösten zweiten Lokalwichtungsfaktoren für alle Strahlen, die der entsprechenden Seite der ersten Steuerungsebene zugeordnet sind, bezeichnet.

Da nämlich $c_{pp}$ den Bruchteil der Untervolumen-Gesamtstrahlendosis an der Gesamtstrahlendosis bezeichnet, ergibt sich dass $c_{pp}D_{pr}$ außerhalb einer Patch-Ebene die Untervolumen-Gesamtstrahlendosis bezeichnet. Für eine gegebene Untervolumen-Gesamtstrahlendosis erfolgt dann eine Berechnung der Teilstrahlendosen $D_i$ für die verschiedenen Strahlen, die der entsprechenden Seite der Patch-Ebene zugeordnet sind. Hierbei bezeichnet $w_i$ den zu dem

jeweiligen Strahl gehörigen Wichtungsfaktor. Sind mehrere Patch-Ebenen vorhanden, so können die Faktoren $c_{pp}$ aufeinander abgestimmt werden. Dies stellt sicher, dass die Summe aller Untervolumen-Gesamtstrahlendosen an jedem Punkt innerhalb des Zielvolumens gleich der Gesamtstrahlendosis ist.

Ein Strahl kann mehreren Spalt-Ebenen zugeordnet sein. Die zweiten Lokalwichtungsfaktoren $c_{sp}$ nehmen jeweils eine Modifikation der Wichtungsfaktoren $w_i$ zwischen 100% und 0% deren Werte vor, d.h. nehmen selbst Werte zwischen 0 und 1 an. Dies wird in obiger durch Multiplikation mit $0 \leq c_{sp,j,l} \leq 1$ erreicht. Der Bruch in obiger Formel berechnet dann den Anteil eines Strahls an der Untervolumen-Gesamtstrahlendosis durch Normierung auf die Summe aller Teilstrahlendosis-Anteile aller mit der entsprechenden Seite verknüpften Strahlen.

Aus obiger Formel ist es ersichtlich, dass, wenn z. B. ein Strahlwichtungsfaktor $w_i$ eines Strahls geändert wird, welcher nicht mit der jeweiligen Seite einer Patch-Ebene verknüpft ist, für die die Berechnung isoliert durchgeführt wird, sich keiner der Parameter in obiger Formel ändert und somit die Berechnung nicht wiederholt werden muss. Dies ist der Fall, da die Berechnung isoliert für die einer Seite einer Patch-Ebene zugeordneten Strahlen erfolgt, d.h. nur die relevanten Strahlen in die Berechnung einbezogen werden. Insbesondere bleiben die Wichtungen der verschiedenen Strahlen zueinander korrekt. Wichtig hierfür ist, dass sich die Berechnung gemäß obiger Formel, das heißt das Ausführen der Summe im Nenner, nur über diejenigen Strahlen erstreckt, welche mit derselben Seite einer Patch-Ebene, das heißt der ersten Steuerungsebene verknüpft sind.

Durch das Verwenden des Produkts in Bezug auf die zweiten Steuerungsfaktoren ist es möglich, dass auch sich überlappende Spalt-Ebenen, welche zu demselben Strahl gehören, rechnerisch von obiger Formel erfasst werden können. Typischerweise nehmen die zweiten Steuerungsfaktoren $c_{sp}$ Werte zwischen 0 und 1 jeweils in Bezug auf die Position gegenüber einer zweiten Steuerungsebene an. Wird z. B. die lokalen Teilstrahlendosis für einen Punkt innerhalb von zwei Steuerungsebenen berechnet (welche sich schneiden können), so können mehreren $c_{sp}$-Faktoren Werte ungleich 0 und ungleich 1 annehmen. Die Modifikation des Strahlwichtungsfaktors $w_i$ erfolgt dann entsprechend.

Insbesondere können die ersten und zweiten Lokalwichtungsfaktoren ortsaufgelöst in Bezug auf eine erste bzw. zweite Steuerungsebene innerhalb der entsprechenden Ebenen definiert sein. Dann ist es möglich, dass die Teilstrahlendosen ortsaufgelöst in Abhängigkeit von ihrer Positionierung zu den Steuerungsebenen für jedes von den Strahlen abgerasteten Volumenelement berechnet werden. Die Berechnung erfolgt ortsaufgelöst. Die graduelle Variation der Teilstrahlendosen wurde bereits voranstehenden diskutiert.

Weiterhin kann das Verfahren gemäß dem gegenwärtig diskutierten Aspekt das Festlegen von Strahlen ohne Zuordnung zu der ersten oder zweiten Steuerungsebene umfassen, wobei die Teilstrahlendosen dieser Strahlen vor dem isolierten Berechnen von der Gesamtstrahlendosis abgezogen werden. Strahlen, die weder eine Zuordnung zu einer Seite einer ersten Steuerungsebene, das heißt Patch-Ebene, oder zu einer Seite einer zweiten Steuerungsebene, das heißt Spalt-Ebene, aufweisen, werden auch als normale Strahlen bezeichnet. Solche Strahlen applizieren die Teilstrahlendosis ohne eine Abhängigkeit vom Ort gleichmäßig im Zielvolumen. Das macht es möglich, vor dem Berechnen der Teilstrahlendosis von Strahlen, welche eine Zuordnung zu einer ersten oder zweiten Steuerungsebene erfahren, die Gesamtstrahlendosis um die Summe der Teilstrahlendosen aller Strahlen, welche keine Zuordnung zu Steuerungsebenen aufweisen, zu korrigieren. Dann kann das Berechnen der Teilstrahlendosis gemäß dem Verfahren gemäß dem gegenwärtig diskutierten Aspekt entsprechend mit der korrigierten Gesamtstrahlendosis fortgesetzt werden.

Es sollte verstanden werden, dass es auch möglich ist, dass solche Strahlen ohne Zuordnung ein Zielvolumen überdecken, welches größer als das maximale zugängliche Gesichtsfeld ist. Dann kann es möglich sein, ein erfindungsgemäßes Verfahren zur Aufspaltung dieser Strahlen mittels Spalt-Ebenen durchzuführen, wobei diese untergeordnete Aufspaltung losgelöst von den übergeordneten Spalt-Ebenen ist zu denen der Strahl nicht zugeordnet ist.

Es ist auch möglich, dass das Verfahren gemäß dem gegenwärtig diskutierten Aspekt weiterhin das Festlegen von Strahlwichtungsfaktoren für Strahlen ohne Zuordnung zu ersten oder zweiten Steuerungsebenen umfasst und weiterhin das Anpassen der Gesamtstrahlendosis für Strahlen, die ersten und zweiten Steuerungsebenen zugeordnet sind, basierend auf den Strahlwichtungsfaktoren für Strahlen ohne Zuordnung zu ersten oder zweiten Steuerungsebenen umfasst. Werden auch Strahlen ohne Zuordnung zu Steuerungsebenen, das heißt normale Strahlen, Strahlwichtungsfaktoren zugeordnet, so können diese Strahlen eine Gewichtung in Bezug auf die Gewichtung der Strahlen mit Zuordnung zu Steuerungsebenen haben. Hierzu kann z. B. zur Berechnung der Teilstrahlendosis eines normalen Strahls derart erfolgen, dass für alle einer Steuerungsebene zugeordneten Strahlen ein akkumulierter Wichtungsfaktor von z. B. 1 oder einem anderen Wert festgelegt wird. Besitzt dann der normale Strahl einen Wichtungsfaktor z.B. von 5, so weist der normale Strahl eine Teilstrahlendosis auf, welche um Faktor 5 gegenüber der Summe der durch z.B. einer ersten Steuerungsebene zugeordneten Strahlen applizierten Teilstrahlendosis größer ist.

Gemäß einem weiteren Aspekt wird ein Verfahren zum Berechnen von lokalen Teilstrahlendosen in einer Strahlentherapieanlage zum Applizieren einer Gesamtstrahlendosis in einem Zielvolumen mit mehreren Strahlen bereitgestellt. Das Verfahren umfasst das Festlegen mindestens einer zweiten Steuerungsebene zur Steuerung der Positionierung der Strahlen, wobei jede der mindestens einen zweiten Steuerungsebene das Zielvolumen in zwei Untervolumen aufteilt. Das Verfahren umfasst weiterhin das Zuordnen mindestens eines Strahls zu jeder der mindestens einen zweiten Steuerungsebenen, wobei ein einer zweiten Steuerungsebene zugeordneter Strahl durch die jeweilige zweite Steuerungsebene derart zweigeteilt wird, dass die lokale Teilstrahlendosis der so erhaltenen zwei Strahlen jeweils in unterschiedlichen, durch die jeweilige zweite Steuerungsebene definierten Untervolumen ungleich Null ist. Das Verfahren umfasst weiterhin das Festlegen von Strahlen ohne Zuordnung zu zweiten Steuerungsebenen, das Korrigieren der Gesamtstrahlendosis durch Subtraktion der lokalen Teilstrahlendosen der Strahlen ohne Zuordnung von der Gesamtstrahlendosis und das Berechnen der lokalen Teilstrahlendosis für alle einer zweiten Steuerungsebene zugeordneten Strahlen.

Gemäß dem gegenwärtig diskutierten Aspekt der Erfindung ist es demnach möglich, normale Strahlen, das heißt Strahlen ohne Zuordnung zu einer ersten oder zweiten Steuerungsebene, mit einer zweiten Steuerungsebene, das heißt einer Spalt-Ebene, zu kombinieren. Wie oben bereits dargelegt, wird vor dem Berechnen der Teilstrahlendosen derjenigen Strahlen, welche einer zweiten Steuerungsebene zugeordnet sind, eine Korrektur der Gesamtstrahlendosis derart vorgenommen, dass die Gesamtstrahlendosis um die Summe der Teilstrahlendosen der normalen Strahlen korrigiert wird.

Gemäß dem gegenwärtig diskutierten Aspekt ist es auch möglich, dass für jeden Strahl ein Strahlengewicht festgelegt wird, wobei die Strahlengewichte die relativen Verhältnisse der Teilstrahlendosis verschiedener Strahlen zueinander festlegen. Das Festlegen der Strahlengewichte gemäß dem gegenwärtig diskutierten Aspekt erfolgt wie bereits oben erläutert.

Hierbei kann insbesondere das Strahlengewicht aller einer zweiten Steuerungsebene zugeordneten Strahlen in Summe Eins sein. Dies wurde bereits obenstehend in Bezug auf den weiteren Aspekt der folgenden Erfindung erläutert.

Es ist auch möglich, dass das Strahlgewicht der Strahlen, die mit unterschiedlichen Seiten einer zweiten Steuerungsebene verknüpft sind, d.h. durch eine Steuerungsebene zweigeteilt werden, identisch ist. Dann kann es möglich sein in Bezug auf die Positioniergenauigkeit möglichst robuste Strahlen zu erhalten.

Insbesondere kann die Summe aller lokalen Teilstrahlendosen aller Strahlen an jeder Position innerhalb des Zielvolumens gleich der Gesamtstrahlendosis sein. Ist nämlich die Summe aller Teilstrahlendosen gleich der Gesamtstrahlendosis, so wird sichergestellt, dass eine Bestrahlung des Zielobjekts, z. B. des Tumors, gemäß einem vorher festgelegten Behandlungsplan, d.h. einer verschriebenen Dosis, erfolgt. Hierbei ist die verschriebene Dosis bzw. die Gesamtstrahlendosis gemäß den klinisch erforderlichen Parametern festgelegt.

Es ist auch möglich, dass die zweiten Steuerungsebenen eine endliche Dicke aufweisen und wobei Strahlen, die durch eine zweite Steuerungsebene zweigeteilt sind, durch einen örtlichen Verlauf der lokalen Teilstrahlendosis innerhalb der zweiten Steuerungsebene einen graduellen Übergang der lokalen Teilstrahlendosis gewährleisten. Obenstehend wurden bereits Effekte diskutiert, die in Bezug auf erste Steuerungsebenen mit endlicher Dicke auftreten können. Entsprechende Effekte können auch auftreten, wenn zweite Steuerungsebenen eine endliche Dicke aufweisen.

Obenstehend wurde der graduelle Übergang von Teilstrahlendosen für Strahlen diskutiert, welche einer Seite einer ersten Steuerungsebene, einer Patch-Ebene, zugeordnet bzw. verknüpft sind. Hierbei kann es auch möglich sein, dass ein Strahl eine lokale Teilstrahlendosis aufweist, welche auf den zwei Seiten einer Patch-Ebene zwar unterschiedlich ist, aber auf beiden Seiten ungleich 0 ist. In Bezug auf eine zweite Steuerungsebene, das heißt in Bezug auf eine Spalt-Ebene, kann es jedoch möglich sein, dass die lokale Teilstrahlendosis nur auf einer Seite der Spalt-Ebene ungleich Null ist.

Insbesondere kann die lokale Teilstrahlendosis für einen einer ersten Steuerungsebene zugeordneten Strahl auf den unterschiedlichen Seiten der ersten Steuerungsebene ungleich Null sein und die lokale Teilstrahlendosis für einen einer zweiten Steuerungsebene zugeordneten Strahl auf genau einer Seite der Seiten der zweiten Steuerungsebene gleich 0 sein.

Dies ist typischerweise der Fall, da eine zweite Steuerungsebene, das heißt eine Spalt-Ebene, dann verwendet wird, wenn z. B. das Gesichtsfeld eines Strahls nicht ausreichend ist, um das gesamte Zielvolumen zu überdecken.

In Bezug auf den graduellen Übergang der Teilstrahlendosen innerhalb einer zweiten Steuerungsebene bedeutet dies, dass der graduelle Übergang z. B. von einem endlichen Wert der lokalen Teilstrahlendosis auf einer Seite der Spalt-Ebene hin zu einem verschwindenden Wert der lokalen Teilstrahlendosis auf der anderen Seite der zweiten Steuerungsebene erfolgen kann.

Es ist möglich, dass die Strahlen durch einen Strahlursprung charakterisiert sind, wobei der Strahlursprung die Position bezeichnet, an dem der Strahl erzeugt wird. Dabei ist es insbesondere möglich, dass zwei Strahlen, welche durch eine zweite Steuerungsebene zweigeteilt sind, im Wesentlichen gleiche Strahlursprünge haben. Dies ist der Fall, wenn z. B. während dem Applizieren von zwei Strahlen, welche durch eine zweite Steuerungsebene geteilt sind, das Zielvolumen gegenüber dem Strahlursprung rotiert wird. Auch eine Verschiebung oder eine Kombination aus Verschiebung und Drehung des Zielvolumens ist möglich.

Gemäß einem weiteren Aspekt wird eine Strahlentherapieanlage bereitgestellt, wobei die Strahlentherapieanlage ein Behandlungs-Planungssystem, eine Verarbeitungsvorrichtung und eine Strahlerzeugungsvorrichtung umfasst. Das Behandlungs-Planungssystem ist konfiguriert zum Berechnen von lokalen Teilstrahlendosen zum Applizieren einer Gesamtstrahlendosis in einem Zielvolumen mit mehreren Strahlen folgende Schritte durchzuführen: Festlegen mindestens einer ersten Steuerungsebene zur Steuerung der Dosierung der Strahlen, wobei jede der mindestens einen Steuerungsebene das Zielvolumen in zwei Untervolumen aufteilt, jeweils für die Vorderseite und die Rückseite jeder der mindestens einen ersten Steuerungsebene, Zuordnen mindestens eines Strahls, für jede erste Steuerungsebene und Festlegen einer Untervolumen-Gesamtstrahlendosen, für jeweils jedes der zwei Untervolumen als ein Bruchteil der Gesamtstrahlendosis. Weiterhin ist das Behandlungs-Planungssystem konfiguriert, die folgenden Schritte durchzuführen: Festlegen mindestens einer Steuerungsebene zur Steuerung der Positionierung der Strahlen, wobei jede der mindestens einen zweiten Steuerungsebene das Zielvolumen in zwei Untervolumen aufteilt und Zuordnen mindestens eines Strahls zu jeder der mindestens eines Strahls zu jeder der mindestens einen zweiten Steuerungsebene, wobei ein zu einer zweiten Steuerungsebene zugeordneter Strahl durch die jeweilige zweite Steuerungsebene derart zweigeteilt wird, dass die lokale Teilstrahlendosis der so erhaltenen zwei Strahlen jeweils in unterschiedlichen, durch die jeweilige zweite Steuerungsebene definierten Untervolumen ungleich Null ist. Weiterhin ist die Verarbeitungsvorrichtung konfiguriert, den folgenden Schritt durchzuführen: Für mindestens eine Seite einer ersten Steuerungsebene, isoliertes Berechnen der entsprechenden Teilstrahlendosen aller dieser ersten Steuerungsebene zugeordneter Strahlen, so dass die Summe der lokalen Teilstrahlendosen der restlichen dieser ersten Steuerungsebene zugeordneten Strahlen die Differenz zwischen der jeweilig Untervolumen-Gesamtstrahlendosis und der Gesamtstrahlendosis ergibt. Weiterhin ist die Strahlenerzeugungsvorrichtung konfiguriert, Strahlen mit der von der Verarbeitungsvorrichtung berechneten Teilstrahlendosis zu applizieren.

Für eine Strahlentherapieanlage gemäß dem gegenwärtig diskutierten Aspekt können Effekte erzielt werden, welche den Effekten entsprechen, die durch die Verfahren gemäß den anderen Aspekten der gegenwärtigen Erfindung erzielt werden.

Gemäß einem weiteren Aspekt wird eine Strahlentherapieanlage bereitgestellt, welche ein Behandlungs-Planungssystem, eine Verarbeitungsvorrichtung und eine Strahlenerzeugungsvorrichtung umfasst. Hierbei ist das Behandlungs-Planungssystem konfiguriert zum Berechnen der lokalen Teilstrahlendosis zum Applizieren einer Gesamtstrahlendosis in einem Zielvolumen mit mehreren Strahlen folgende Schritte durchzuführen: Festlegen mindestens einer zweiten Steuerungsebene zur Steuerung der Positionierung der Strahlen, wobei jede der mindestens einen zweiten Steuerungsebene das Zielvolumen in zwei Untervolumen aufteilt und Zuordnen mindestens eines Strahl zu jeder der mindestens einen zweiten Steuerungsebene, wobei ein zu einer zweiten Steuerungsebene zugeordneter Strahl durch die jeweilige zweite Steuerungsebene derart zweigeteilt wird, dass die lokale Teilstrahlendosis der so erhaltenen zwei Strahlen jeweils in unterschiedlichen durch die jeweilige zweite Steuerungsebene definierten Untervolumen gleich Null ist. Weiterhin ist das Behandlungs-Planungssystem konfiguriert, den folgenden Schritt durchzuführen: Festlegen von Strahlen ohne Zuordnung zu zweiten Steuerungsebenen. Weiterhin ist die Verarbeitungsvorrichtung konfiguriert, die folgenden Schritte durchzuführen: Korrigieren der Gesamtstrahlendosis durch Subtraktion der lokalen Teilstrahlendosen der Strahlen ohne Zuordnung von der Gesamtstrahlendosis und Berechnen der lokalen Teilstrahlendosen für alle einer zweiten Steuerungsebene zugeordneten Strahlen, wobei die Strahlenerzeugungsvorrichtung konfiguriert ist, Strahlen mit der von der Verarbeitungsvorrichtung berechneten lokalen Teilstrahlendosis zu applizieren.

Für eine Strahlentherapieanlage gemäß dem gegenwärtig diskutierten Aspekt können Effekte erzielt werden, welche den Effekten entsprechen, die in Bezug auf die anderen Aspekte der gegenwärtigen Erfindung diskutiert wurden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden, wobei

Fig. 1 eine schematische Ansicht einer Strahlentherapieanlage ist,

Fig. 2 ein Querschnitt einer Patch-Ebene mit einem einfallenden Strahl ist,

Fig. 3 eine Aufsicht zweier Spalt-Ebenen mit drei einfallenden Strahlen ist,

Fig. 4 eine graduelle Variation einer Teilstrahlendosis innerhalb einer Spalt-Ebene illustriert,

Fig. 5 ein Querschnitt von sechs Spalt-Ebenen ist,

Fig. 6 die Rotation eines Liegetisches zwischen der Anwendung zweier divergenter Strahlen mit einer Spalt-Ebene zeigt,

Fig. 7 die Translation eines Liegetisches zwischen der Anwendung zweier divergenter Strahlen mit einer Spalt-Ebene zeigt,

Fig. 8 die graduelle Variation einer Strahlintensität innerhalb einer Spalt-Ebene unter Berücksichtigung einer minimal anwendbaren Strahlintensität zeigt,

Fig. 9 die Zuordnung von vier Strahlen zu zwei Patch-Ebenen illustriert,

Fig. 10 die Zuordnung von zwei Strahlen zu einer Patch-Ebene illustriert, wobei weiterhin ein Strahl ohne Zuordnung zu einer Patch-Ebene vorhanden ist,

Fig. 11 die Zuordnung von drei Strahlen zu einer Patch-Ebene illustriert, wobei die Patch-Ebene eine Dicke aufweist,

Fig. 12 die Zuordnung von zwei Strahlen zu einer Patch-Ebene illustriert, wobei die zwei Strahlen jeweils durch eine Split-Ebene zweigeteilt werden, sodass insgesamt vier Strahlen erhalten werden,

Fig. 13 die Berechnung der lokalen Teilstrahlendosis innerhalb einer Patch-Ebene und innerhalb einer Split-Ebene illustriert,

Fig. 14 die Berechnung der lokalen Teilstrahlendosis innerhalb zweier Spalt-Ebenen illustriert und

Fig. 15 ein Flussdiagramm eines Verfahrens zum Betreiben einer Strahlentherapieanlage darstellt.

Fig. 1 zeigt schematisch eine Strahlentherapieanlage 200, welche eine Strahlerzeugungsvorrichtung 201 umfasst. Die Strahlerzeugungsvorrichtung 201 dient zum Erzeugen eines Partikelstrahls 202 oder einer elektromagnetischen Strahlung 202 zum Behandeln eines in der Strahlentherapieanlage 200 angeordneten Patienten 203. Erfindungsgemäß können sowohl Partikel- als auch Photonen- oder Elektronenstrahlen Anwendung finden. Im Folgenden wird jedoch hauptsächlich Bezug auf Partikelstrahlen genommen, was jedoch nicht limitierend ausgelegt werden darf.

Der Patient 203 ist entlang einer Longitudinalrichtung angeordnet, die als Achse A bezeichnet ist. Der Patient ist beispielsweise auf einer verfahrbaren und rotierbaren Patientenliege 207 gelagert. Die Strahlerzeugungsvorrichtung 201 kann beispielsweise ein Linearbeschleuniger, ein so genannter Linear Accelerator (LINAC), oder eine Strahlungsquelle, beispielsweise eine Kobalt-60-Strahlungsquelle, umfassen Insbesondere kann die Strahlenerzeugungsvorrichtung 201 um die Achse A rotiert werden. Dies ermöglicht es, den Partikelstrahl unter verschiedenen Strahlwinkeln auf den auf den Patienten 203 auftreffen zu lassen.

Der Ort, an dem der Strahl 202 erzeugt wird, wird durch den Strahlursprung U bezeichnet. Insbesondere kann sich nahe am Strahlursprung U ein Lamellenkollimator 210 befinden. Ein Lamellenkollimator umfasst typischerweise in mehreren Reihen angeordnete Lamellen, welche eine hohe Absorption des Strahls 202 aufweisen. Die Lamellen lassen sich individuell entlang einer Achse bewegen, was einer Blende mit einstellbarer Form entspricht. Dadurch kann der Strahl 202 gemäß der Kontur dieser Blende fokussiert werden und gezielt in eine Zielfläche innerhalb des Patienten 203 geleitet werden. Insbesondere ist es möglich, die Kontur der Lamellen während einer Bestrahlung zu verändern. Zum Beispiel gibt es die sogenannte intensitätsmodulierte Strahlentherapie ("Intensity modulated radiation therapy", IMRT), bei welcher sowohl die Anordnung der Lamellen über der Zeit variiert wird, als auch gleichzeitig die Bestrahlungsdosis variiert wird. Dies macht es möglich, unterschiedliche Zielflächen mit unterschiedlichen Dosen zu bestrahlen bzw. komplexe örtliche Dosisvariationen zu ermöglichen. Die Funktionsweise eines Lamellenkollimators ist dem Fachmann bekannt, sodass auf eine weitere Ausführung hier verzichtet wird.

Für Partikelstrahlen wie z.B. Protonen- oder Schwerionenstrahlen kann der Strahl mittels aktiver Strahlführung 210 durch Ablenkmagnetfelder geführt werden. Es ist auch möglich den Strahl mittels passiver Systeme zu formen oder die Energieverteilung zu verschieben bzw. aufzuweiten.

Bei einmaliger Positionierung der Strahlerzeugungsvorrichtung 201 wird das Zentrum des durch den Lamellenkollimator 210 voll geöffneten Zustand bzw. durch volle Ablenkung mittels Ablenkmagnetfelder der Strahlfilterung 210 zugänglichen Bereichs als Isozentrum eines Strahles bezeichnet. Andere Strahlführungs- und Fokussierungssysteme können anderweitig begrenzte Gesichtsfelder aufweisen. Das heißt, das Isozentrum bezeichnet z.B. den Mittelpunkt diejenige Fläche, die maximal durch einen Strahl ohne Verfahren der Strahlerzeugungsvorrichtung 201, z. B. in Form von Rotation um die Achse A, zugänglich ist. Im Allgemeinen ist das Isozentrum der Punkt im Raum, um den sich alle isozentrischen Winkel drehen: Gantry, isozentrischer Tischwinkel, Kollimatorwinkel.

Typischerweise appliziert ein Strahl 202 in Volumenelementen, welche einen unterschiedlichen Abstand von der Oberfläche des Patienten 203 haben, unterschiedliche Bruchteile seiner insgesamt applizierten Dosis. Hierbei gibt es eine Eindringtiefe, in der eine maximale Dosis, das heißt ein Maximum der applizierten Dosis über Eindringtiefe, auftritt. Insbesondere variiert die Position dieses Dosismaximums in Abhängigkeit von der Strahlungsart und der Energie der Strahlung. Auch wird zwischen Eintritt der Strahlung in den Patienten 203 und der Position der maximalen applizierten Dosis ein gewisser Bruchteil der Dosis appliziert. Typischerweise wird dieser Bruchteil der Dosis in gesundes Gewebe, z.B. die Haut, appliziert und soll deshalb minimiert werden. Die Abhängigkeit der Eindringtiefe und des Dosisprofils von der Art des Strahls 202 und dessen Energie ist dem Fachmann bekannt und wird deshalb hier nicht weiter diskutiert.

Die Strahlentherapieanlage 200 umfasst weiterhin eine Verarbeitungsvorrichtung 205, welche mit der der Strahlerzeugungsvorrichtung 201 gekoppelt ist, um diese zu steuern. Die Verarbeitungsvorrichtung 205 umfasst beispielsweise einen Mikroprozessor oder eine programmierbare Steuervorrichtung, welche ein Programm, beispielsweise eine Software, ausführen kann. Das Programm oder die Software können beispielsweise mithilfe eines Datenträgers 206 in die Verarbeitungsvorrichtung 205 geladen werden. Die Verarbeitungsvorrichtung 205 ist weiterhin mit einem Behandlungs-Planungssystem 208 gekoppelt, um beispielsweise einen bestimmten Bestrahlungsplan von dem Behandlungs-Planungssystem 208 zu empfangen.

Das Behandlungs-Planungssystem 208 kann dazu verwendet werden, einen Behandlungsplan festzulegen. Im Allgemeinen erfolgt das wie im Folgenden dargelegt, wobei von Fall zu Fall das Schema leicht variieren kann:

1. Definiere Struktursätze für die Zielvolumen und die gefährdeten Organe (OAR)

2.a) Definiere verschriebene Dosen, die innerhalb der Ziele appliziert werden müssen.

2.b) Definiere verschriebene Dosen für gefährdete Organe, die nicht überschritten werden dürfen.

3.a) Definiere Ausgangsplanparameter, Definition des lateralen Scanrasters, z. B. Bestrahlungstypen, Anzahl der Strahlen, Isozentren, Strahlwinkel, Spalt-Ebenen, Patch-Ebenen, Strahlwichtungsfaktoren, Definition des lateralen Scanrasters, Energieabstand/Scanpunktabstand entlang der Tiefe des Strahls.

3.b) Definiere Optimierungsparameter, z. B. Optimierungsstrategie, Dosisberechnungsverfahren, Auflösung eines Dosiskubus, d.h. örtliche Auflösung der mit der ein Strahl gerastert wird, Zielkriterium, um die Optimierungsschleife zu verlassen.

4. Basierend auf den Verschreibungen, wie sie in den Punkten 2a/b dargelegt sind, werden Dosisbeschränkungen für die Ziele und die OAR definiert, z. B. physikalische Beschränkungen, wie minimale und maximale Dosenbeschränkungen, DVH-Beschränkungen oder auch biologische Beschränkungen, z. B. Tumor Control Probability: Wahrscheinlichkeit, den Tumor zu vernichten TCP, Normal Tissue Complication Probability: Wahrscheinlichkeit, dass es zu Nebenwirkungen im Normalgewebe/Risikoorgan kommt NTCP, Equivalent Uniform Dose: Homogen bestrahlte Dosis in einem Volumen, die zu demselben biologischen Effekt führt wie eine nicht-homogene Dosisverteilung in demselben Volumen (z.B. Teilbestrahlung eines Volumens) EUD, etc.

5. Ein zumeist iterativer Optimierungsprozess wird gestartet, wobei der Optimierer versucht, alle Beschränkungen, wie sie unter Punkt 4 definiert sind, zu erfüllen. Für verschiedene Strahlen, wie zuvor festgelegt, wird eine Teilstrahlendosis berechnet. Eine Gesamtstrahlendosis wird als Summe der Teilstrahlendosen erhalten, welche möglichst nahe an der verschriebenen Dosis liegen sollte. Im Fall von Biologischen Dosisverteilungen muss die biologische Effektivität der Strahlung berücksichtigt werden.

6. Das Ergebnis von Schritt 5 wird untersucht, Parameter werden ggf. geändert und eine Reoptimierung oder, für den Fall, dass alle Ziele erreicht wurden, eine zweite, unabhängige Dosisberechnung wird gestartet und der Plan wird zur Behandlung des Patienten verwendet.

Basierend auf den aus einem Bestrahlungsplan erhaltenen Teilstrahlendosen, d.h. der von einem Strahl in einem bestimmten Volumenelement applizierten Dosis, können die entsprechenden Dosenbeschränkungen für jeden Ort angepasst automatisch werden, bevor die Optimierung gestartet wird.

Im Rahmen der Erstellung eines Behandlungsplanes ist es möglich, Spalt-Ebenen und Patch-Ebenen zur Steuerung der Behandlung des Patienten mittels Behandlungs-Planungssystem 208 zu erstellen. Dies wird nachfolgend näher erläutert.

Erstens kann für den Fall, dass die Zielprojektion in Strahlrichtung das maximale Bestrahlungsfeldgebiet überschreitet, der ursprüngliche Strahl in zwei oder mehrere Unterstrahlen mit unterschiedlichen Isozentren oder Zielpunkte aufgespaltet werden. Das wird typischerweise Strahlenspaltung ("Beamsplit") genannt und das Aufspalten kann dadurch geschehen, dass mittels Behandlungs-Planungssystem 208 eine Spalt-Ebene ("Split-Plane") definiert wird, um zu differenzieren, auf welcher Seite welcher Strahl welche Dosis applizieren soll.

Zweitens kann, wenn ein dosimetrischer Vorteil erwartet wird, die Zielfläche in zwei verschiedene Abschnitte aufgespaltet werden, wobei ein oder mehrere Strahlen einen Abschnitt bestrahlen, wobei andere Strahlen den zweiten Abschnitt bestrahlen. Das ist insbesondere der Fall für gescannte Partikelstrahlbehandlung z.B. mittels Protonen oder Schwerionen. Auch für andere Bestrahlungsgegebenheiten kann es ein Anwendungsfeld geben. Dieses Anwendungsfeld wird Strahlpatch ("Beampatch") genannt und die Flächen werden durch Patch-Ebenen bezeichnet.

Wenn mittels Behandlungs-Planungssystem 208 entweder eine Spalt-Ebene oder eine Patch-Ebene definiert wird, dann haben diese beiden Fälle gemeinsam, dass das Zielvolumen in mehrere Untervolumen aufgeteilt wird, in welchen die Strahlen spezifische Dosen applizieren. Weiterhin haben diese zwei Fälle gemeinsam, dass, um die Robustheit des Bestrahlungsplans gegenüber Patientenbewegung oder Organbewegung zu maximieren, zwischen den Spalt-Ebenen und Patch-Ebenen eine Intensitätsrampe für jeden Strahl als Übergang von einer Zielebene zu einer benachbarten Zielebene definiert wird.

Jedoch gibt es einige Unterschiede zwischen aufgespalteten Strahlen (Spalt-Ebene) und gepatchten Strahlen (Patch-Ebene): Aufgespaltete Strahlen werden angewendet, wenn geometrische Strahlbeschränkungen, wie z. B. die maximale Feldgröße und Strahlform, die durch Gerätelimitierungen gegeben werden, verletzt werden. Im Falle der aufgespalteten Strahlen muss demnach das Isozentrum für jeden aufgespalteten Strahl verschoben werden, um diese Beschränkungen zu kompensieren.

Patchstrahlen werden hingegen typischerweise angewendet, um eine bessere Dosisverteilung gegenüber dem Fall ohne Patch-Ebenen zu erreichen. Zum Beispiel können zwei entgegengesetzte Partikelstrahlen verwendet werden. Dann reduziert eine Patch-Ebene die Dosis an jedem Strahleneingang signifikant, so dass normales Gewebe von zu großer Dosisapplikation ausgenommen werden kann. Ein weiteres Beispiel ist der Fall, dass ein gefährdetes Organ zumindest teilweise von dem Ziel umgeben ist. Patch-Ebenen können die Dosis, die in dem gefährdeten Organ appliziert wird, in diesem Fall signifikant reduzieren, da die Strahlen nicht das gefährdete Organ durchkreuzen. Deshalb teilen sich mit Patch-Ebenen verknüpfte Strahlen häufig dasselbe Isozentrum, auch wenn in manchen Fällen Vorteile daraus erwachsen, wenn mehrere Isozentren verwendet werden.

Weiterhin deponieren aufgespaltete Strahlen, d.h. einer Spalt-Ebene verknüpfte Strahlen, keine Dosis in Zielvolumina, die über die Spalt-Ebene hinweg liegen. Deren Intensitäten über die Spalt-Ebene hinaus ist Null. Das ist notwendig, um die Einschränkung der maximalen Feldgröße für jeden Strahl zu erfüllen. Diese Einschränkung ist hingegen für Patchstrahlen nicht notwendigerweise benötigt. In diesem Fall kann der Benutzer mittels Behandlungs-Planungssystem 208 ein Restdosislevel für die Fläche definieren, mit der der Patch-Strahl nicht verknüpft ist.

Weiterhin können mittels Behandlungs-Planungssystem 208 Strahlgewichte von Strahlen festgesetzt werden, um den Dosisbeitrag eines jeden Strahles zu steuern. Hingegen muss die Dosis in einem durch eine Patch-Ebene definierten Untervolumen fest sein und darf nicht mittels Strahlengewichten verändert werden. Anderenfalls würde der Benutzer die Möglichkeit erlangen, die Teilstrahlendosis eines Strahls mittels zweier Parameter zu verändern, d.h. über die Patch-Ebene und über das Strahlgewicht. Deshalb wird die erwünschte Teilstrahlendosis während der Optimierung der Strahlengewichte verändert.

Insbesondere ist es nun mittels Behandlungs-Planungssystem 208 möglich, sowohl Spalt-Ebenen als auch Patch-Ebene gleichzeitig zu definieren. Es gibt klinische Fälle, wo dies sinnvoll ist. Zum Beispiel ist dies der Fall, wenn entlang der Wirbelsäule bestrahlt werden muss. Hier ist das Rückenmark das gefährdete Organ, welches von Dosisapplizierung ausgenommen werden kann, indem zwei um 90° gegeneinander versetzte Patchstrahlen angewendet werden. Trotzdem ist die Zielfläche zu groß, als dass sie von einem einzelnen Strahl erfasst werden könnte. Deshalb müssten die Patchstrahlen mittels einer Spalt-Ebene weiterhin aufgespaltet werden.

Ein weiterer klinischer Fall, in dem die Kombination von aufgespalteten Strahlen und Patchstrahlen sinnvoll ist, ist ein gradueller Übergang der Dosis zwischen unmittelbar benachbarten Regionen eines Zielvolumens, welche unter unterschiedlichen Umständen bzw. mit unterschiedlichen Parametersätzen bestrahlt werden. Indem überlappende Strahlen und entsprechende Dosisrampen in dieser Fläche angewendet werden, können potentielle lokale Abweichungen von der gewünschten Dosis, sogenannte "Hot-Spots" und "Cold-Spots", welche durch Ungenauigkeiten während des Behandlungs-Setups hervorgerufen werden, verhindert werden.

Um die Unterschiede zwischen einer Spalt-Ebene und einer Patch-Ebene näher zu erläutern, werden in den folgenden Figuren 2 und 3 diese beiden Fälle zunächst isoliert betrachtet. In Fig. 2 trifft z.B. ein Partikelstrahl 3a von links auf ein Zielvolumen 4 (schraffiert dargestellt) auf. Zum Beispiel kann sich der Partikelstrahl 3a von einem Strahlursprung (nicht gezeigt) bei einer kleinen Ortskoordinate x durch den Patienten hin zu dem Zielvolumen 4 bewegen. Das Zielvolumen 4 kann z. B. ein Tumor sein. Das Zielvolumen kann auch eine Tiefe senkrecht zur Zeichenebene der Fig. 2 haben (nicht dargestellt).

Insbesondere sei z.B. eine bestimmte Position $x_0$ innerhalb des Zielvolumens. Es ist gewünscht, eine bestimmte Gesamtstrahlendosis ($D_{pr}$) innerhalb des Zielvolumens 4 zu applizieren. Die Gesamtstrahlendosis kann aus einem Bestrahlungsplan erhalten werden, wie in Bezug auf Fig. 1 diskutiert. Durch geeignete Wahl der Partikelenergie kann, wie voranstehend in Bezug auf Fig. 1 erläutert, erreicht werden, dass ein Strahl in Abhängigkeit der Position x, entlang des in Fig. 2 dargestellten Schnitts, eine bestimmte Dosis appliziert.

In Fig. 2 ist z.B. mittels eines Behandlungs-Planungssystems, wie voranstehend in Bezug auf Fig. 1 erläutert, eine Patch-Ebene 1 an einer bestimmten Position $x_0$ entlang des in Fig. 2 dargestellten Querschnitts innerhalb des Zielvolumens 4 platziert. Die Patch-Ebene 1 weist entlang der Richtung x eine bestimmte Dicke auf. Diese Dicke wird begrenzt durch die Vorderseite 10 und die Rückseite 11 der Patch-Ebene 1.

Es gibt verschiedene Gründe, warum es vorteilhaft sein kann, eine Patch-Ebene 1 in dem Beispiel von Fig. 2 zu verwenden. Eine Möglichkeit ist, dass sich das Zielvolumen 4 tief innerhalb des Patienten befindet. Wenn die verschriebene Gesamtstrahlendosis mittels z. B. nur eines Strahls innerhalb des Zielvolumens 4 appliziert wird, hat dies zur Folge, dass eine große Dosis zwischen der Hautoberfläche des Patienten und dem Zielvolumen 4 auf dem Weg des einen Strahls appliziert wird. Dies ist der Fall, da, wie in Bezug auf Fig. 1 schon beschrieben, immer ein Bruchteil der innerhalb eines bestimmten Volumens applizierten Dosis auch auf dem Weg zu diesem Volumen hin durch einen Strahl appliziert wird. Insbesondere wird dieser Bruchteil der Dosis auf dem Weg zum Zielvolumen, das heißt bei $x < x_0$ typischerweise im gesunden Gewebe appliziert und ist deshalb zu minimieren. Eine Möglichkeit, um diese Dosis zu minimieren, ist es, anstatt eines Strahls zwei Strahlen zu verwenden. Diese zwei Strahlen können unterschiedliche, z.B.

entgegengesetzte Strahlursprünge haben und dementsprechend auf unterschiedlichen Wegen durch den Patienten zu dem Zielvolumen 4 gelangen. Dann wird entlang der jeweiligen Strahlwege immer nur ein kleinerer Bruchteil an Dosis appliziert.

In Fig. 2 befindet sich die Patch-Ebene 1 an der Position $x=x_0$. Der Patch-Strahl 3a ist mit der Vorderseite 10 der Patch-Ebene 1 verknüpft und gelangt in Fig. 2 von links in das Zielvolumen 4. Ein weiterer Patch-Strahl ist nicht grafisch indiziert, kann aber z. B. von der entgegengesetzten Seite, in Fig. 2 von rechts, zum Zielvolumen 4 gelangen.

Zum Beispiel mittels eines Behandlungs-Planungssystems 208 kann der Patch-Ebene 1, bzw. dem Patchstrahl 3a, eine Untervolumen-Gesamtstrahlendosis zugeordnet werden. Die Untervolumen-Gesamtstrahlendosis $D_o$ wird durch einen Bruchteil $F_o$ der Gesamtstrahlendosis $D_{pr}$ in dem einer der beiden Seiten einer Patch-Ebene zugewandten Untervolumen definiert. In dem Beispiel von Fig. 2 ist die linke Seite des Untervolumens 4, das heißt die Seite bei $x < x_1$ (der Vorderseite 10 der Patch-Ebene 1 zugewandt) mit einer Untervolumen-Gesamtstrahlendosis assoziiert, die sich ergibt zu

$$D_o = F_o D_{pr}.$$

Hierbei wird betont, dass immer noch innerhalb dieses Untervolumens für $x < x_1$ die Gesamtstrahlendosis $D_{pr}$ appliziert wird. Dies erfolgt aber nicht durch den Patchstrahl 3a alleine (dieser appliziert nur die Untervolumen-Gesamtstrahlendosis $D_o$), sondern durch weitere Strahlen, die z. B. der Rückseite 11 der Patch-Ebene 1 zugeordnet sind. Diese applizieren dann den fehlenden Bruchteil $(1-F_o)D_{pr}$ der Gesamtstrahlendosis $D_{pr}$.

Hierbei kann die Untervolumen-Gesamtstrahlendosis $D_o$ oder $D_r$ jeweils aus der verschriebenen Gesamtstrahlendosis $D_{pr}$ durch Multiplikation mit dem Vorderseiten bzw. Rückseiten Dosisbruchteil $F_o$ bzw. $F_r$, der im Behandlungsplan festgelegt wird, errechnet werden:

$$D_{o/r} = F_{o/r} D_{pr} .$$

Hierbei ist die jeweilige Untervolumen-Gesamtstrahlendosis $D_o$ oder $D_r$ diejenige Dosis, die durch einen Strahl oder eine Anzahl von Strahlen, die mit der dem jeweiligen Untervolumen zugewandten Seite der Patch-Ebene verknüpft sind, appliziert wird.

Es ist insbesondere möglich, entlang der Richtung x (d.h. der Strahlrichtung des Partikelstrahls 3a in Fig. 2) für verschiedene Punkte die jeweilig applizierte Teilstrahlendosis zu berechnen.

$$D_i = c_{pp} \frac{w_i}{\sum_{j=1}^{n} w_j} D_{pr} \quad ;$$

$$1 - F_{o/r} \le c_{pp} \le F_{o/r} .$$

n bezeichnet die Anzahl der mit der gleichen Seite, in obigem Fall mit der Vorderseite 10 der Patch-Ebene 1, verknüpften Strahlen.

Hierbei ist der Faktor $c_{pp}$ ein erster Lokalwichtungsfaktor, welcher den lokalen Bruchteil der Gesamtstrahlendosis $D_{pr}$ bezeichnet.

Die ersten Lokalrichtungsfaktoren $c_{pp}$ unterscheiden sich hierbei insbesondere in den unterschiedlichen Seiten der Patch-Ebene zugewandten Untervolumina und innerhalb der Patch-Ebene auch als Funktion des Ortes x. Insbesondere ist es möglich, innerhalb der Patch-Ebene, wie es in Fig. 2 dargestellt ist, einen graduellen Übergang in der lokal applizierten Dosis mittels eines graduellen Übergangs der ersten Lokalrichtungsfaktoren $c_{pp}$ zu erreichen.

Im Fall des in Fig. 2 dargestellten Ausführungsbeispiels ist das nur ein Strahl mit der Vorderseite 10 verknüpft, das heißt n=1. Es ist aber insbesondere möglich, n>1 zu wählen. Dies kann von Vorteil sein, wenn unterschiedliche Strahlursprünge für Strahlen, die mit derselben Seite einer Patch-Ebene verknüpft sind, gewünscht sind.

$w_i$ stellt sogenannte Strahlwichtungsfaktoren dar. Die Strahlwichtungsfaktoren $w_i$ definieren den relativen Beitrag verschiedener Strahlen zur Untervolumen-Gesamtstrahlendosis.

Zum Beispiel ist es möglich, verschiedene Strahlen mit unterschiedlichen Strahlwichtungsfaktoren $w_i$ zu haben, die dann gemäß der jeweiligen Strahlwichtungsfaktoren nur einen bestimmten Bruchteil der Untervolumen-Gesamtstrahlendosis applizieren. Dadurch ist es z. B. möglich, bei mehreren mit einer bestimmten Seite einer Patch-Ebene verknüpften Strahlen, verschiedenen Strahlen unterschiedliche Dosen zuzuordnen und dementsprechend die auf dem Weg zum Zielvolumen in gesundem Gewebe applizierte Dosis dosimetrisch zu optimieren. Dementsprechend tragen sowohl alle Strahlen, die mit einer Seite der Patch-Ebene verknüpft sind, zu der jeweiligen Untervolumen-Gesamtstrahlendosis $D_o$ und $D_r$ bei. Weiterhin zeigt die Strahlenwichtung mittels Wichtungsfaktoren $w_i$ den erwarteten Effekt auf die Dosisverteilung, das heißt die Strahlengewichte tragen relativ nur zu der verknüpften Seite der Patch-Ebene bei.

Hierbei sollte beachtet werden, dass für den Fall, dass sich die Summation aus Gleichung 2 über alle Strahlen erstrecken würde, die mit dem Zielvolumen 4 verknüpft sind, die jeweilige Untervolumen-Gesamtstrahlendosis $D_o$ und $D_r$ durch die Strahlengewichte $w_i$ modifiziert werden würde. Dies ist nicht erwünscht.

Im Allgemeinen kann der erste Lokalwichtungsfaktor $c_{pp}$ jede Funktion in Abhängigkeit vom relativen Abstand zu einer Patch-Ebene sein. Der in Fig. 1 dargestellte graduelle Übergang in Form einer Intensitätsrampe kann für bestimmte klinische Anwendungen relevant sein.

Insbesondere sollte klar sein, dass für den Fall, dass sowohl Patch-Ebenen als auch Spalt-Ebenen vorhanden sind, die Gleichung 2 verallgemeinert werden muss. Dies wird weiter untenstehend erläutert. Zunächst soll aber in der nachfolgenden Fig. 3, der Fall der Spalt-Ebenen isoliert diskutiert werden.

In Bezug auf Fig. 3 wird nachfolgend ein Ausführungsbeispiel beschrieben, bei dem nur Spalt-Ebenen verwendet werden, um die Applikation einer Gesamtstrahlenleistung in einem Zielvolumen sicherzustellen. Insbesondere kann in bestimmten klinischen Fällen das gewünschte Zielvolumen bzw. der erwünschte Strahl größer sein, als hardwareseitig geliefert werden kann. Deshalb muss der Strahl (der "Spaltstrahl") in mehrere Unterstrahlen aufgespaltet werden und z. B. der Patient zwischen den verschiedenen Bestrahlvorgängen der verschiedenen Unterstrahlen bewegt werden. Die Unterstrahlen sollten zu einem bestimmten benutzerdefinierbaren Grad überlappen, um lokale Abweichungen von der beschriebenen Gesamtdosis in den Übergangsbereichen, z. B. aufgrund von Ungenauigkeiten der Positionierung des Patienten, ("hot spots" und "cold spots") zu vermeiden.

Im Folgenden wird in Bezug auf Fig. 3 eine Aufspaltungsstrategie für Strahlen beispielhaft in dem Fall einer eindimensionalen Spaltung des Strahls gezeigt. Es sollte verstanden werden, dass die Aufspaltung des Strahls auch in anderen beziehungsweise bei Mehrfachaufspaltung in verschiedenen Richtungen erfolgen kann, z.B. einen beliebigen Winkel zur in Fig. 3 dargestellten Spaltebene, sowohl innerhalb der in Fig. 3 dargestellten Zeichenebene, also auch senkrecht zu der Zeichenebene.

L soll im Folgenden die Länge einer Schnittebene durch das Zielvolumen bezeichnen. Das Zielvolumen bezeichnet z.B. die Größe eines Tumors und optional einen umgebenden Positionierungspuffer (sog. "expansion margins"). Innerhalb des Positionierungspuffers können Strahlen und z.B. Spaltebenen platziert werden, wodurch es möglich sein kann, die Dosiskonformität im Tumor zu erhöhen.

Die maximal von einem Strahl zugängliche Länge sei mit $l_{max}$ bezeichnet und die benötigte Länge der Überlappung zwischen zwei benachbarten Strahlen sei mit d bezeichnet. Als weitere Annahme sei $l_{max} > D$ und $L > l_{max}$. $l_i$ ist die resultierende Länge eines Strahls i, wobei $l_i$ immer kleiner $l_{max}$ ist. Die maximale Strahllänge $L_{max}$, die man aus der Kombination von n Strahlen erhalten kann, ist dann

$$L_{max} = n \cdot l_{max} - (n-1) \cdot d \ .$$

Deshalb ist die Anzahl von Strahlen, die benötigt wird, um das Ziel gleichförmig mit Dosis zu applizieren,

$$n = \left\lceil \frac{L-d}{l_{max} - d} \right\rceil .$$

Hierbei bezeichnet der Operator ⌈ ⌉ das Aufrunden bzw. die sogenannte "ceiling" Funktion. Normalerweise wird L nicht genau solche Werte annehmen, dass genau n Strahlen der Länge $l_{max}$ mit Überlappung von genau d in die Länge L hineinpassen. Deshalb kann die Position der Strahlen-Isozentren variiert werden und dementsprechend optimiert werden. Um sicherzustellen, dass die verschiedenen Strahlen nicht stark unterschiedliche Größen aufweisen, bzw. nicht ein Strahl sehr klein ist und die restlichen Strahlen ein sehr großes Zielvolumen bedienen, kann die Größe $l_i$ der Strahlen I gleichmäßig als

$$l_i \equiv const_{\forall i} = l = \frac{L+(n-1)d}{n}\;; \quad \text{wo} \quad l \le l_{\max}\,,$$

berechnet werden. Indem die linke Seite dieser Gleichung als ein Ursprung verwendet werden kann, kann das Isozentrum eines jeden Strahls darauf basierend relativ zu diesem Ursprung berechnet werden, als

$$x_i = (i+0.5)l - id\;; \quad 0 \le i \le n-1\,.$$

Während im obigen Fall eine spezifische Anordnung des Zielvolumens im Koordinatensystem gewählt wurde, sind jedoch auch andere Anordnungen möglich. Dann kann es nötig sein, einen Positionsvektor zum Anfang des Zielvolumens hinzu zu addieren.

Die Position $s_j$ jeder Spalt-Ebene j kann darauf basierend berechnet werden als

$$s_j = j(l-d) + \frac{d}{2}\;; \quad 1 \le j \le n-1\,.$$

Es sollte beachtet werden, dass diese Gleichungen derart definiert sind, dass die Nummerierung der Strahlen mit i=0 beginnt. Hingegen beginnt die Nummerierung der Spalt-Ebenen mit j=1.

Weiterhin wird als Größe x eine Koordinate innerhalb der jeweiligen Spalt-Ebene bezeichnet, wohingegen die Koordinate x im Ausführungsbeispiel der Fig. 2 (Patch-Ebene) eine Koordinate senkrecht zu der jeweiligen Patch-Ebene bezeichnet hat.

Wie aus Fig. 3 ersichtlich ist, kann dies z. B. darin resultieren, dass ein bestimmtes Zielvolumen 4 durch drei Strahlen 3a, 3b, 3c bestrahlt wird. In Fig. 3 sind die Strahlen senkrecht zur Figurenebene orientiert. Das Zielvolumen 4 im Ausführungsbeispiel der Fig. 3 ist so groß, dass ein einzelner Strahl aufgrund von hardwareseitigen Limitationen die Dosis nicht gleichmäßig über dieses Zielvolumen, das heißt ein Querschnitt des Zielvolumens, applizieren kann. Eine mögliche hardwareseitige Beschränkung ist z. B. die maximale Öffnung des Lamellenfilters oder die maximal mögliche Ablenkung eines Partikelstrahls, wie in Bezug auf Fig. 1 beschrieben. Das maximale Gesichtsfeld 7 der Strahlen ist in Fig. 3 gepunktet dargestellt. Die Größe des Zielvolumens L ist hierbei größer als $l_{max}$, wodurch es nötig sein kann, den Strahl durch Spalt-Ebenen aufzuspalten. Deshalb ist es notwendig, die Bestrahlung bzw. Applizierung der Dosis in drei Stufen durch drei Strahlen 3a, 3b, 3c vorzunehmen. Zwischenzeitlich muss entweder das Zielvolumen 4 derart verfahren werden, dass die unterschiedlichen Strahlen unterschiedliche Bereiche des Zielvolumens erreichen können oder die Strahlenerzeugungsvorrichtung muss gegenüber dem Zielvolumen entsprechend bewegt werden. Im Beispiel von Fig. 3 werden drei Strahlen 3a, 3b, 3c verwendet, um jeweils eine Gesamtdosis zu applizieren. Die Isozentren 5 der verschiedenen Strahlen weisen gegeneinander gleiche Abstände auf. Dies ist durch obenstehende Berechnung erreicht. Hierzu sei angemerkt, dass es für Patch-Ebenen nicht notwendig ist, dass verschiedene Strahlen, die jeweils mit einer Patch-Ebene verknüpft sind, unterschiedliche Isozentren aufweisen. Die Positionen der zwei Spalt-Ebenen sind durch deren Zentren 2 markiert. Die erste Spalt-Ebene befindet sich an der Koordinate l-d+d/2, die zweite Spalt-Ebene befindet sich an der Koordinate 2(l-d)+d/2.

Wie aus Fig. 3 weiterhin ersichtlich ist, weisen die beiden Spalt-Ebenen eine endliche Dicke d auf. Die Spalt-Ebenen werden durch Vorderseiten 10 und Rückseiten 11 begrenzt. Es ist z. B. möglich, wie nachfolgend in Bezug auf Fig. 4 beschrieben, innerhalb der Dicke einer Spalt-Ebene, einen graduellen Verlauf der verschiedenen applizierten Teilstrahlendosen $D_i$ zu gewährleisten. Dadurch kann es möglich sein, lokale Abweichungen von der Gesamtstrahlendosis $D_{pr}$ zu verhindern. Solche Abweichungen können z. B. aufgrund ungenauer Positionierung zwischen der Applizierung von zwei Strahlen geschehen. Dies wird in Bezug auf Fig. 4 näher diskutiert.

In Fig. 4 ist gezeigt, wie durch einen linearen örtlichen Verlauf der Teilstrahlendosis $D_i$ der beiden Strahlen 3a und 3b sowohl gewährleistet werden kann, dass in jedem Bereich, das heißt für alle Positionen x die Gesamtstrahlendosis $D_{pr}$ appliziert wird, und dass lokale Abweichungen von der Gesamtstrahlendosis $D_{pr}$ vermieden werden können. Hierbei wird die Teilstrahlendosis $D_i$ des Strahls 3a im Bereich zwischen x=l-d und x=l graduell von der maximalen Teilstrahlendosis im Fall von Fig. 4, $D_i = D_{pr}$, zum minimalen Wert der Dosis, das heißt $D_i = 0$ variiert. Dies kann mittels linearer Interpolation erreicht werden. Für die Strahlen 3a und 3b bedeutet dies

$$D_1 = \begin{cases} D_{pr} & ; x \le l-d \\ 0 & ; x > l \\ D_{pr} - \frac{D_{pr}}{d}(x-l+d) & ; x \in [l-d, l] \end{cases}$$

und

$$D_2 = \begin{cases} D_{pr} & ; x > l \\ 0 & ; x \le l-d \\ D_{pr} - D_1 & ; x \in [l-d, l] \end{cases},$$

wobei $D_1$ die Teilstrahlendosis des Strahls 3a bezeichnet und $D_2$ die Teilstrahlendosis des Strahls 3b bezeichnet. Es ist dann möglich, diese zwei Gleichungen derart umzuformulieren, dass Lokalwichtungsfaktoren $c_{sp,1}$ und $c_{sp,2}$ für die zwei Strahlen 3a und 3b in Bezug auf die Spalt-Ebene erhalten werden. Dann ergibt sich

$$\begin{aligned} D_1 &= c_{sp,1} \cdot D_{pr} \\ D_2 &= c_{sp,2} \cdot D_{pr} \end{aligned} \quad (1)$$

Diese Gleichungen lassen sich direkt auf jedes Dosisberechnungsverfahren anwenden, in dem eine lineare Abhängigkeit zwischen den bestrahlten Intensitäten und dem applizierten Dosen gegeben ist. Für herkömmliche Behandlungsplanung mit Photonen, Elektronen und anderen Teilchen mit konstanter radiobiologischer Wirksamkeit wird diese Voraussetzung erfüllt. Für jede nicht-lineare Abhängigkeit zwischen Intensität und applizierter Dosis, wie das z.B. für die biologische Dosis von Schwerionen der Fall ist, können die $c_{sp}$-Faktoren angepasst werden, um die biologische Wirksamkeit eines Strahls mit in Betracht zu ziehen.

Voranstehend wurde in Bezug auf die Ausführungsbeispiele, welche in Fig. 3 und Fig. 4 dargestellt wurden, die Anordnung und Dosierung von Strahlen, die mittels einer Spalt-Ebene verknüpft sind, erläutert. Insbesondere wurde in Fig. 3 (z. B. im Gegensatz zu Fig. 2) eine Aufsicht auf die zwei Spalt-Ebenen 2 dargestellt. Das bedeutet, dass die Orientierung der Strahlen in Fig. 3 in die Figurenebene hinein oder aus der Figurenebene heraus orientiert ist. Hingegen ist die Orientierung des Strahls in Fig. 2 innerhalb der Zeichenebene. Ein solcher Fall ist auch in der Fig. 5 dargestellt, in welcher nachfolgend ein Fall beschrieben wird, in dem sechs Spalt-Ebenen 2a-2g vorhanden sind, welche die Positionierung und Dosierung von insgesamt acht Strahlen 3a-3h steuern. Wie aus Fig. 5 ersichtlich ist, treffen hierbei vier Strahlen 3a bis 3d in Fig. 5 von oben auf das Zielvolumen 4 (gestrichelt dargestellt) auf. Weitere vier Strahlen 3e bis 3h treffen in Fig. 5 von unten dargestellt auf das Zielvolumen 4 auf. Hierbei sind die von oben auftreffenden Strahlen 3a bis 3d durch die Spalt-Ebenen 2a bis 2c positioniert, während die Strahlen 3e bis 3h durch die Spalt-Ebenen 2e bis 2g positioniert sind. Insbesondere haben z. B. die Spalt-Ebenen 2a und 2e unterschiedliche Positionen entlang des Zielvolumens. Dies ist möglich, da die Spalt-Ebenen 2a bis 2c für die Strahlen 3a bis 3d völlig unabhängig von den Spalt-Ebenen 2e bis 2g für die Strahlen 3e bis 3h z.B. mittels des Behandlungs-Planungssystem aus Fig. 1 festgelegt werden können. Nichtsdestotrotz wird im gesamten Zielvolumen 4 überall die gleiche Gesamtstrahlendosis $D_{pr}$ appliziert. Dies

geschieht durch eine Addition der Teilstrahlendosen $D_i$ der verschiedene Strahlen.

Im Allgemeinen kann für den Fall, dass zwei Strahlen, welche aus unterschiedlichen Richtungen auf ein Zielvolumen treffen, eine Spaltung mittels Spalt-Ebenen vorgenommen werden muss, diese Spaltung unabhängig voneinander für die zwei Strahlen vorgenommen werden. Deshalb sind mit einer Spalt-Ebene zwei (gespaltene) Strahlen verknüpft, wenn die Spaltung nur in einer Richtung vorgenommen wird (eindimensional). Im Falle von zweidimensionaler Strahlspaltung für große Zielvolumina kann die Anzahl von mit einer Spalt-Ebene verknüpften Strahlen zunehmen. Hierbei kann es insbesondere vorkommen, dass Spalt-Ebenen, welche zu unterschiedlichen ursprünglichen Strahlen (ungespaltenen Strahlen) gehören, überlappen.

Nachfolgend wird in Bezug auf die in Fig. 6 und Fig. 7 dargestellten Ausführungsbeispiele näher erläutert, wie das Positionieren zwischen der sequenziellen Anwendung zweier, z. B. durch eine Spalt-Ebene getrennte, Strahlen erfolgen kann. In Fig. 6 wird eine Möglichkeit dargestellt, in der ein Liegetisch 207 in Bezug auf den Strahlursprung U rotiert wird. Hingegen wird in Fig. 7 eine Möglichkeit dargestellt, in der der Liegetisch 207 in Bezug auf den Strahlursprung U bewegt wird. Wie aus Fig. 6 ersichtlich ist, erfolgt bei einer Rotation des Liegetisches 207 um den Strahlursprung die Bestrahlung eines Zielvolumens aus demselben Strahlursprung U. Hingegen wird, wenn der Liegetisch 207 sich in Bezug auf den Strahlursprung U bewegt, der Strahlursprung U aus dem Referenzkoordinatensystem des Liegetisches 207 unter zwei verschiedenen Positionen erscheinen. Dies ist aus Fig. 7 ersichtlich. Sowohl in Fig. 6 als auch in Fig. 7 werden zwei Strahlen 3a und 3b zur Applizierung einer Dosis in einem Zielvolumen verwendet. In beiden Fällen ist eine Spalt-Ebene 2 vorhanden. Die Spalt-Ebene 2 hat eine Vorderseite 10 und eine Rückseite 11. Vorderseite und Rückseite 10, 11 begrenzen eine Überlappungsregion, die in den Fig. 6 und 7 gestrichelt dargestellt ist.

Wird der Liegetisch zwischen der Anwendung von zwei Strahlen bewegt, so kann dies folgendes bewirken: Jede Kollisionskontrolle in einem Bestrahlungsplan (siehe Fig. 1) muss wiederholt werden, um sicherzustellen, dass keine Strahlendosis in z. B. gefährdeten Organen passiert. Dies kann darin resultieren, dass ein Strahl, welcher in einer vorhergehenden Kollisionskontrolle als anwendbar befundet wurde, nach Bewegung des Liegetisches zurückgewiesen wird.

Für divergierende Strahlen, d.h. Strahlen deren Durchmesser mit zunehmendem Abstand zum Strahlursprung U zunimmt, hat die Bewegung des Liegetisches die Folge, dass neben einer lateralen Translation des Liegetisches auch eine Rotation, Verkippung oder Verdrehung des Liegetisches möglich ist. Dies ist näher in Fig. 6 dargestellt. Insbesondere in dem Fall von besonders divergenten Strahlen, das bedeutet die Quell-Ziel-Entfernung (FID) ist sehr klein, kann das Rotieren des Tisches 207 die Strahldivergenz kompensieren. Die Rotation des Liegetisches ist mittels einer gestrichelten Linie dargestellt. Zwei Strahlen 3a und 3b werden verwendet, um eine Dosis innerhalb eines Zielvolumens zu applizieren. Insbesondere weisen die Strahlen eine Überlappung auf. Die Größe der Überlappung nimmt mit zunehmendem Abstand zum Strahlursprung U zu, was an der Divergenz der beiden Strahlen liegt. Eine Spalt-Ebene 2 befindet sich innerhalb dieses Überlappungsbereiches. Die Spalt-Ebene 2 ist durch eine Vorderseite 10 und eine Rückseite 11 gekennzeichnet.

Durch das Rotieren des Liegetisches kann die Strahldivergenz kompensiert werden. Durch die Rotation des Liegetisches wird nämlich auch der Überlappungsbereich als Funktion zunehmender Entfernung zum Strahlursprung U größer. Das heißt, dass über die Tiefe eines Zielvolumens immer sichergestellt werden kann, dass die Überlappung der Strahlen ausreicht, um innerhalb einer Spalt-Ebene 2 einen graduellen Übergang der Strahlen 3a und 3b zu gewährleisten.

Jedoch kann wenn, die Überlappung zwischen den Strahlen durch die Tiefe des Zielvolumen hindurch gleich oder größer als die Dicke der Spalt-Ebene ist, das Verfahren des Liegetisches zwischen dem Anwenden zweier aufeinanderfolgender, durch eine Spalt-Ebene verknüpften Strahlen, auch in Form einer Translation erfolgen. Dann ist keine Verkippung oder Drehung des Liegetisches nötig. Ein solcher Fall ist in Fig. 7 gezeigt. In Fig. 7 oben wird die Anwendung von zwei Strahlen 3a und 3b hintereinander gezeigt, wobei zwischen der Anwendung der beiden Strahlen 3a und 3b der Liegetisch 207 verfahren wurde. Deshalb haben die Strahlen 3a und 3b in Fig. 7 oben unterschiedliche Strahlenursprünge U. Wie schon in Bezug auf Fig. 6 diskutiert, weisen die Strahlen 3a und 3b z.B. aufgrund eines geringen Abstandes zum Strahlursprung U eine hohe Divergenz auf. Das bedeutet, dass sich über die Tiefe des Zielvolumens 4 der Durchmesser des Strahls ändert. Befindet sich nun innerhalb des Zielvolumens 4 eine Spalt-Ebene 2, welche durch eine Vorderseite 10 und eine Rückseite 11 gekennzeichnet ist, wobei ein Strahl 3a mit der Vorderseite 10 verknüpft ist und ein weiterer Strahl 3b mit der Rückseite 11 verknüpft ist, so muss sichergestellt sein, dass die Dicke der Spalt-Ebene nicht die minimale Überlappung der beiden Strahlen 3a und 3b überschreitet. Das bedeutet insbesondere, dass die Dicke der Spalt-Ebene 2 durch den Punkt beschränkt ist, der dem Strahlursprung U am nächsten ist. Dies ist vergrößert in Fig. 7 unten gezeigt, wo ersichtlich ist, dass die Dicke der Spalt-Ebene 2 innerhalb eines Zielvolumens 4 immer innerhalb des Überlapps zwischen den beiden Strahlen 3a und 3b liegen muss. Dann ist es möglich, dass innerhalb der Dicke der Spalt-Ebene, das heißt zwischen Vorderseite 10 und Rückseite 11 der Spalt-Ebene 2, ein gradueller Übergang der Teilstrahlendosen, die zu den zwei Strahlen 3a und 3b gehörig sind, zu gewährleisten. Wie aus Fig. 7 untenstehend ersichtlich ist, liefert linksseitig der Spalt-Ebene 2 der Strahl 3a 100% der Gesamtstrahlendosis (und der Strahl 3b 0% der Gesamtstrahlendosis), während dies auf der rechtsseitigen Seite der Spalt-Ebene 2 genau andersherum ist.

So lange wie der Strahlüberlapp groß genug ist, um die gesamte Spalt-Ebene innerhalb des Zielvolumens abzudecken (das heißt, dass z. B. die Strahlzielpunkte in dem Fall eines gescannten Partikelstrahls innerhalb der gesamten Spalt-Ebene für beide Strahlen positioniert werden können müssen), können gute Optimierungsergebnisse erzielt werden.

Nachfolgend wird in Bezug auf Fig. 8 ein Fall diskutiert, bei dem eine Strahlentherapieanlage nicht beliebig geringe Intensitäten eines Partikelstrahls 3a, 3b zur Verfügung stellen kann. Dies ist insbesondere dann relevant, wenn z. B. wie in Bezug auf die Fig. 3 und 4 diskutiert, ein Übergang zwischen der durch einen Partikelstrahl applizierten Dosis zwischen einem maximalen und einem minimalen Wert über die Schichtdicke einer Spalt-Ebene 2 hinweg verwendet wird. Es ist z.B. möglich, dass ein linearer Zusammenhang zwischen applizierten Dosen und Intensitäten der Strahlen besteht. Jedoch kann es auch notwendig sein, die radiobiologische Wirkung eines Strahls bekannter Intensität zu kennen, um eine Dosis zuordnen zu können. Hierbei kann es sein, das eine Strahlentherapieanlage ein minimales Limit der Strahlenintensität aufweist. Dieses sei als $I_{min}$ bezeichnet. Wenn zwei Strahlen mit Intensitätsprofilen $I_1$ und $I_2$ in Betracht gezogen werden, so muss, wenn entweder $I_1$ oder $I_2$ kleiner als $I_{min}$ werden, die Intensitätsverteilung angepasst werden, so dass alle Intensitäten größer $I_{min}$ sind oder einige von diesen Intensitäten gleich Null sind. Letzteres ist nur der Fall, wenn $I_0$ kleiner $2I_{min}$ ist, was in klinischen Fällen keine Anwendung findet, weswegen es möglich sein kann diese Option in diesem Kontext nicht zu betrachten Wenn Imin besonders klein wird, kann es möglich sein, auf diese Intensitätskorrektur zu verzichten.

Der Übergang zwischen der lokal applizierten Teilstrahlendosis zweier Strahlen z. B. innerhalb (d.h. zwischen einer Vorderseite 10 und einer Rückseite 11) einer Spalt-Ebene 2 oder auch innerhalb einer Patch-Ebene 1 ist in Fig. 8 dargestellt, wobei eine minimale Intensität $I_{min}$ in Betracht gezogen wurde. Nachdem diese Dosisverteilung leicht aufgrund der Intensitätsänderung verändert wird, sollte eine erneute Berechnung des Behandlungsplans durchgeführt werden.

In Bezug auf Fig. 9 wird ein Fall betrachtet, in dem zwei Patch-Ebenen 1a und 1b existieren. Für den Fall, dass ein Strahl zu mehr als einer Patch-Ebene zugeordnet ist, kann die Komplexität des Zuordnens des richtigen Strahls an die richtige Seite einer der jeweiligen Patch-Ebenen für den Benutzer zunehmen. In diesem Fall wird vorgesehen, dass das System, z. B. das Behandlungs-Planungssystem aus Fig. 1, den Prozess des Zuordnens eines Strahls zu einer Seite einer Patch-Ebene unterstützt. Es kann dann insbesondere vorteilhaft sein, über ein entsprechendes Benutzer-Interface sicherzustellen, dass der Benutzer anstatt von Seiten einer bestimmten Patch-Ebene, ein entsprechendes Untervolumen auswählen kann. Dies ist in Bezug auf Fig. 9 illustriert. Die zwei Patch-Ebenen 1a und 1b teilen das Zielvolumen 4 in vier Untervolumen 6a bis 6d auf. Es ist dann für den Benutzer schwierig, die verschiedenen Strahlen 3a bis 3d den entsprechenden Seiten der verschiedenen Patch-Ebenen zuzuordnen. Einfacher ist es, wenn der Benutzer nicht eine Seite, z. B. die Vorder- oder Rückseite, einer bestimmten Patch-Ebene zur Verknüpfung des Strahles auswählen muss, sondern entsprechend nur eines der unterschiedlichen Untervolumina 6a bis 6d auswählen kann. Das System kann in einem solchen Fall die Zuordnung zu den verschiedenen Vorder- und Rückseiten der verschiedenen Patch-Ebenen 1a und 1b automatisch vornehmen. Der entsprechende Ansatz kann auch für eine 3D-Problematik erweitert werden.

In den voranstehenden Fig. 2 bis 9 wurden Fälle diskutiert, in denen jeweils entweder Patch-Ebenen oder Spalt-Ebenen verwendet wurden, um die Positionierung und die Dosierung von Partikelstrahlen zu steuern. Solche Fälle können als Teil eines Solche Fälle können als Teil eines Ausführungsbeispiels gemäß der vorliegenden Erfindung verwendet werden- Im Folgenden soll bezugnehmend auf Fig. 1 ein Fall diskutiert werden, in dem das Behandlungs-Planungssystem 208 derart konfiguriert ist, dass eine Kombination aus Spalt-Ebenen und Patch-Ebenen verwendet wird. Insbesondere ist dann die Verarbeitungsvorrichtung 205 konfiguriert, die einzelnen Teilstrahldosen $D_i$ der verschiedenen Strahlen, die jeweils mit Patch-Ebenen oder Spalt-Ebenen verknüpft sind, richtig zu berechnen. Richtig berechnen heißt in diesem Zusammenhang, dass an jedem Punkt innerhalb eines Zielvolumens 4 sichergestellt ist, dass die durch alle Strahlen applizierte Teilstrahlendosis gleich der Gesamtstrahlendosis $D_{pr}$ ist. Dies soll unter Berücksichtigung von Strahlwichtungsfaktoren $w_i$, wie sie z. B. in Bezug auf die Fig. 2 erläutert wurden, geschehen. Weiterhin geschieht dies unter Verwendung von ersten und zweiten Lokalwichtungsfaktoren $c_{pp}$ und $c_{sp,i}$, welche die jeweiligen Teilstrahlendosen als Funktion der Positionierung in Bezug auf entweder Patch-Ebenen oder Spalt-Ebenen definieren.

Für Spalt-Ebenen ergibt sich die Teilstrahlendosis $D_i$ z. B. Bezug nehmend auf die Beschreibung von Fig. 5 zu

$$D_i = \frac{w_i \prod_k c_{sp,i,k}}{\sum_{j=1}^{n} w_j \prod_l c_{sp,j,l}} D_{pr} \quad \text{wo} \quad 0 \le c_{sp,i/j} \le 1 \; .$$

n bezieht sich hier auf die Gesamtzahl aller Strahlen, die das Zielvolumen bestrahlen sollen. Die $c_{sp}$, d.h. die zweiten Lokalwichtungsfaktoren werden aus Gleichung 1 erhalten. Derart wird sichergestellt, dass nur Strahlen in Betracht gezogen werden, welche eine Dosis an einem bestimmten Punkt in Bezug auf die Spalt-Ebenen beitragen. Das Produkt erstreckt sich hierbei über alle zweiten Lokalwichtungsfaktoren $c_{sp}$ derjenigen Spalt-Ebenen, mit denen der betrachtete Strahl verknüpft ist. Derart kann auch das Überlappen von Spalt-Ebenen, welchen derselbe Strahl zugeordnet ist, berücksichtigt werden.

Werden nun sowohl Spalt-Ebenen als auch Patch-Ebenen in demselben Behandlungsplan verwendet, so wird folgende Formel verwendet:

$$D_i = \frac{w_i \prod_k c_{sp,i,k}}{\sum_{j=1}^{n} w_j \prod_l c_{sp,j,l}} c_{pp} D_{pr} \, . \qquad (2)$$

Hier werden die zweiten Lokalwichtungsfaktoren, das heißt die $c_{sp,i}$-Faktoren der Spalt-Ebenen dazu verwendet, die Strahlwichtungsfaktoren $w_i$ zu wichten. Dies ist der Fall, da die verschiedenen zweiten Lokalwichtungsfaktoren $c_{sp}$ Werte zwischen 0 und 1 annehmen können. Insbesondere erstreckt sich die Berechnung der Gleichung 2 immer nur über diejenigen n Strahlen, die einer bestimmten Patch-Ebenen-Seite, für die der erste Lokalwichtungsfaktor $c_{pp}$ definiert ist, zugeordnet sind.

Dadurch, dass die Berechnung der verschiedenen Strahlendosen isoliert für eine einzelne Seite einer Patch-Ebene (das heißt jeweils nur für einen ersten Strahlwichtungsfaktor $c_{pp}$) vorgenommen wird, kann sichergestellt werden, dass in jedem durch eine Patch-Ebene definierten Untervolumen die entsprechende Untervolumen-Gesamtstrahldosis $D_o$ bzw. $D_r$ erfüllt wird. Dadurch kann weiterhin sichergestellt werden, dass durch Addition der verschiedenen Untervolumen-Gesamtstrahlendosen, das heißt der Untervolumen-Gesamtstrahlendosen, welche durch Strahlen, die mit entgegengesetzten Seiten einer Patch-Ebene verknüpft sind, appliziert werden, die Gesamtstrahlendosis $D_{pr}$ erbracht wird.

Insbesondere werden die ersten und zweiten Lokalwichtungsfaktoren $c_{pp}$ und $c_{sp}$ nicht identisch verwendet. Das heißt, es wird keine Gleichung in der Form

$$D_i = \frac{w_i \prod_k c_{i,k}}{\sum_{j=1}^{n} w_j \prod_l c_{j,l}} D_{pr} \, , \qquad (3)$$

verwendet. Hierbei entspricht $c_{i,k}$ den Faktoren $c_{sp}$ und $c_{pp}$. Eine solche Gleichung 3 ist zwar mathematisch korrekt formuliert, hat aber den Nachteil, dass die Untervolumen-Gesamtstrahlendosis $D_o$ und $D_r$ nicht mehr konstant sind, da die jeweiligen Teilstrahlendosen $D_i$ auch abhängig von den Strahlgewichten $w_i$ sind.

Es ist auch möglich, Strahlen zu definieren, welche nicht mit einer Patch-Ebene oder einer Spalt-Ebene definiert sind. Dies wird nachfolgend näher anhand von Fig. 10 diskutiert. In Fig. 10 ist ein Fall dargestellt, in dem zwei Strahlen 3b und 3c mit einer Patch-Ebene 1 verknüpft sind. Die Strahlen 3b und 3c erreichen das Zielvolumen 4 aus entgegengesetzten Richtungen (in Fig. 10 aus der linken und rechten Seite). Weiterhin ist ein Strahl 3a vorhanden, welcher nicht mit der Patch-Ebene 1 verknüpft ist. Der Strahl 3a ist auch nicht mit einer Spalt-Ebene verknüpft. Eine solche Konfiguration von Strahlen 3a bis 3c kann verwendet werden, wenn sich das Zielvolumen 4 in der Nähe eines gefährdeten Organs 20 befindet. Hierbei ist kann es insbesondere erstrebenswert sein, dass innerhalb des Organs 20 keine oder nur geringe Dosis appliziert wird, während innerhalb des Zielbereichs 4 eine große Dosis appliziert wird.

Eine Möglichkeit, die Strahlwichtungsfaktoren $w_i$ sowohl für die mit der Patch-Ebene 1 verknüpften Strahlen 3b und 3c und auch für den Strahl 3a, der nicht mit der Patch-Ebene verknüpft ist, derart festzulegen, dass die Gesamtstrahlendosis entsprechend den radiologischen Vorgaben erfüllt wird, ist es, die Strahlgewichte aller mit der Patch-Ebene 1 verknüpfter Strahlen gleich 1 zu setzen. In diesem Sinne wird die Gesamtstrahlendosis $D_{pr}$ zunächst zwischen den Strahlen, die weder mit einer Patch-Ebene noch einer Spalt-Ebene verknüpft sind und den restlichen Strahlen verteilt. Zum Beispiel lässt sich der Patch-Ebene in Fig. 10 dann eine Dosis $D_{pr,pp}$ zuordnen, die gegeben ist als

$$D_{pr,pp} = \frac{1}{1 + \sum_m w_m} D_{pr} \, .$$

Es ist aber natürlich auch möglich, der Patch-Ebene eine Wichtung zuzuordnen, die anders als 1 ist. In dem Fall wird obenstehende Gleichung zu

$$D_{pr,pp} = \frac{1}{w_{pp} + \sum_m w_m} D_{pr} \, .$$

Es ist dann möglich, obenstehende Gleichung 2, welche die Teilstrahlendosis für einen Strahl, der sowohl einer Patch-Ebene als auch einer Spalt-Ebene zugeordnet ist, entsprechend umzuformulieren:

$$D_i' = \frac{w_i \prod_k c_{sp,i,k}}{\sum_{j=1}^{n} w_j \prod_l c_{sp,j,l}} c_{pp} D_{pr,pp} \, . \qquad (4)$$

Hierbei wurde die Gesamtstrahlendosis $D_{pr}$ angepasst. Die angepasste Gesamtstrahlendosis $D_{pr,pp}$ ist nun um den Betrag korrigiert, welcher durch Strahlen appliziert wird, die weder einer Patch-Ebene noch einer Split-Ebene zugeordnet sind. Solchen Strahlen lässt sich entsprechend eine Teilstrahlendosis zuordnen:

$$D_q = \frac{w_q}{1 + \sum_m w_m} D_{pr} \, , \text{ bzw.}$$

$$D_q = \frac{w_q}{w_{pp} + \sum_m w_m} D_{pr} \, . \qquad (5)$$

In Bezug auf Fig. 10 ist illustrativ ein numerisches Beispiel in Betracht zu ziehen. Hierzu soll die Gesamtstrahlendosis $D_{pr}$=60 Gray betragen und die Strahlgewichte $w_i$ sollen sein:

für Strahl 3a: $w_1$ = 5;
für Strahl 3b: $w_2$ = 2;
und für Strahl 3c: $w_3$ = 3.

Der Strahl 3b sei der Vorderseite der Patch-Ebene 1 (linke Seite in Fig. 10) zugeordnet. Diese Seite der Patch-Ebene ist dadurch charakterisiert, dass ihr ein Dosisbruchteil von $F_o$ = 70% zugeordnet ist und ein rückseitiger Dosisbruchteil von $F_r$ = 80% zugeordnet ist. Das heißt, dass ein mit der Vorderseite von der Patch-Ebene 1 verknüpfter Strahl 3b eine Untervolumen-Gesamtstrahlendosis $D_o$ von 70% der (um den von Strahl 3a applizierten Teilstrahlendosis korrigierten) Gesamtstrahlendosis $D_{pr,pp}$ in den der Vorderseite der Patch-Ebene zugewandten Untervolumen appliziert.

Wenn man Gleichung 5 in Betracht zieht, dann ist die durch Strahl 3a applizierte Dosis $D_1$ gegeben durch

$$D_1 = \frac{5}{1+5} 60Gy = 50Gy \; .$$

Weiterhin kann die durch die Strahlen 3b und 3c applizierte Teilstrahlendosis berechnet werden. Dies geschieht getrennt und isoliert für die Vorderseite und die Rückseite der Patch-Ebene 1. Zunächst wird die um die durch Strahl 3a applizierte Teilstrahlendosis korrigierte Gesamtstrahlendosis $D_{pr,pp}$ berechnet. Dazu wird Gleichung 4 verwendet und man erhält:

$$D_{pr,pp} = \frac{1}{1+5} 60Gy = 10Gy \; .$$

Dann ist es möglich, weiterhin die Teilstrahlendosen $D_{2,o}$ und $D_{3,o}$ zu berechnen, die jeweils die Teilstrahlendosen der Strahlen 3b und 3c in der Vorderseite der Patch-Ebene 1 zugeordneten Untervolumen (linksseitig in Fig. 10) bezeichnen. Auch ist es möglich, die entsprechende Teilstrahlendosis für die zwei Strahlen 3b und 3c in dem der Rückseite der Patch-Ebene 1 zugewandten Untervolumen (rechtsseitig in Fig. 10) zu berechnen:

$$\left.\begin{aligned} D_{2,o} &= \frac{2}{2} \cdot 0.7 \cdot 10Gy = 7Gy \\ D_{3,o} &= \frac{2}{2} \cdot 0.3 \cdot 10Gy = 3Gy \end{aligned}\right\} = D_{pr,pp}$$

$$\left.\begin{aligned} D_{2,r} &= \frac{3}{3} \cdot 0.2 \cdot 10Gy = 2Gy \\ D_{3,r} &= \frac{3}{3} \cdot 0.8 \cdot 10Gy = 8Gy \end{aligned}\right\} = D_{pr,pp} \; .$$

Es sollte klar sein, dass das oben rotierte numerische Beispiel lediglich illustrativer Natur ist. Natürlich können die verschiedenen Parameter für die verschiedenen Strahlen in ihrem Zahlenwert angepasst werden.

In dem numerischen Beispiel der Fig. 10 ergibt sich, dass die Gesamtstrahlendosis $D_{pr}$ = 60 Gray sich folgendermaßen auf die verschiedenen Strahlen aufteilt: Strahl 3a appliziert 50 Gray innerhalb des Zielvolumens. In Fig. 10 linksseitig der Patch-Ebene 1 (das heißt in dem Untervolumen, das der Vorderseite der Patch-Ebene 1 zugewandt ist) appliziert der Strahl 3b 7 Gray und der Strahl 3c 3 Gray. Die Summe der von den Strahlen 3b und 3c applizierten Untervolumen-Teilstrahlendosis ergibt 10 Gray. Diese 10 Gray sind die fehlenden 10 Gray, die sich in Summe mit den 50 Gray der durch Strahl 3a applizierten Teilstrahlendosis zu der Gesamtstrahlendosis von 60 Gray summieren. In dem in Fig. 10 rechtsseitig dargestellten Untervolumen (dem Untervolumen, welches der Rückseite der Patch-Ebene 1 zugewandt ist) ist das Verhältnis der durch die Strahlen 3b und 3c applizierten Teilstrahlendosen anders. Der Strahl 3b appliziert nämlich eine Teilstrahlendosis von 2 Gray und der Strahl 3c appliziert eine Teilstrahlendosis von 8 Gray. Nichtsdestotrotz ist die Gesamtstrahlendosis gleich 60 Gray.

In Fig. 11 ist ein Ausführungsbeispiel beschrieben, das dem Ausführungsbeispiel von Fig. 10 ähnlich ist. Wiederum ist eine Patch-Ebene 1 vorhanden. In dem Ausführungsbeispiel von Fig. 11 weist die Patch-Ebene im Gegensatz zu dem Ausführungsbeispiel von Fig. 10 eine endliche Dicke auf. Die Vorderseite 10 ist in Fig. 11 linksseitig dargestellt, während die Rückseite 11 der Patch-Ebene 1 rechtsseitig dargestellt ist. Im Gegensatz zu dem Ausführungsbeispiel der Fig. 10 weist das Ausführungsbeispiel der Fig. 11 keine Strahlen auf, die nicht der Patch-Ebene 1 zugeordnet sind. Insbesondere sind die Strahlen 3a und 3b der Vorderseite der Patch-Ebene 1 zugeordnet und der Strahl 3c der Rückseite der Patch-Ebene. Alle drei Strahlen weisen unterschiedliche Strahlenursprünge auf. Zum Beispiel fällt der Strahl 3a in Fig. 11 von links oben in das Zielvolumen 4 ein. Der Strahl 3b hingegen, der auch mit der Vorderseite 10 der Patch-Ebene 1 verknüpft ist, fällt in Fig. 11 von der linken unteren Seite in das Zielvolumen 4 ein. Es ist nun möglich, unter Verwendung der voranstehenden Gleichungen die jeweiligen Teilstrahlendosen für die verschiedenen Strahlen zu berechnen. Hierbei ist insbesondere zu beachten, dass die Berechnung der Teilstrahlendosen für die Strahlen 3a und 3b getrennt von der Berechnung der Teilstrahlendosis für den Strahl 3c stattfindet. Dies ist der Fall, da die Berechnung immer isoliert für eine Seite einer Patch-Ebene durchgeführt wird und zwar nur für diejenigen Strahlen, die dieser Seite der Patch-Ebene zugeordnet sind. Im Fall von Fig. 11 sind z. B. die Strahlen 3a und 3b der Vorderseite, das heißt der linken Seite, der Patch-Ebene 1 zugeordnet.

Beispielhaft sollen für die Gesamtstrahlendosis $D_{pr}$ = 60 Gray und die Strahlgewichte $w_1$ = 5 (Strahl 3a), $w_2$ = 2 (Strahl 3b), $w_3$ = 3 (Strahl 3c) durchgeführt werden. Die der Patch-Ebene zugeordneten Dosisbruchteile betragen $F_o$ = 70% für das der Vorderseite zugewandte Untervolumen und $F_r$ = 80% für das der Rückseite zugewandte Untervolumen. Das bedeutet, dass die Strahlen, welche mit der Vorderseite der Patch-Ebene verknüpft sind, 70% der Gesamtstrahlendosis in diesem Untervolumen, das heißt dem der Vorderseite der Patch-Ebene 1 zugewandten Untervolumen (linksseitiges Untervolumen in Fig.11) applizieren. Hingegen applizieren die Strahlen, die der Rückseite 11 der Patch-Ebene 1 zugeordnet sind, in dem rückseitigen Untervolumen (rechtsseitiges Untervolumen in Fig. 11) 80% der Gesamtstrahlendosis. Die jeweils fehlenden 30% (Vorderseite) und 20% (Rückseite) der Untervolumen-Gesamtstrahlendosis werden durch die jeweils mit der entgegengesetzten Seite der Patch-Ebenen verknüpften Strahlen appliziert, das heißt in dem Ausführungsbeispiel der Fig. 11 werden in den der Vorderseite 10 der Patch-Ebene zugewandten Untervolumen 20% der Gesamtstrahlendosis durch den Strahl 3c appliziert und 30% der Gesamtstrahlendosis in dem der Rückseite 11 zugewandten Untervolumen der Patch-Ebene 1 durch die Strahlen 3a und 3b.

Die Berechnung gemäß der voranstehenden Gleichung 2 ergibt dann

$$\left.\begin{array}{l}\left.\begin{array}{l}D_{1,o}=0.7\cdot\frac{5}{5+2}60Gy=30Gy\\ D_{2,o}=0.7\cdot\frac{2}{5+2}60Gy=12Gy\end{array}\right\}=42Gy=0.7\cdot 60Gy\\ D_{3,o}=0.3\cdot\frac{1}{1}60Gy=18Gy\end{array}\right\}=D_{pr}$$

$$\left.\begin{array}{l}\left.\begin{array}{l}D_{1,r}=0.2\cdot\frac{5}{5+2}60Gy=8.6Gy\\ D_{2,r}=0.2\cdot\frac{2}{5+2}60Gy=3.4Gy\end{array}\right\}=12Gy=0.2\cdot 60Gy\\ D_{3,r}=0.8\cdot\frac{1}{1}60Gy=48Gy\end{array}\right\}=D_{pr}$$

In Gleichung 2 wurde $c_{sp}$=1 gesetzt, da keine Spalt-Ebene vorhanden ist.

Anhand dieser Gleichungen wird sichergestellt, dass in jedem Punkt innerhalb des Zielvolumens 4 die gleiche Gesamtstrahlendosis (nämlich in dem Ausführungsbeispiel von Fig. 11, $D_{pr}$=60 Gray) appliziert wird. Gleichzeitig werden die relativen Strahlgewichte ($w_1$ = 5, $w_2$ = 2 und $w_3$ = 3 der Strahlen 3a bis 3c) eingehalten. Zum Beispiel ist das Verhältnis von 8,6 Gray zu 3,4 Gray (das heißt der Teilstrahlendosen der Strahlen 3a und 3b in dem der Rückseite der Patch-Ebene zugewandten Untervolumen) durch ein Verhältnis von 5:2 gemäß der Strahlgewichte beschrieben.

In Bezug auf Fig. 12 ist ein Beispiel dargestellt, bei dem sowohl eine Patch-Ebene 1 als auch zwei Spalt-Ebenen 2a und 2b vorhanden sind. Die Patch-Ebene 1 hat eine Vorderseite 10, die in Fig. 12 nach oben orientiert ist, und eine Rückseite 11, die in Fig. 12 nach unten orientiert ist. Der Vorderseite 10 der Patch-Ebene 1 sind die Strahlen 3a und 3b zugeordnet, während der Rückseite 11 der Patch-Ebene 1 die Strahlen 3c und 3d zugeordnet sind. Die Strahlen 3a und 3b treffen aus einer Richtung auf das Zielvolumen 4 auf, die unterschiedlich zu der Richtung der Strahlen 3c und 3d ist.

Ein in Fig. 12 dargestellter Anwendungsfall könnte z. B. sein, dass das Zielvolumen 4 eine zu große Ausdehnung hat, so dass ein einzelner Strahl, der z. B. aus der Richtung der Strahlen 3a und 3b auf das Zielvolumen 4 auftreffen soll, nicht das gesamte Zielvolumen 4 abdecken kann. Mögliche Gründe hierfür sind, dass hardwareseitige Limitierungen nicht erlauben, den Durchmesser eines Strahles so groß zu wählen, dass das Zielvolumen 4 auf einmal erfasst wird. Dann ist es notwendig, zwei Strahlen (Strahlen 3a und 3b in Fig. 12) zu verwenden, welche mittels einer Spalt-Ebene (Spalt-Ebene 2a in Fig. 12) miteinander verknüpft sind. Hierbei ist der Strahl 3a der Vorderseite 10b der Spalt-Ebene 2a zugeordnet und der Strahl 3b der Rückseite 11b der Spalt-Ebene 2a zugeordnet. Entsprechendes gilt für die Strahlen 3c und 3d in Bezug auf die Spalt-Ebene 2b.

Unter Verwendung der voranstehenden Gleichung 2 ist es möglich, die Teilstrahlendosen der verschiedenen Strahlen zu berechnen. Es wird wiederum ein numerisches Beispiel zum besseren Illustration dargelegt. Hierbei sei der Patch-Ebene 1 ein Dosisbruchteil von $F_o$ = 60% in Bezug auf die Vorderseite zugeordnet und ein Dosisbruchteil von $F_r$ = 80% in Bezug auf die Rückseite zugeordnet. Diese Dosisbruchteile wurden bereits in Bezug z. B. auf die voranstehende Fig. 11 ausführlich diskutiert. Die Strahlwichtungsfaktoren für die verschiedenen Strahlen lauten: für Strahl 3a $w_{1.1}$ = 1; für Strahl 3b: $w_{1.2}$ = 2; für Strahl 3c: $w_{2.1}$ = 3; für Strahl 3d: $w_{2.2}$ = 4. Hierbei indizieren die doppelten Indizes auch die Zugehörigkeit zu einer der Spalt-Ebenen 2a und 2b. Die Berechnung der Teilstrahlendosen ergibt dann für die verschiedenen Strahlen:

$$\left.\begin{aligned} D_{1.1,area1.1} &= \frac{1\cdot 1}{1\cdot 1+2\cdot 0}\cdot 0.6\cdot 60Gy = 36Gy \\ D_{1.2,area1.1} &= 0Gy \\ D_{2.1,area1.1} &= \frac{3\cdot 1}{3\cdot 1+4\cdot 0}\cdot 0.4\cdot 60Gy = 24Gy \\ D_{2.2,area1.1} &= 0Gy \end{aligned}\right\} = 60Gy = D_{pr} \ .$$

Es sei angemerkt, dass wiederum die Wahl der verschiedenen numerischen Faktoren rein exemplarisch ist und unterschiedliche numerische Faktoren gewählt werden können.

Weiterhin ist in dem Beispiel der Figur 12 die Position der Spalt-Ebenen entlang der Patch-Ebene identisch ist: Das bedeutet, dass die Position der Spalt-Ebene 2a gleich der Position der Spalt-Ebene 2b in Bezug auf die Patch-Ebene 1 ist. Dies erlaubt eine einfache Berechnung mit obenstehender Gleichung 2, da die verschiedenen Lokalwichtungsfaktoren für die verschiedenen Strahlen an gleichen Punkten entlang der Patch-Ebene gleich 1 bzw. gleich 0 werden.

Fig. 13 ist eine Vergrößerung eines Ausschnittes der Fig. 12. Insbesondere ist der Ausschnitt vergrößert, in dem sich die Patch-Ebene 1 mit den Spalt-Ebenen 2a und 2b schneidet. Auch in Fig. 13 verlaufen die Spalt-Ebenen 2a und 2b in vertikaler Richtung und die Patch-Ebene 1 in horizontaler Richtung. Es sind jeweils die Vorder- und Rückseiten der verschiedenen Spalt-Ebenen und Patch-Ebenen dargestellt. Die Vorderseite 10a der Patch-Ebene und die Rückseite 11a der Patch-Ebene 1 sind mit einer Strich-Punkt-Linie dargestellt. Die Vorderseiten der Spalt-Ebenen 2a und 2b sind mit den Bezeichnern 10b und 10c identifiziert. Die Rückseiten der Spalt-Ebenen 2a und 2b sind mit den Bezeichnern 11b und 11c identifiziert.

Es soll nun für den Punkt P die Teilstrahlendosis für die verschiedenen Strahlen berechnet werden. Aus Fig. 12 ist ersichtlich, dass vier Strahlen 3a bis 3d (in Fig. 13 nicht gezeigt) zur Dosis beitragen. Innerhalb der verschiedenen Ebenen, innerhalb der Patch-Ebene 1 und innerhalb der Split-Ebenen 2a und 2b soll ein linearer Dosisgradient angewendet werden. Unter dieser Vorraussetzung und der Tatsache, dass sich die Spalt-Ebenen 2a, 2b an derselben Position befinden, ergibt sich für die Position des Punktes P:

$$c_{sp,3a} = c_{sp,3c} = 0.9 \rightarrow c_{sp,3b} = c_{sp,3d} = 0.1$$

$$c_{pp,3a} = c_{pp,3c} = 0.55 \rightarrow c_{pp,3b} = c_{pp,3d} = 0.45.$$

Hierbei und in den folgenden zwei Gleichungen indizieren die Indizes 3a-3d die Strahlen 3a - 3d. Mit der voranstehenden Gleichung kann die Dosis der verschiedenen Strahlen berechnet werden. Die Faktoren $c_{sp}$ und $c_{pp}$ ergeben sich durch lineare Skalierung entsprechend der Position des Punktes P und der Spalt-Ebenen- und Patch-Ebenen-Dicke. Dies erfolgt wiederum jeweils isoliert für die Vorderseite 10a und die Rückseite 11a der Patch-Ebene 1. Für die Vorderseite 10a der Patch-Ebene 1 ergeben sich die Dosisbeiträge zum Punkt P der Strahlen 3a und 3b im Übergangsbereich zu:

$$D_{3a,trans} = \frac{1 \cdot 0.9}{1 \cdot 0.9 + 2 \cdot 0.1} \cdot 0.55 \cdot 60Gy = 27Gy$$
$$D_{3b,trans} = \frac{2 \cdot 0.1}{1 \cdot 0.9 + 2 \cdot 0.1} \cdot 0.55 \cdot 60Gy = 6Gy$$

Für die Rückseite der Patch-Ebene 1 ergeben sich die Dosisbeiträge am Punkt P der Strahlen 3c und 3d im Übergangsbereich entsprechend zu

$$D_{3c,trans} = \frac{3 \cdot 0.9}{3 \cdot 0.9 + 4 \cdot 0.1} \cdot 0.45 \cdot 60Gy = 23.5Gy$$
$$D_{3d,trans} = \frac{4 \cdot 0.1}{3 \cdot 0.9 + 4 \cdot 0.1} \cdot 0.45 \cdot 60Gy = 3.5Gy$$

Wie ersichtlich ist, ergibt die Summe der Teilstrahlendosen für die Strahlen 3a bis 3d die Gesamtstrahlendosis $D_{pr}$=60 Gray. Gleichzeitig sind die Strahlengewichte entsprechend berücksichtigt. Die Lokalwichtungsfaktoren $c_{sp}$ und $c_{pp}$ nehmen weiterhin eine Wichtung der lokalen Dosis derart vor, dass ein gradueller Übergang der applizierten Teilstrahlendosen gewährleistet wird.

Noch einmal Bezug nehmend auf Fig. 12 soll nun ein einfacher Fall illustriert werden. Es ist möglich, die Gleichung 3 zur Berechnung der Teilstrahlendosen $D_i$ der Strahlen 3a bis 3d in Fig. 12 zu verwenden. Die Parameter, das heißt die Dosisbruchteile und die Strahlengewichte, sollen entsprechend dem in Fig. 12 diskutierten Ausführungsbeispiel gewählt sein.
Dann ergibt die Anwendung der oben genannten Formel

$$\left.\begin{aligned} D_{1.1,area1.1} &= \frac{1 \cdot 1 \cdot 0.6}{1 \cdot 1 \cdot 0.6 + 2 \cdot 0 \cdot 0.6 + 3 \cdot 1 \cdot 0.4 + 4 \cdot 0 \cdot 0.4} \cdot 60Gy = 0.33 \cdot 60Gy \\ D_{1.2,area1.1} &= 0Gy \\ D_{2.1,area1.1} &= \frac{3 \cdot 1 \cdot 0.4}{1 \cdot 1 \cdot 0.6 + 2 \cdot 0 \cdot 0.6 + 3 \cdot 1 \cdot 0.4 + 4 \cdot 0 \cdot 0.4} \cdot 60Gy = 0.66 \cdot 60Gy \\ D_{2.2,area1.1} &= 0Gy \end{aligned}\right\} = 60Gy = D_{pr}\ .$$

Offensichtlich liefert die Berechnung gemäß obiger Formel immer noch die verschriebene Gesamtstrahlendosis $D_{pr}$=60Gy. Jedoch ergibt die Berechnung, dass die Untervolumen-Teilstrahlendosen, die den entsprechenden Untervolumina, welche der Vorder- bzw. Rückseite der Patch-Ebene zugeordnet sind, von 60% zu 33% verändert wurde. Diese Veränderung ist nicht gemäß des Bestrahlungsplans, wo ein Dosisbruchteil von 60% vorgegeben wurde. Deshalb ist die Berechnung gemäß Gleichung 2 wie voranstehend in Bezug auf das Ausführungsbeispiel von Fig. 12 diskutiert, besser geeignet, um eine Teilstrahlendosis für die verschiedenen beteiligten Strahlen derart zu berechnen, dass die Gesamtstrahlendosis sich aus der Summe der Teilstrahlendosis ergibt und gleichzeitig die jeweils vorgegebenen Untervolumen-Gesamtstrahlendosen eingehalten werden.

In Bezug auf Fig. 14 ist nachfolgend ein Beispiel beschrieben, in dem insgesamt sechs Strahlen 3a-3f mit einer Patch-Ebene verknüpft sind. Die Vorderseite 10a der Patch-Ebene 1 und die Rückseite 11a sind indiziert. Weiterhin existieren vier Spalt-Ebenen 2a, 2b, 2c, 2d. Die Vorderseiten der Spalt-Ebenen 2a, 2b, 2c, 2d sind mit 10b-10e indiziert, die Rückseiten entsprechend mit 11b-11e. Hierbei sind die Strahlen 3a und 3b mit Spalt-Ebene 2c, die Strahlen 3b und 3c mit Spalt-Ebene 2d, die Strahlen 3d und 3e mit Spalt-Ebene 2a und die Strahlen 3e und 3f mit Spalt-Ebene 2b verknüpft. Ein siebter Strahl 3g ist mit keiner dieser Steuerungsebenen 1, 2a, 2b, 2c, 2d verknüpft.

Dieses Beispiel illustriert das Vorhandensein mehrerer zweiten Lokalwichtungsfaktoren $c_{sp}$. Weiterhin wird die Verwendung von Strahlgewichten $w_i$ für Strahlen mit und ohne Zuordnung zu Steuerungsebenen illustriert. Die numerischen Beispiele sind rein illustrativ und nicht beschränkend. Es ist klar, dass auch andere Parameter je nach Anwendungsfall gewählt werden können.

Die Strahlgewichte der Strahlen 3a-3c (ein Fig. 14 von oben) :

$$w_1 = w_2 = w_3 = 3 ,$$

wobei hier und in den folgenden Erläuterungen zu Fig. 14 jeweils die Indizes 1-7 die Strahlen 3a-3g bezeichnen.

Die Strahlgewichte der Strahlen 3d - 3f /in Fig. 14 von unten) :

$$w_4 = w_5 = w_6 = 4 .$$

Zur Berechnung der korrigierten Gesamtstrahlendosis $D_{pr}$* wird der Patch-Ebene ein Gewicht zugeordnet, welches zur Berechnung der Teilstrahlendosis $D_7$ des Strahls 3g verwendet wird:

$$w_{pp} = 2 .$$

Entsprechend lautet das Gewicht des Strahls 3g, ohne Zuordnung zu der Patch-Ebene 1:

$$w_7 = 1 .$$

Die Lokalwichtungsfaktoren der Patch-Ebene 1:

$$c_{pp,1} = 0{,}7; c_{pp,2} = 0{,}8 .$$

Die Gesamtstrahlendosis $D_{pr} = 60Gy$.

Die Teilstrahlendosis des Strahls 3g berechnet sich dann zu:

$$D_7 = \frac{1}{1+2} 60Gy = 20Gy$$

Das bedeutet, dass die Summe der Teilstrahlendosen aller mit Patch-Ebene verknüpfter Strahlen $D_{pr}$* = 40 Gy beträgt.

Darauf basierend kann die Berechnung der Teilstrahlendosen für die verschiedenen Strahlen 3a-3f erfolgen und zwar für den Punkt P, wie er in Fig. 14 indiziert ist. Punkt P befindet sich in Fig. 14 unterhalb der Patch-Ebene innerhalb der Spalt-Ebene 2b. Beispielhaft werden in Bezug auf diese Position die folgenden zwei Lokalwichtungsfaktoren verwendet: Strahl 3b: $c_{sp,2} = 0.6$; Strahl 3c: $c_{sp,3} = 0.4$; Strahl 3a: $c_{sp,1} = 0$; Strahl 3e: $c_{sp,5} = 0.3$; Strahl 3f: $c_{sp,6} = 0.7$; Strahl 3d:

$$c_{sp,4} = 0 .$$

Strahl 3b:

$$D_2 = \frac{3(1 \cdot 0,6)}{3(1 \cdot 0,6) + 3(1 \cdot 0,4)} \cdot 0,2 \cdot 40Gy = 4,8Gy\ .$$

Strahl 3c:

$$D_3 = \frac{3(1 \cdot 0,4)}{3(1 \cdot 0,6) + 3(1 \cdot 0,4)} \cdot 0,2 \cdot 40Gy = 3,2Gy\ .$$

Strahl 3a:

$$D_1 = 0\ .$$

Strahl 3e:

$$D_5 = \frac{4(1 \cdot 0,3)}{4(1 \cdot 0,3) + 4(1 \cdot 0,7)} \cdot 0,8 \cdot 40Gy = 9,6Gy\ .$$

Strahl 3f:

$$D_6 = \frac{4(1 \cdot 0,7)}{4(1 \cdot 0,3) + 4(1 \cdot 0,7)} \cdot 0,8 \cdot 40Gy = 22,4Gy\ .$$

Strahl 3d:

$$D_4 = 0\ .$$

Es ist ersichtlich, dass die Summe der Teilstrahlendosen der Strahlen 3a - 3f wiederum 40 Gy beträgt.

In Bezug auf Fig. 15 ist näher illustriert, welche Schritte notwendig sind, um eine ortsaufgelöste Berechnung der Teilstrahlendosis $D_i$ durchzuführen. Das Verfahren beginnt mit Schritt S0. Zunächst wird in Schritt S1 eine Gesamtstrahlendosis $D_{pr}$ definiert. Zum Beispiel kann die Gesamtstrahlendosis anhand einer verschriebenen Dosis aus einem Bestrahlungsplan definiert werden. Die verschriebene Dosis wird typischerweise unter klinischen Aspekten gewählt. Dann erfolgt in Schritt S2 die Definition eines Zielvolumens. Das Zielvolumen kann zum Beispiel ein Tumor sein. Das Zielvolumen kann insbesondere zum Beispiel anhand von dreidimensionalen Bilddatensätzen festgelegt werden, welche zum Beispiel mittels bildgebenenden Verfahren der Magnetresonanztomographie oder der Computertomographie erhalten wurden.

Dann erfolgt in Schritt S3 das Festlegen einer ersten Steuerungsebene. Die erste Steuerungsebene entspricht einer Patch-Ebene. Die erste Steuerungsebene kann geometrisch mittels geeigneter Techniken innerhalb des dreidimensionalen Bilddatensatzes in das Zielvolumen hineingelegt werden.

In Schritt S4 wird der in Schritt S3 festgelegten ersten Steuerungsebene jeweils ein Dosisbruchteil $F_o$ bzw. $F_r$ zugewiesen, welche den Bruchteil der Untervolumen-Gesamtstrahlendosis an der Gesamtstrahlendosis für alle mit einer Seite der ersten Steuerungsebene verknüpften Strahlen bezeichnet. Hierbei bezeichnet die Untervolumen-Gesamtstrahlendosis diejenige Dosis, die in dem Untervolumen, welches einer Seite der ersten Steuerungsebene zugewandt ist, durch alle mit dieser Seite verknüpften Strahlen appliziert wird. Dies wurde in Bezug auf Fig. 2 beschrieben.

Es ist zum Beispiel möglich, dass eine in Schritt S3 festgelegte erste Steuerungsebene eine gewisse Dicke aufweist. Die Position der ersten Steuerungsebene kann dann zum Beispiel durch den Mittelpunkt der Steuerungsebene beschrieben werden und die Steuerungsebene ist weiterhin gekennzeichnet durch eine Vorder- und eine Rückseite. Insbesondere ist es dann möglich, für mit der Steuerungsebene verknüpfte Strahlen einen graduellen Übergang der Teilstrahlendosis als Funktion des Ortes innerhalb der ersten Steuerungsebene zu ermöglichen. Dies geschieht durch eine ortsaufgelöste Festlegung der ersten Lokalwichtungsfaktoren cpp. Die ersten Lokalwichtungsfaktoren sind jeweils einer ersten Steuerungsebene bzw. einer Seite der ersten Steuerungsebene zugeordnet. Dies wurde in Bezug auf Fig. 2 diskutiert.

In Schritt S5 wird dann ein Strahl festgelegt und der jeweilige Strahl mit einer Seite der Patch-Ebene verknüpft bzw. zugeordnet. Hierbei ist zu beachten, dass ein Strahl jeweils eindeutig mit einer Seite einer Patch-Ebene verknüpft ist. D.h., dass ein Strahl insbesondere nicht mit mehreren Seiten verschiedener Patch-Ebenen oder derselben Patch-Ebene verknüpft sein kann. In Schritt S6 wird überprüft, ob ein weiterer Strahl notwendig ist. Zum Beispiel können dosimetrische Gründe dafür sprechen, mehrere Strahlen zur Applikation der Gesamtstrahlendosis $D_{pr}$ innerhalb des Zielvolumens zu verwenden. Dies wurde voranstehend beschrieben. Wird in Schritt S6 festgestellt, dass ein weiterer Strahl benötigt wird, so kann Schritt S5 wiederholt werden und der weitere Strahl wird festgelegt.

Wird jedoch in Schritt S6 festgestellt, dass kein weiterer Strahl benötigt ist, so wird in Schritt S7 überprüft, ob eine weitere Patch-Ebene notwendig ist. Ist dies der Fall, werden die Schritte S3-S6 wiederholt. Wird jedoch festgestellt, dass keine weitere Patch-Ebene benötigt ist, so setzt das Verfahren mit S8 fort. In Schritt S8 wird für jeden Strahl überprüft, ob eine Spaltung des Strahls notwendig ist. Zum Beispiel kann aufgrund von hardwareseitigen Limitationen eine Spaltung eines Strahls in mehrere Strahlen notwendig sein. Wird zum Beispiel ein Lamellenkollimator, um die Strahlen zu fokussieren, so hat dieser Lamellenkollimator typischerweise ein begrenztes Gesichtsfeld. Ist die Größe des Strahls, welche in Schritt S5 bestimmt wurde jedoch größer als diese maximale Gesichtsfeld, so ist eine Spaltung des Strahls notwendig und auf Schritt S8 folgt Schritt S9.

In Schritt S9 wird für den jeweiligen Strahl eine zweite Steuerungsebene, d.h. eine Spalt-Ebene festgelegt. Es ist auch möglich, mehrere Spalt-Ebenen festzulegen. Wie in Bezug auf Fig. 3 voranstehend beschrieben wurde, gibt es verschiedene Möglichkeiten, die Position und die Anzahl der benötigten Spalt-Ebenen bei gegebener Strahlgröße zu berechnen. Diese Berechnungen können in Schritt S9 zur Festlegung der Spalt-Ebene herangezogen werden. In Schritt S10 erfolgt dann die Spaltung des Strahls in mehrere, z.B. zwei Strahlen, und Verknüpfung der so erhaltenen Strahlen mit der jeweiligen Spalt-Ebene.

Es sollte verstanden werden, dass der ursprüngliche Strahl dann nicht mehr für die weitere Prozessierung relevant ist. Es werden aus einem Strahl zum Beispiel durch Definition einer Spalt-Ebene zwei Strahlen verwendet, welche dann physikalisch zu einem späteren Zeitpunkt Dosis applizieren. Der Ausgangsstrahl, welcher zum Beispiel in Schritt S5 festgelegt wurde, ist ein rein virtueller Strahl, der im Laufe der Berechnung, wie sie gegenwärtig diskutiert wird, verwendet wird. Er hat jedoch keinen physikalischen Einfluss z.B. im Bestrahlungsvorgang.

In Schritt S11 werden dann für jeden Strahl zweite Lokalwichtungsfaktoren $c_{sp}$ festgelegt. Diese zweiten Lokalwichtungsfaktoren erlauben eine ortsaufgelöste Berechnung der Teilstrahlendosen für die verschiedenen Strahlen in Bezug auf die Positionierung zu zweiten Steuerungsebenen.

In Schritt S11 wird festgestellt, ob ein weiterer Strahl vorhanden ist. Ist ein weiterer Strahl vorhanden, so wird Schritt S8 erneut durchgeführt, d.h. es erfolgt erneut eine Überprüfung für den nun selektierten Strahl, ob eine Aufspaltung in mehrere Strahlen notwendig ist. Wird in Schritt S8 festgestellt, dass keine Aufspaltung notwendig ist, so werden Schritte S9-S11 ausgelassen. Anderenfalls werden die Schritte S9 und S11 erneut durchgeführt.

In Bezug auf Strahlen, welche mit zweiten Steuerungsebenen, d.h. Spalt-Ebenen verknüpft sind, sollte verstanden werden, dass ein Strahl auch zum Beispiel zwei oder mehreren zweiten Steuerungsebenen zugeordnet sein kann. Dies wurde in Bezug auf Fig. 5 erläutert. Dies stellt einen Unterschied zwischen der Verknüpfung von Strahlen mit zweiten Steuerungsebenen, wie sie in Schritt S10 stattfindet, und der Verknüpfung von Strahlen mit ersten Steuerungsebenen, wie sie in Schritt S5 stattfindet, dar.

Wird in Schritt S12 festgestellt, dass kein weiterer Strahl vorhanden ist, so folgt darauf Schritt S13. Schritt S13 ist ein optionaler Schritt. In Schritt S13 können Strahlen festgelegt werden, welche keine Zuordnung zu einer ersten Steuerungsebene, d.h. einer Patch-Ebene haben. Solche Strahlen applizieren eine Teilstrahlendosis typischerweise homogen über das gesamte Zielvolumen, welches in Schritt S2 festgelegt wurde. Anschließend werden in Schritt S14 für alle Strahlen, d.h. für Strahlen, welche mit einer ersten oder zweiten Steuerungsebene verknüpft sind, als auch für Strahlen, welche keine solche Verknüpfung aufweisen, Strahlgewichte $w_i$ festegelegt. Solche Strahlgewichte $w_i$ wurden voranstehend in Bezug auf die Figuren 10-14 diskutiert. Die Strahlgewichte $w_i$ erlauben eine relative Wichtung der Teilstrahlendosen verschiedener Strahlen zueinander. Die Verwendung von Teilstrahlendosen kann eine Optimierung unter dosimetrischen Gesichtpunkten erlauben.

In Schritt S15 erfolgt dann die Berechnung der Teilstrahlendosen $D_i$ für jeden Strahl, welche keine Verknüpfung mit einer ersten oder zweiten Steuerungsebene aufweist. Zum Beispiel wurde diese Berechnung in Bezug auf die Fig.

10 voranstehend beschrieben. Die Summe der Teilstrahlendosen $D_i$ derjenigen Strahlen, welche keine Verknüpfung mit einer ersten oder zweiten Steuerungsebene aufweisen, wird dann verwendet, um eine korrigierte Gesamtstrahlendosis $D_{pr}$* in Schritt S16 zugerechnet. Diese korrigierte Gesamtstrahlendosis wird in den nachfolgenden Schritte S17 und folgende für die Berechnung der Teilstrahlendosen der verknüpften Strahlen verwendet.

Diese Berechnung startet in Schritt S17, indem jeweils eine Patch-Ebene selektiert wird. Dann erfolgt in Schritt S18 und S19 getrennt für jeweils die Vorder- und die Rückseite der selektierten Patch-Ebene die Berechnung der Teilstrahlendosis $D_i$ für diejenigen Strahlen, die mit der gerade ausgewählten Vorder- bzw. Rückseite der Patch-Ebene verknüpft sind. In Schritt S18 wird die Teilstrahlen Dosis $D_i$ isoliert für alle Strahlen berechnet, welche mit der Vorderseite der selektierten Patch-Ebene verknüpft sind. Diese Berechnung erfolgt auf Grundlage der ersten und zweiten Lokalwichtungsfaktoren $c_{sp}$ und $c_{pp}$, als auch der korrigierten Gesamtstrahlendosis $D_{pr}$* wie sie aus Schritt S16 erhalten wurde. Es sollte verstanden werden, dass wenn Schritt S13 nicht erfolgt, d.h. keine Strahlen ohne Zuordnung zu ersten oder zweiten Steuerungsebenen existieren, die korrigierte Gesamtstrahlendosis $D_{pr}$* gleich der Gesamtstrahlendosis $D_{pr}$ , wie sie in Schritt S1 festgelegt wurde ist. In Schritt S19 erfolgt dann die entsprechende Berechnung für alle Strahlen, welche mit der Rückseite der selektierten Patch-Ebene verknüpft sind.

In Schritt S20 wird anschließend überprüft, ob weitere Ebenen vorhanden sind. Sind weitere Patch-Ebenen vorhanden, so werden die Schritte S17-S19 erneut durchgeführt. Anderenfalls sind alle Teilstrahlendosen der verschiedenen Strahlen berechnet, und es ist möglich in Schritt S21 eine Bestrahlung des Zielvolumens gemäß den berechneten Parametern der verschiedenen Strahlen durchzuführen. Dann kommt in Schritt S22 das Verfahren zu einem Ende.

Es sollte verstanden werden, dass die Reihenfolge der Schritte, wie sie in Bezug auf Fig. 15 obenstehend diskutiert wurden, variabel ist. Zum Beispiel ist es möglich, Schritt S13, d.h. das Festlegen von Strahlen ohne Zuordnung zu Patch-Ebenen, vor z.B. Schritt S3 durchzuführen, d.h. vor der Festlegung von Strahl mit Zuordnung zu Patch-Ebenen. Ebenso ist es möglich, erst die Strahlen mit Spalt-Ebenen zu verknüpfen (Schritte S8-S12) und dann die gespaltenen Strahlen Patch-Ebenen zuzuordnen (Schritte S3-S7).

Obwohl die Erfindung im Detail durch die bevorzugten Ausführungsbeispiele näher illustrier und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zum Berechnen von lokalen Teilstrahlendosen (Di) in einer Strahlentherapieanlage zum Applizieren einer Gesamtstrahlendosis ($D_{pr}$) in einem Zielvolumen mit mehreren Strahlen, das Verfahren umfassend:

    - Festlegen mindestens einer ersten Steuerungsebene zur Steuerung der Dosierung der Strahlen (3, 3a, 3b, 3c, 3d, 3e, 3f), wobei jede der mindestens einen ersten Steuerungsebene (1) das Zielvolumen (4) in zwei Untervolumen aufteilt,
    - Jeweils für die Vorderseite (10, 10a, 10b, 10c, 10d, 10e) und die Rückseite (11, 11a, 11b, 11c, 11d, 11e) jeder der mindestens einen ersten Steuerungsebene: Zuordnen mindestens eines Strahls,
    - Für jede erste Steuerungsebene (1, 1a, 1b): Festlegen einer Untervolumen-Gesamtstrahlendosis ($D_o$, $D_r$) für jeweils jedes der zwei Untervolumen als ein Bruchteil ($F_o$, $F_r$) der Gesamtstrahlendosis ($D_{pr}$),

    das Verfahren weiterhin umfassend:

    - Festlegen mindestens einer zweiten Steuerungsebene (2, 2a, 2b, 2c, 2d, 2e, 2f, 2g) zur Steuerung der Positionierung der Strahlen, wobei jede der mindestens einen zweiten Steuerungsebene (2, 2a, 2b, 2c, 2d, 2e, 2f, 2g) das Zielvolumen (4) in zwei Untervolumen aufteilt,
    - Zuordnen mindestens eines Strahls (3, 3a, 3b, 3c, 3d, 3e, 3f) zu jeder der mindestens einen zweiten Steuerungsebene (2, 2a, 2b, 2c, 2d, 2e, 2f, 2g),

    wobei ein zu einer zweiten Steuerungsebene (2, 2a, 2b, 2c, 2d, 2e, 2f, 2g) zugeordneter Strahl (3, 3a, 3b, 3c, 3d, 3e, 3f) durch die jeweilige zweite Steuerungsebene derart zweigeteilt wird, dass die lokale Teilstrahlendosis ($D_i$) der so erhaltenen zwei Strahlen (3, 3a, 3b, 3c, 3d, 3e, 3f) jeweils in unterschiedlichen, durch die jeweilige zweite Steuerungsebene definierten, Untervolumen ungleich Null ist,
    das Verfahren weiterhin umfassend:

    - Für mindestens eine Seite einer ersten Steuerungsebene: isoliertes Berechnen der entsprechenden lokalen Teilstrahlendosen ($D_i$) aller dieser ersten Steuerungsebene (1, 1a, 1b) zugeordneten Strahlen, sodass die Summe der lokalen Teilstrahlendosen derjenigen Strahlen, die der dem jeweiligen Untervolumen zugewandten Seite der jeweiligen ersten Steuerungsebene (1, 1a, 1b) zugeordnet sind, die jeweilige Untervolumen-Gesamtstrahlendosis ($D_o$, $D_r$) ergibt und die Summe der lokalen Teilstrahlendosen der restlichen dieser ersten Steuerungsebene (1, 1a, 1b) zugeordneten Strahlen (3, 3a, 3b, 3c, 3d, 3e, 3f) die Differenz zwischen der jeweiligen Untervolumen-Gesamtstrahlendosis (Do, Dr) und Gesamtstrahlendosis ($D_{pr}$) ergibt.

**2.** Verfahren nach Anspruch 1, wobei ein Strahl (3, 3a, 3b, 3c, 3d, 3e, 3f) jeweils eindeutig einer Seite einer ersten Steuerungsebene (1, 1a, 1b) zugeordnet ist.

**3.** Verfahren nach einem der Ansprüche 1 oder 2, wobei die lokalen Teilstrahlendosen ($D_i$) eines einer Seite einer ersten Steuerungsebene (1, 1a, 1b) zugeordneten Strahls in den durch diese erste Steuerungsebene (1, 1a, 1b) definierten Untervolumen unterschiedlich sind

**4.** Verfahren nach einem der voranstehenden Ansprüche, wobei die ersten Steuerungsebenen eine endliche Dicke aufweisen und wobei Strahlen, die auf unterschiedlichen Seiten der jeweiligen ersten Steuerungsebene (1, 1a, 1b) unterschiedliche lokale Teilstrahlendosen ($D_i$) aufweisen, durch einen örtlichen Verlauf der lokalen Teilstrahlendosis ($D_i$) innerhalb der ersten Steuerungsebene (1, 1a, 1b) einen graduellen Übergang der lokalen Teilstrahlendosis ($D_i$) gewährleisten.

**5.** Verfahren nach einem der voranstehenden Ansprüche, wobei für jeden Strahl (3, 3a, 3b, 3c, 3d, 3e, 3f) ein Strahlengewicht ($w_i$) festgelegt wird, wobei die Strahlengewichte die relativen Verhältnisse der Teilstrahlendosen ($D_i$) verschiedener Strahlen (3, 3a, 3b, 3c, 3d, 3e, 3f) zueinander festlegen.

**6.** Verfahren nach Anspruch 5, weiterhin innerhalb des Zielvolumens (4) für eine bestimmte Seite einer bestimmten ersten Steuerungsebene (1, 1a, 1b) umfassend:

- Festlegen von ersten Lokalwichtungsfaktoren ($c_{pp}$) für jeden Abstand senkrecht zu der bestimmten ersten Steuerungsebene, wobei die ersten Lokalwichtungsfaktoren ($c_{pp}$) den Bruchteil der Untervolumen-Gesamtstrahlendosis an der Gesamtstrahlendosis ($D_{pr}$) definieren,
- Für jeden der bestimmen Seite der bestimmten ersten Steuerungsebene (1, 1a, 1b) zugeordneten Strahl (3, 3a, 3b, 3c, 3d, 3e, 3f): Festlegen von zweiten Lokalwichtungsfaktoren ($c_{sp,i}$) für jeden Abstand senkrecht zu einer zweiten Steuerungsebene (2, 2a, 2b, 2c, 2d, 2e, 2f, 2g), zu denen der jeweilige Strahl (3, 3a, 3b, 3c, 3d, 3e, 3f) zugeordnet ist, wobei die zweiten Lokalwichtungsfaktoren ($c_{sp,i}$) die Strahlengewichte ($w_i$) der Strahlen (3, 3a, 3b, 3c, 3d, 3e, 3f) in Abhängigkeit von der Position zu zweiten Steuerungsebenen (2, 2a, 2b, 2c, 2d, 2e, 2f, 2g) modifizieren,
- Für jeden der bestimmen Seite der bestimmten ersten Steuerungsebene (1, 1a, 1b) zugeordneten Strahl: Berechnen der lokalen Teilstrahlendosis ($D_i$) für jedes Untervolumen für den jeweiligen Strahl, basierend auf Elementen, die aus der folgenden Gruppe ausgewählt werden: Strahlwichtungsfaktoren ($w_i$), erste Lokalwichtungsfaktoren ($c_{pp}$), zweite Lokalwichtungsfaktoren ($c_{sp,i}$), Gesamtstrahlendosis ($D_{pr}$).

**7.** Verfahren nach Anspruch 6, wobei in der Berechnung der lokalen Teilstrahlendosis die zweiten Lokalwichtungsfaktoren ($_{csp,i}$) die Strahlwichtungsfaktoren ($w_i$) zwischen 0% und 100% ihres Wertes modifizieren.

**8.** Verfahren nach Anspruch 6 oder 7, wobei ersten und zweiten Steuerungsebenen (2, 2a, 2b, 2c, 2d, 2e, 2f, 2g) eine Dicke aufweisen und die ersten und zweiten Lokalwichtungsfaktoren jeweils innerhalb der entsprechenden Steuerungsebene als Funktion der Position variieren.

**9.** Verfahren nach einem der Ansprüche 5 - 8, wobei das Berechnen ortsaufgelöst gemäß folgender Formel für eine Seite einer ersten Steuerungsebene (1, 1a, 1b) erfolgt:

$$D_i = \frac{w_i \prod_k c_{sp,i,k}}{\sum_{j=1}^{n} w_j \prod_l c_{sp,j,l}} c_{pp} D_{pr} \quad ,$$

wobei $w_i$ die Strahlwichtungsfaktoren, $c_{pp}$ den entsprechenden ortsaufgelösten ersten Lokalwichtungsfaktor, $c_{pp}D_{pr}$ die Untervolumen-Gesamtstrahlendosis bezeichnet, $D_{pr}$ die Gesamtstrahlendosis und $c_{sp}$ die ortsaufgelösten zweiten Lokalwichtungsfaktoren für alle Strahlen, die der entsprechenden Seite der ersten Steuerungsebene (1, 1a, 1b) zugeordnet sind, bezeichnet.

**10.** Verfahren nach einem der voranstehenden Ansprüche, weiterhin umfassend: Festlegen von Strahlen (3, 3a, 3b, 3c, 3d, 3e, 3f) ohne Zuordnung zu ersten oder zweiten Steuerungsebenen (2, 2a, 2b, 2c, 2d, 2e, 2f, 2g), wobei die Teilstrahlendosen ($D_i$) dieser Strahlen (3, 3a, 3b, 3c, 3d, 3e, 3f) vor dem isolierten Berechnen von der Gesamtstrahlendosis ($D_{pr}$) abgezogen werden.

**11.** Verfahren nach Anspruch 10, weiterhin umfassend:

- Festlegen von Strahlwichtungsfaktoren ($w_i$) für Strahlen (3, 3a, 3b, 3c, 3d, 3e, 3f) ohne Zuordnung zu ersten oder zweiten Steuerungsebenen (2, 2a, 2b, 2c, 2d, 2e, 2f, 2g) und
- Anpassen der Gesamtstrahldosis ($D_{pr}$) für Strahlen, die ersten und / oder zweiten Steuerungsebenen (2, 2a, 2b, 2c, 2d, 2e, 2f, 2g) zugeordnet sind basierend auf den Strahlwichtungsfaktoren ($w_i$) für Strahlen (3, 3a, 3b, 3c, 3d, 3e, 3f) ohne Zuordnung zu ersten oder zweiten Steuerungsebenen (2, 2a, 2b, 2c, 2d, 2e, 2f, 2g).

**12.** Verfahren zum Berechnen von lokalen Teilstrahlendosen ($D_i$) in einer Strahlentherapieanlage zum Applizieren einer Gesamtstrahlendosis ($D_{pr}$) in einem Zielvolumen (4) mit mehreren Strahlen, das Verfahren umfassend:

- Festlegen mindestens einer zweiten Steuerungsebene (2, 2a, 2b, 2c, 2d, 2e, 2f, 2g) zur Steuerung der Positionierung der Strahlen, wobei jede der mindestens einen zweiten Steuerungsebene (2, 2a, 2b, 2c, 2d, 2e, 2f, 2g) das Zielvolumen (4) in zwei Untervolumen aufteilt,
- Zuordnen mindestens eines Strahls zu jeder der mindestens einen zweiten Steuerungsebenen (2, 2a, 2b, 2c, 2d, 2e, 2f, 2g),

wobei ein zu einer zweiten Steuerungsebene (2, 2a, 2b, 2c, 2d, 2e, 2f, 2g) zugeordneter Strahl (3, 3a, 3b, 3c, 3d, 3e, 3f) durch die jeweilige zweite Steuerungsebene derart zweigeteilt wird, dass die lokale Teilstrahlendosis (Di) der so erhaltenen zwei Strahlen (3, 3a, 3b, 3c, 3d, 3e, 3f) jeweils in unterschiedlichen, durch die jeweilige zweite Steuerungsebene definierten, Untervolumen ungleich Null ist,
das Verfahren weiterhin umfassend:

- Festlegen von Strahlen (3, 3a, 3b, 3c, 3d, 3e, 3f) ohne Zuordnung zu zweiten Steuerungsebenen (2, 2a, 2b, 2c, 2d, 2e, 2f, 2g),
- Korrigieren der Gesamtstrahlendosis durch Subtraktion der lokalen Teilstrahlendosen der Strahlen (3, 3a, 3b, 3c, 3d, 3e, 3f) ohne Zuordnung von der Gesamtstrahlendosis,
- Berechnen der lokalen Teilstrahlendosen (Di) für alle einer zweiten Steuerungsebene (2, 2a, 2b, 2c, 2d, 2e, 2f, 2g) zugeordneten Strahlen.

**13.** Verfahren nach Anspruch 12, wobei für jeden Strahl (3, 3a, 3b, 3c, 3d, 3e, 3f) ein Strahlengewicht ($w_i$) festgelegt wird, wobei die Strahlengewichte die relativen Verhältnisse der Teilstrahlendosen ($D_i$) verschiedener Strahlen (3, 3a, 3b, 3c, 3d, 3e, 3f) zueinander festlegen.

**14.** Verfahren nach Anspruch 13, wobei zur Korrektur der Gesamtstrahlendosis das Strahlengewicht aller einer zweiten Steuerungsebene (2, 2a, 2b, 2c, 2d, 2e, 2f, 2g) zugeordneten Strahlen (3, 3a, 3b, 3c, 3d, 3e, 3f) in Summe eins ist.

**15.** Verfahren nach einem der voranstehenden Ansprüche, wobei die Summe der lokalen Teilstrahlendosen ($D_i$) aller Strahlen (3, 3a, 3b, 3c, 3d, 3e, 3f) an jeder Position innerhalb des Zielvolumens (4) gleich der Gesamtstrahlendosis ist.

**16.** Verfahren nach einem der voranstehenden Ansprüche, wobei die zweiten Steuerungsebenen (2, 2a, 2b, 2c, 2d, 2e, 2f, 2g) eine endliche Dicke aufweisen und wobei Strahlen, die durch eine zweite Steuerungsebene zweigeteilt werden, durch einen örtlichen Verlauf der lokalen Teilstrahlendosis ($D_i$) innerhalb der zweiten Steuerungsebene (2, 2a, 2b, 2c, 2d, 2e, 2f, 2g) einen graduellen Übergang der lokalen Teilstrahlendosis ($D_i$) gewährleisten.

**17.** Verfahren nach einem der voranstehenden Ansprüche, wobei die lokale Teilstrahlendosis ($D_i$) für einen einer ersten Steuerungsebene (1, 1a, 1b) zugeordneten Strahl (3, 3a, 3b, 3c, 3d, 3e, 3f) auf den unterschiedlichen Seiten der ersten Steuerungsebene (1, 1a, 1b) ungleich Null ist und wobei die lokale Teilstrahlendosis für einen einer zweiten Steuerungsebene (2, 2a, 2b, 2c, 2d, 2e, 2f, 2g) zugeordneten Strahl (3, 3a, 3b, 3c, 3d, 3e, 3f) auf genau einer der Seiten der zweiten Steuerungsebene (2, 2a, 2b, 2c, 2d, 2e, 2f, 2g) gleich Null ist.

**18.** Verfahren nach einem der voranstehenden Ansprüche, wobei die Strahlen (3, 3a, 3b, 3c, 3d, 3e, 3f) durch einen Strahlursprung charakterisiert sind, wobei der Strahlursprung die Position bezeichnet, an dem ein Strahl (3, 3a, 3b, 3c, 3d, 3e, 3f) erzeugt wird.

**19.** Verfahren nach Anspruch 18, wobei zwei Strahlen, die durch eine zweite Steuerungsebene zweigeteilt sind, im Wesentlichen gleiche Strahlursprünge haben.

**20.** Verfahren nach einem der voranstehenden Ansprüche, wobei die zweiten Steuerungsebenen (2, 2a, 2b, 2c, 2d, 2e, 2f, 2g) derart festgelegt werden, dass Strahlen, welche ein Teilstrahlendosis ($D_i$) in einem Zielvolumen (4) aufweisen, welches größer ist, als das dem Strahl (3, 3a, 3b, 3c, 3d, 3e, 3f) maximal zugängliche Zielvolumen (4), durch eine zweite Steuerungsebene zweigeteilt werden.

**21.** Verfahren nach einem der voranstehenden Ansprüche, wobei die ersten Steuerungsebenen (1, 1a, 1b) Patch-Ebenen eines Bestrahlungsplans bezeichnen und die zweiten Steuerungsebenen (2, 2a, 2b, 2c, 2d, 2e, 2f, 2g) Split-Ebenen eines Bestrahlungsplans bezeichnen.

**22.** Strahlentherapieanlage, ein Behandlungs-Planungssystem (208), eine Verarbeitungsvorrichtung (205) und eine Strahlerzeugungsvorrichtung (201) umfassend,
wobei das Behandlungs-Planungssystem (208) konfiguriert ist, zum Berechnen von lokalen Teilstrahlendosen ($D_i$) zum Applizieren einer Gesamtstrahlendosis ($D_{pr}$) in einem Zielvolumen (4) mit mehreren Strahlen (3, 3a, 3b, 3c, 3d, 3e, 3f) folgende Schritte durchzuführen:

- Festlegen mindestens einer ersten Steuerungsebene (1, 1a, 1b) zur Steuerung der Dosierung der Strahlen, wobei jede der mindestens einen ersten Steuerungsebene (1, 1a, 1b) das Zielvolumen (4) in zwei Untervolumen aufteilt,
- Jeweils für die Vorderseite (10, 10a, 10b, 10c, 10d, 10e) und die Rückseite (11, 11a, 11b, 11c, 11d, 11e) jeder der mindestens einen ersten Steuerungsebene: Zuordnen mindestens eines Strahls,
- Für jede erste Steuerungsebene (1, 1a, 1b): Festlegen einer Untervolumen-Gesamtstrahlendosen ($D_o$, $D_r$) für jeweils jedes der zwei Untervolumen als ein Bruchteil ($F_o$, $F_r$) der Gesamtstrahlendosis ($D_{pr}$),
- Festlegen mindestens einer zweiten Steuerungsebene (2, 2a, 2b, 2c, 2d, 2e, 2f, 2g) zur Steuerung der Positionierung der Strahlen, wobei jede der mindestens einen zweiten Steuerungsebene (2, 2a, 2b, 2c, 2d, 2e, 2f, 2g) das Zielvolumen (4) in zwei Untervolumen aufteilt,
- Zuordnen mindestens eines Strahl (3, 3a, 3b, 3c, 3d, 3e, 3f) zu jeder der mindestens einen zweiten Steuerungsebene (2, 2a, 2b, 2c, 2d, 2e, 2f, 2g),

wobei ein zu einer zweiten Steuerungsebene (2, 2a, 2b, 2c, 2d, 2e, 2f, 2g) zugeordneter Strahl (3, 3a, 3b, 3c, 3d, 3e, 3f) durch die jeweilige zweite Steuerungsebene derart zweigeteilt wird, dass die lokale Teilstrahlendosis (Di) der so erhaltenen zwei Strahlen (3, 3a, 3b, 3c, 3d, 3e, 3f) jeweils in unterschiedlichen, durch die jeweilige zweite Steuerungsebene definierten, Untervolumen ungleich Null ist,
wobei die Verarbeitungsvorrichtung (205) konfiguriert ist folgenden Schritt durchzuführen:

- Für mindestens eine Seite einer ersten Steuerungsebene: isoliertes Berechnen der entsprechenden lokalen Teilstrahlendosen ($D_i$) aller dieser ersten Steuerungsebene (1, 1a, 1b) zugeordneten Strahlen, sodass die Summe der lokalen Teilstrahlendosen derjenigen Strahlen, die der dem jeweiligen Untervolumen zugewandten Seite der jeweiligen ersten Steuerungsebene (1, 1a, 1b) zugeordnet sind, die jeweilige Untervolumen-Gesamtstrahlendosis ($D_o$, $D_r$) ergibt und die Summe der lokalen Teilstrahlendosen der restlichen dieser ersten Steuerungsebene (1, 1a, 1b) zugeordneten Strahlen (3, 3a, 3b, 3c, 3d, 3e, 3f) die Differenz zwischen der jeweiligen Untervolumen-Gesamtstrahlendosis ($D_o$, $D_r$) und Gesamtstrahlendosis ($D_{pr}$) ergibt,

wobei die Strahlerzeugungsvorrichtung (201) konfiguriert ist, Strahlen (3, 3a, 3b, 3c, 3d, 3e, 3f) mit der von der Verarbeitungsvorrichtung (205) berechneten lokalen Teilstrahlendosis ($D_i$) zu applizieren.

**23.** Strahlentherapieanlage nach Anspruch 22, welche konfiguriert ist, ein Verfahren nach einem der Ansprüche 2-11 durchzuführen.

**24.** Strahlentherapieanlage, ein Behandlungs-Planungssystem (208), eine Verarbeitungsvorrichtung (205) und eine Strahlerzeugungsvorrichtung (201) umfassend, wobei das Behandlungs-Planungssystem (208) konfiguriert ist, zum Berechnen von lokalen Teilstrahlendosen ($D_i$) zum Applizieren einer Gesamtstrahlendosis ($D_{pr}$) in einem Zielvolu-

men (4) mit mehreren Strahlen, folgende Schritte durchzuführen:

- Festlegen mindestens einer zweiten Steuerungsebene (2, 2a, 2b, 2c, 2d, 2e, 2f, 2g) zur Steuerung der Positionierung der Strahlen, wobei jede der mindestens einen zweiten Steuerungsebene (2, 2a, 2b, 2c, 2d, 2e, 2f, 2g) das Zielvolumen (4) in zwei Untervolumen aufteilt,
- Zuordnen mindestens eines Strahl (3, 3a, 3b, 3c, 3d, 3e, 3f) zu jeder der mindestens einen zweiten Steuerungsebene (2, 2a, 2b, 2c, 2d, 2e, 2f, 2g),

wobei ein zu einer zweiten Steuerungsebene (2, 2a, 2b, 2c, 2d, 2e, 2f, 2g) zugeordneter Strahl (3, 3a, 3b, 3c, 3d, 3e, 3f) durch die jeweilige zweite Steuerungsebene derart zweigeteilt wird, dass die lokale Teilstrahlendosis (Di) der so erhaltenen zwei Strahlen (3, 3a, 3b, 3c, 3d, 3e, 3f) jeweils in unterschiedlichen, durch die jeweilige zweite Steuerungsebene definierten, Untervolumen ungleich Null ist,
wobei das Behandlungs-Planungssystem (208) konfiguriert ist, den folgenden Schritt durchzuführen: Festlegen von Strahlen (3, 3a, 3b, 3c, 3d, 3e, 3f) ohne Zuordnung zu zweiten Steuerungsebenen (2, 2a, 2b, 2c, 2d, 2e, 2f, 2g), wobei die Verarbeitungsvorrichtung konfiguriert ist, die folgenden Schritte durchzuführen:

- Korrigieren der Gesamtstrahlendosis durch Subtraktion der lokalen Teilstrahlendosen der Strahlen (3, 3a, 3b, 3c, 3d, 3e, 3f) ohne Zuordnung von der Gesamtstrahlendosis,
- Berechnen der lokalen Teilstrahlendosen (Di) für alle einer zweiten Steuerungsebene (2, 2a, 2b, 2c, 2d, 2e, 2f, 2g) zugeordneten Strahlen,

wobei die Strahlerzeugungsvorrichtung (201) konfiguriert ist, Strahlen (3, 3a, 3b, 3c, 3d, 3e, 3f) mit der von der Verarbeitungsvorrichtung (205) berechneten lokalen Teilstrahlendosis (Di) zu applizieren.

**25.** Strahlentherapieanlage nach Anspruch 24, welche konfiguriert ist, ein Verfahren nach einem der Ansprüche 13-21 durchzuführen.

## Claims

**1.** Method for calculating local partial radiation doses ($D_i$) in a radiotherapy facility, for the purpose of applying a total radiation dose ($D_{pr}$) in a target volume using a plurality of beams, wherein said method comprises:

- determining at least one first control plane for controlling the dosage of the beams (3, 3a, 3b, 3c, 3d, 3e, 3f), wherein each of the at least one first control planes (1) divides the target volume (4) into two subvolumes,
- in each case for the front side (10, 10a, 10b, 10c, 10d, 10e) and the rear side (11, 11a, 11b, 11c, 11d, 11e) of each of the at least one first control planes: assigning at least one beam,
- for each first control plane (1, 1a, 1b): determining a subvolume total radiation dose ($D_o$, $D_r$) for in each case each of the two subvolumes as a fraction ($F_o$, $F_r$) of the total radiation dose ($D_{pr}$),

wherein said method further comprises:

- determining at least one second control plane (2, 2a, 2b, 2c, 2d, 2e, 2f, 2g) for controlling the positioning of the beams, wherein each of the at least one second control planes (2, 2a, 2b, 2c, 2d, 2e, 2f, 2g) divides the target volume (4) into two subvolumes,
- assigning at least one beam (3, 3a, 3b, 3c, 3d, 3e, 3f) to each of the at least one second control planes (2, 2a, 2b, 2c, 2d, 2e, 2f, 2g),

wherein a beam (3, 3a, 3b, 3c, 3d, 3e, 3f) which is assigned to a second control plane (2, 2a, 2b, 2c, 2d, 2e, 2f, 2g) is divided into two by the respective second control plane in such a way that the local partial radiation dose ($D_i$) of the two beams (3, 3a, 3b, 3c, 3d, 3e, 3f) thus obtained is in each case unequal to zero in different subvolumes that are defined by the respective second control plane,
wherein said method further comprises:

- for at least one side of a first control plane:

  individually calculating the corresponding local partial radiation doses ($D_i$) of all the beams that are assigned to this first control plane (1, 1a, 1b), such that the sum of the local partial radiation doses of those beams which are assigned to that side of the respective first control plane (1, 1a, 1b) facing the respective subvolume gives the respective subvolume total radiation dose ($D_o$, $D_r$), and the sum of the local partial radiation doses of the remaining beams (3, 3a, 3b, 3c, 3d, 3e, 3f) that are assigned to this first control plane (1, 1a, 1b) gives the difference between the respective subvolume total radiation dose ($D_o$, $D_r$) and total radiation dose ($D_{pr}$).

2. Method according to claim 1, wherein a beam (3, 3a, 3b, 3c, 3d, 3e, 3f) is specifically assigned to one side of a first control plane (1, 1a, 1b) in each case.

3. Method according to one of the claims 1 or 2, wherein the local partial radiation doses ($D_i$) of a beam which is assigned to one side of a first control plane (1, 1a, 1b) are different in the subvolumes defined by said first control plane (1, 1a, 1b).

4. Method according to one of the preceding claims, wherein the first control planes have a finite thickness, and wherein beams which have different local partial radiation doses ($D_i$) on different sides of the respective first control plane (1, 1a, 1b) ensure a gradual transition of the local partial radiation dose ($D_i$) by virtue of a positional sequence of the local partial radiation dose ($D_i$) within the first control plane (1, 1a, 1b).

5. Method according to one of the preceding claims, wherein a radiation weighting factor ($w_i$) is determined for each beam (3, 3a, 3b, 3c, 3d, 3e, 3f), wherein the radiation weighting factors determine the relative ratios of the partial radiation doses ($D_i$) of various beams (3, 3a, 3b, 3c, 3d, 3e, 3f) to one another.

6. Method according to claim 5, further comprising within the target volume (4) for a specified side of a specified first control plane (1, 1a, 1b):

- determining first local weighting factors ($c_{pp}$) for each distance perpendicular to the specified first control plane, wherein the first local weighting factors ($c_{pp}$) define the fraction of the subvolume total radiation dose relative to the total radiation dose ($D_{pr}$),
- for each beam (3, 3a, 3b, 3c, 3d, 3e, 3f) that is assigned to the specified side of the specified first control plane (1, 1a, 1b): determining second local weighting factors ($c_{sp,i}$) for each distance, perpendicular to a second control plane (2, 2a, 2b, 2c, 2d, 2e, 2f, 2g), to which the respective beam (3, 3a, 3b, 3c, 3d, 3e, 3f) is assigned, wherein the second local weighting factors ($c_{sp,i}$) modify the radiation weighting factors ($w_i$) of the beams (3, 3a, 3b, 3c, 3d, 3e, 3f) as a function of the position relative to second control planes (2, 2a, 2b, 2c, 2d, 2e, 2f, 2g),
- for each beam that is assigned to the specified side of the specified first control plane (1, 1a, 1b): calculating the local partial radiation dose ($D_i$) for each subvolume for the respective beam on the basis of elements which are selected from the following group: radiation weighting factors ($w_i$), first local weighting factors ($c_{pp}$), second local weighting factors ($c_{sp,i}$), total radiation dose ($D_{pr}$).

7. Method according to claim 6, wherein the radiation weighting factors ($w_i$) are modified in value between 0% and 100% by the second local weighting factors ($c_{sp,i}$) in the calculation of the local partial radiation dose.

8. Method according to claim 6 or 7, wherein first and second control planes (2, 2a, 2b, 2c, 2d, 2e, 2f, 2g) have a thickness and the first and second local weighting factors vary in each case within the corresponding control plane as a function of the position.

9. Method according to one of the claims 5 to 8, wherein the calculation is location-specific and is performed for a side of a first control plane (1, 1a, 1b) according to the following formula:

$$D_i = \frac{w_i \prod_k c_{sp,i,k}}{\sum_{j=1}^{n} w_j \prod_l c_{sp,j,l}} c_{pp} D_{pr} ,$$

where $w_i$ designates the radiation weighting factors, $c_{pp}$ designates the corresponding location-specific first local weighting factor, $c_{pp}D_{pr}$ designates the subvolume total radiation dose, $D_{pr}$ designates the total radiation

dose, and $c_{sp}$ designates the location-specific second local weighting factors for all beams that are assigned to the corresponding side of the first control plane (1, 1a, 1b).

**10.** Method according to one of the preceding claims, further comprising: determining beams (3, 3a, 3b, 3c, 3d, 3e, 3f) that are not assigned to first or second control planes (2, 2a, 2b, 2c, 2d, 2e, 2f, 2g), wherein the partial radiation doses ($D_i$) of these beams (3, 3a, 3b, 3c, 3d, 3e, 3f) are deducted from the total radiation dose ($D_{pr}$) before the individual calculation.

**11.** Method according to claim 10, further comprising:

- determining radiation weighting factors ($w_i$) for beams (3, 3a, 3b, 3c, 3d, 3e, 3f) that are not assigned to first or second control planes (2, 2a, 2b, 2c, 2d, 2e, 2f, 2g), and
- adapting the total radiation dose ($D_{pr}$) for beams which are assigned to first and/or second control planes (2, 2a, 2b, 2c, 2d, 2e, 2f, 2g) on the basis of the radiation weighting factors ($w_i$) for beams (3, 3a, 3b, 3c, 3d, 3e, 3f) that are not assigned to first or second control planes (2, 2a, 2b, 2c, 2d, 2e, 2f, 2g).

**12.** Method for calculating local partial radiation doses ($D_i$) in a radiotherapy facility, for the purpose of applying a total radiation dose ($D_{pr}$) in a target volume (4) using a plurality of beams, wherein said method comprises:

- determining at least one second control plane (2, 2a, 2b, 2c, 2d, 2e, 2f, 2g) for controlling the positioning of the beams (3, 3a, 3b, 3c, 3d, 3e, 3f), wherein each of the at least one second control planes (2, 2a, 2b, 2c, 2d, 2e, 2f, 2g) divides the target volume (4) into two subvolumes,
- assigning at least one beam to each of the at least one second control planes (2, 2a, 2b, 2c, 2d, 2e, 2f, 2g),

wherein a beam (3, 3a, 3b, 3c, 3d, 3e, 3f) which is assigned to a second control plane (2, 2a, 2b, 2c, 2d, 2e, 2f, 2g) is divided into two by the respective second control plane in such a way that the local partial radiation dose ($D_i$) of the two beams (3, 3a, 3b, 3c, 3d, 3e, 3f) thus obtained is in each case unequal to zero in different subvolumes that are defined by the respective second control plane,
wherein said method further comprises:

- determining beams (3, 3a, 3b, 3c, 3d, 3e, 3f) that are not assigned to second control planes (2, 2a, 2b, 2c, 2d, 2e, 2f, 2g),
- correcting the total radiation dose by means of subtracting the local partial radiation doses of the beams (3, 3a, 3b, 3c, 3d, 3e, 3f) that are not assigned from the total radiation dose,
- calculating the local partial radiation doses ($D_i$) for all beams that are assigned to a second control plane (2, 2a, 2b, 2c, 2d, 2e, 2f, 2g).

**13.** Method according to claim 12, wherein a radiation weighting factor ($w_i$) is determined for each beam (3, 3a, 3b, 3c, 3d, 3e, 3f), wherein the radiation weighting factors determine the relative ratios of the local partial radiation doses ($D_i$) of various beams (3, 3a, 3b, 3c, 3d, 3e, 3f) to one another.

**14.** Method according to claim 13, wherein for the purpose of correcting the total radiation dose the radiation weighting factor of all beams (3, 3a, 3b, 3c, 3d, 3e, 3f) assigned to a second control plane (2, 2a, 2b, 2c, 2d, 2e, 2f, 2g) has a sum total of one.

**15.** Method according to one of the preceding claims, wherein the sum of the local partial radiation doses ($D_i$) of all beams (3, 3a, 3b, 3c, 3d, 3e, 3f) at every position within the target volume (4) is equal to the total radiation dose.

**16.** Method according to one of the preceding claims, wherein the second control planes (2, 2a, 2b, 2c, 2d, 2e, 2f, 2g) have a finite thickness, and wherein beams which are divided into two by a second control plane ensure a gradual transition of the local partial radiation dose ($D_i$) by virtue of a positional sequence of the local partial radiation dose ($D_i$) within the second control plane (2, 2a, 2b, 2c, 2d, 2e, 2f, 2g).

**17.** Method according to one of the preceding claims, wherein the local partial radiation dose ($D_i$) for a beam (3, 3a, 3b, 3c, 3d, 3e, 3f) which is assigned to a first control plane (1, 1a, 1b) is unequal to zero on the different sides of the first control plane (1, 1a, 1b), and wherein the local partial radiation dose for a beam (3, 3a, 3b, 3c, 3d, 3e, 3f) that is assigned to a second control plane (2, 2a, 2b, 2c, 2d, 2e, 2f, 2g) is equal to zero on precisely one of the sides of the second control plane (2, 2a, 2b, 2c, 2d, 2e, 2f, 2g).

**18.** Method according to one of the preceding claims, wherein the beams (3, 3a, 3b, 3c, 3d, 3e, 3f) are **characterised by** a beam source, wherein the beam source designates the position at which a beam (3, 3a, 3b, 3c, 3d, 3e, 3f) is generated.

**19.** Method according to claim 18, wherein two beams which are divided into two by a second control plane have essentially identical beam sources.

**20.** Method according to one of the preceding claims, wherein the second control planes (2, 2a, 2b, 2c, 2d, 2e, 2f, 2g) are determined in such a way that beams having a partial radiation dose ($D_i$) in a target volume (4) which is larger than the maximal target volume (4) that can be accessed by the beam (3, 3a, 3b, 3c, 3d, 3e, 3f) are divided into two by a second control plane.

**21.** Method according to one of the preceding claims, wherein the first control planes (1, 1a, 1b) designate patch planes of an irradiation plan and the second control planes (2, 2a, 2b, 2c, 2d, 2e, 2f, 2g) designate split planes of an irradiation plan.

**22.** Radiotherapy facility comprising a treatment planning system (208), a processing device (205) and a beam generating device (201),
wherein the treatment planning system (208) is so configured as to perform the following steps in order to calculate local partial radiation doses ($D_i$) for the purpose of applying a total radiation dose ($D_{pr}$) in a target volume (4) using a plurality of beams (3, 3a, 3b, 3c, 3d, 3e, 3f):

- determining at least one first control plane (1, 1a, 1b) for controlling the dosage of the beams, wherein each of the at least one first control planes (1, 1a, 1b) divides the target volume (4) into two subvolumes,
- in each case for the front side (10, 10a, 10b, 10c, 10d, 10e) and the rear side (11, 11a, 11b, 11c, 11d, 11e) of each of the at least one first control planes: assigning at least one beam,
- for each first control plane (1, 1a, 1b): determining a subvolume total radiation dose ($D_o$, $D_r$) for in each case each of the two subvolumes as a fraction ($F_o$, $F_r$) of the total radiation dose ($D_{pr}$),
- determining at least one second control plane (2, 2a, 2b, 2c, 2d, 2e, 2f, 2g) for controlling the positioning of the beams, wherein each of the at least one second control planes (2, 2a, 2b, 2c, 2d, 2e, 2f, 2g) divides the target volume (4) into two subvolumes,
- assigning at least one beam (3, 3a, 3b, 3c, 3d, 3e, 3f) to each of the at least one second control planes (2, 2a, 2b, 2c, 2d, 2e, 2f, 2g),

wherein a beam (3, 3a, 3b, 3c, 3d, 3e, 3f) which is assigned to a second control plane (2, 2a, 2b, 2c, 2d, 2e, 2f, 2g) is divided into two by the respective second control plane in such a way that the local partial radiation dose ($D_i$) of the two beams (3, 3a, 3b, 3c, 3d, 3e, 3f) thus obtained is in each case unequal to zero in different subvolumes that are defined by the respective second control plane,
wherein the processing device (205) is so configured as to perform the following steps:

- for at least one side of a first control plane: individually calculating the corresponding local partial radiation doses ($D_i$) of all the beams that are assigned to this first control plane (1, 1a, 1b), such that the sum of the local partial radiation doses of those beams which are assigned to that side of the respective first control plane (1, 1a, 1b) facing the respective subvolume gives the respective subvolume total radiation dose ($D_o$, $D_r$), and the sum of the local partial radiation doses of the remaining beams (3, 3a, 3b, 3c, 3d, 3e, 3f) that are assigned to this first control plane (1, 1a, 1b) gives the difference between the respective subvolume total radiation dose ($D_o$, $D_r$) and total radiation dose ($D_{pr}$),

wherein the beam generating device (201) is so configured as to apply beams (3, 3a, 3b, 3c, 3d, 3e, 3f) using the local partial radiation dose ($D_i$) calculated by the processing device (205).

**23.** Radiotherapy facility according to claim 22, being so configured as to perform a method according to one of the claims 2 to 11.

**24.** Radiotherapy facility comprising a treatment planning system (208), a processing device (205) and a beam generating device (201), wherein the treatment planning system (208) is so configured as to perform the following steps in order to calculate local partial radiation doses ($D_i$) for the purpose of applying a total radiation dose ($D_{pr}$) in a target volume (4) using a plurality of beams:

- determining at least one second control plane (2, 2a, 2b, 2c, 2d, 2e, 2f, 2g) for controlling the positioning of the beams, wherein each of the at least one second control planes (2, 2a, 2b, 2c, 2d, 2e, 2f, 2g) divides the target volume (4) into two subvolumes,
- assigning at least one beam (3, 3a, 3b, 3c, 3d, 3e, 3f) to each of the at least one second control planes (2, 2a, 2b, 2c, 2d, 2e, 2f, 2g),

wherein a beam (3, 3a, 3b, 3c, 3d, 3e, 3f) which is assigned to a second control plane (2, 2a, 2b, 2c, 2d, 2e, 2f, 2g) is divided into two by the respective second control plane in such a way that the local partial radiation dose ($D_i$) of the two beams (3, 3a, 3b, 3c, 3d, 3e, 3f) thus obtained is in each case unequal to zero in different subvolumes that are defined by the respective second control plane,
wherein the treatment planning system (208) is so configured as to perform the following step: determining beams (3, 3a, 3b, 3c, 3d, 3e, 3f) that are not assigned to second control planes (2, 2a, 2b, 2c, 2d, 2e, 2f, 2g),
wherein the processing device is so configured as to perform the following steps:

- correcting the total radiation dose by means of subtracting the local partial radiation doses of the beams (3, 3a, 3b, 3c, 3d, 3e, 3f) that are not assigned from the total radiation dose,
- calculating the local partial radiation doses ($D_i$) for all beams that are assigned to a second control plane (2, 2a, 2b, 2c, 2d, 2e, 2f, 2g),

wherein the beam generating device (201) is so configured as to apply beams (3, 3a, 3b, 3c, 3d, 3e, 3f) using the local partial radiation dose ($D_i$) calculated by the processing device (205).

**25.** Radiotherapy facility according to claim 24, being so configured as to perform a method according to one of the claims 13 to 21.

**Revendications**

**1.** Procédé pour calculer des doses de rayonnement partielles locales (Di) dans une installation de radiothérapie dans le but d'appliquer une dose de rayonnement totale ($D_{pr}$) dans un volume cible avec une pluralité de rayons, le procédé comprenant :

- la détermination d'au moins un premier plan de commande pour commander le dosage des rayons (3, 3a, 3b, 3c, 3d, 3e, 3f), chacun du au moins un premier plan de commande (1) divisant le volume cible (4) en deux sous-volumes,
- pour le côté antérieur (10, 10a, 10b, 10c, 10d, 10e) et le côté postérieur (11, 11a, 11b, 11c, 11d, 11e) de chacun du au moins un plan de commande : affectation d'au moins un rayon,
- pour chaque premier plan de commande (1, 1a, 1b) : détermination d'une dose de rayonnement totale de sous-volume ($D_o$, $D_r$) pour chacun des deux sous-volumes sous forme d'une fraction ($F_o$, $F_r$) de la dose de rayonnement totale ($D_{pr}$),

le procédé comprenant en outre :

- la détermination d'au moins un second plan de commande (2, 2a, 2b, 2c, 2d, 2e, 2f, 2g) pour commander le positionnement des rayons, chacun du au moins un second plan de commande (2, 2a, 2b, 2c, 2d, 2e, 2f, 2g) divisant le volume cible (4) en deux sous-volumes,
- affectation d'au moins un rayon (3, 3a, 3b, 3c, 3d, 3e, 3f) à chacun du au moins un second plan de commande (2, 2a, 2b, 2c, 2d, 2e, 2f, 2g),

un rayon (3, 3a, 3b, 3c, 3d, 3e, 3f) affecté à un second plan de commande (2, 2a, 2b, 2c, 2d, 2e, 2f, 2g) étant divisé en deux par le second plan de commande respectif, de telle sorte que la dose de rayonnement partielle locale ($D_i$) des deux rayons (3, 3a, 3b, 3c, 3d, 3e, 3f) ainsi obtenus, est à chaque fois non nulle dans des sous-volumes différents définis par le second plan de commande respectif,
le procédé comprenant en outre :

- pour au moins un côté d'un premier plan de commande : calcul isolé des doses de rayonnement partielles locales respectives ($D_i$) de tous les rayons affectés à ce premier plan de commande (1, 1a, 1b), de telle sorte que la somme des doses de rayonnement partielles locales des rayons affectés au côté tourné vers le sous-volume respectif du premier plan de commande respectif (1, 1a, 1b) donne la dose de rayonnement totale de sous-volume respective ($D_o$, $D_r$) et que la somme des doses de rayonnement partielles locales du reste des rayons (3, 3a, 3b, 3c, 3d, 3e, 3f) affectés à ce premier plan de commande (1, 1a, 1b) donne la différence entre la dose de rayonnement totale de sous-volume respective ($D_o$, $D_r$) et la dose de rayonnement totale ($D_{pr}$).

**2.** Procédé selon la revendication 1, dans lequel un rayon (3, 3a, 3b, 3c, 3d, 3e, 3f) est affecté à chaque fois explicitement à un côté d'un premier plan de commande (1, 1a, 1b).

**3.** Procédé selon l'une des revendications 1 ou 2, dans lequel les doses de rayonnement partielles locales ($D_i$) d'un rayon affecté à un côté d'un premier plan de commande (1, 1a, 1b) sont différentes dans les sous-volumes définis par ce premier plan de commande (1, 1a, 1b).

**4.** Procédé selon l'une des revendications précédentes, dans lequel les premiers plans de commande présentent une épaisseur finie et dans lequel les rayons présentant des doses de rayonnement partielles locales ($D_i$) différentes sur des côtés différents du premier plan de commande respectif (1, 1a, 1b) garantissent, grâce à une évolution locale de la dose de rayonnement partielle locale ($D_i$) à l'intérieur du premier plan de commande (1, 1a, 1b), une transition progressive de la dose de rayonnement partielle locale ($D_i$).

**5.** Procédé selon l'une des revendications précédentes, dans lequel un poids de rayonnement ($w_i$) est défini pour chaque rayon (3, 3a, 3b, 3c, 3d, 3e, 3f), les poids de rayonnement définissant les rapports relatifs des doses de rayonnement partielles locales ($D_i$) de différents rayons (3, 3a, 3b, 3c, 3d, 3e, 3f) les unes par rapport aux autres.

**6.** Procédé selon la revendication 5, comprenant en outre, à l'intérieur du volume cible (4), pour un côté déterminé d'un premier plan de commande déterminé (1, 1a, 1b) :

- la détermination de premiers facteurs de pondération locale ($c_{pp}$) pour chaque distance perpendiculaire au premier plan de commande déterminé, les premiers facteurs de pondération locale ($c_{pp}$) définissant la fraction de la dose de rayonnement totale de sous-volume dans la dose de rayonnement totale ($D_{pr}$),
- pour chaque rayon (3, 3a, 3b, 3c, 3d, 3e, 3f) affecté au côté déterminé du premier plan de commande déterminé (1, 1a, 1b) : détermination de seconds facteurs de pondération locale ($C_{sp,i}$) pour chaque distance perpendiculaire à un second plan de commande (2, 2a, 2b, 2c, 2d, 2e, 2f, 2g) auquel le rayon respectif (3, 3a, 3b, 3c, 3d, 3e, 3f) est affecté, les seconds facteurs de pondération locale ($c_{sp,i}$) modifiant les poids de rayonnement ($w_i$) des rayons (3, 3a, 3b, 3c, 3d, 3e, 3f) en fonction de la position par rapport aux seconds plan de commande (2, 2a, 2b, 2c, 2d, 2e, 2f, 2g),
- pour chaque rayon affecté au côté déterminé du premier plan de commande déterminé (1, 1a, 1b) : calcul de la dose de rayonnement partielle locale ($D_i$) pour chaque sous-volume pour le rayon respectif, sur la base d'éléments choisis dans le groupe suivant : facteurs de pondération de rayonnement ($w_i$), premiers facteurs de pondération locale ($c_{pp}$), seconds facteurs de pondération locale ($c_{sp,i}$), dose de rayonnement totale ($D_{pr}$).

**7.** Procédé selon la revendication 6, dans lequel, lors du calcul de la dose de rayonnement partielle locale, les seconds facteurs de pondération locale ($c_{sp,i}$) modifient les facteurs de pondération de rayonnement ($w_i$) entre 0% et 100 % de leur valeur.

**8.** Procédé selon la revendication 6 ou 7, dans lequel les premiers et seconds plans de commande (2, 2a, 2b, 2c, 2d, 2e, 2f, 2g) présentent une épaisseur, et les premiers et seconds facteurs de pondération locale varient chacun à l'intérieur du plan de commande correspondant en fonction de la position.

**9.** Procédé selon l'une des revendications 5 à 8, dans lequel le calcul est effectué en résolution spatiale selon la formule suivante, pour un côté d'un premier plan de commande (1, 1a, 1b) :

$$D_i = \frac{w_i \prod_k c_{sp,i,k}}{\sum_{j=1}^{n} w_j \prod_l c_{sp,j,l}} c_{pp} D_{pr} \quad ,$$

$w_i$ désignant les facteurs de pondération de rayonnement, $c_{pp}$ le premier facteur de pondération locale correspondant en résolution spatiale, $c_{pp}D_{pr}$ la dose de rayonnement totale de sous-volume, $D_{pr}$ désignant la dose de rayonnement totale et $c_{sp}$ les seconds facteurs de pondération locale en résolution spatiale pour tous les rayons affectés au côté correspondant du premier plan de commande (1, 1a, 1b).

**10.** Procédé selon l'une des revendications précédentes, comprenant en outre : la détermination de rayons (3, 3a, 3b, 3c, 3d, 3e, 3f) sans affectation à des premiers ou seconds plans de commande (2, 2a, 2b, 2c, 2d, 2e, 2f, 2g), les doses de rayonnement partielles ($D_i$) de ces rayons (3, 3a, 3b, 3c, 3d, 3e, 3f) étant soustraites de la dose de rayonnement totale ($D_{pr}$) avant le calcul isolé.

**11.** Procédé selon la revendication 10, comprenant en outre :

- la détermination de facteurs de pondération de rayonnement ($w_i$) pour des rayons (3, 3a, 3b, 3c, 3d, 3e, 3f) sans affectation à des premiers ou seconds plans de commande (2, 2a, 2b, 2c, 2d, 2e, 2f, 2g) et
- ajustement de la dose de rayonnement totale ($D_{pr}$) pour des rayons affectés à des premiers et/ou seconds plans de commande (2, 2a, 2b, 2c, 2d, 2e, 2f, 2g), sur la base des facteurs de pondération de rayonnement ($w_i$) pour des rayons (3, 3a, 3b, 3c, 3d, 3e, 3f) sans affectation à des premiers ou seconds plans de commande (2, 2a, 2b, 2c, 2d, 2e, 2f, 2g).

**12.** Procédé pour calculer des doses de rayonnement partielles locales ($D_i$) dans une installation de radiothérapie dans le but d'appliquer une dose de rayonnement totale ($D_{pr}$) dans un volume cible (4) avec une pluralité de rayons, le procédé comprenant :

- la détermination d'au moins un second plan de commande (2, 2a, 2b, 2c, 2d, 2e, 2f, 2g) pour commander le positionnement des rayons, chacun du au moins un second plan de commande (2, 2a, 2b, 2c, 2d, 2e, 2f, 2g) divisant le volume cible (4) en deux sous-volumes,
- affectation d'au moins un rayon à chacun des au moins un seconds plans de commande (2, 2a, 2b, 2c, 2d, 2e, 2f, 2g),

un rayon (3, 3a, 3b, 3c, 3d, 3e, 3f) affecté à un second plan de commande (2, 2a, 2b, 2c, 2d, 2e, 2f, 2g) étant divisé en deux par le second plan de commande respectif, de telle sorte que la dose de rayonnement partielle locale (Di) des deux rayons (3, 3a, 3b, 3c, 3d, 3e, 3f) ainsi obtenus est à chaque fois non nulle dans des sous-volumes différents définis par le second plan de commande respectif,
le procédé comprenant en outre :

- la détermination de rayons (3, 3a, 3b, 3c, 3d, 3e, 3f) sans affectation à des seconds plans de commande (2, 2a, 2b, 2c, 2d, 2e, 2f, 2g),
- la correction de la dose de rayonnement totale par soustraction des doses de rayonnement partielles locales des rayons (3, 3a, 3b, 3c, 3d, 3e, 3f) sans affectation de la dose de rayonnement totale,
- le calcul des doses de rayonnement partielles locales (Di) pour tous les rayons affectés à un second plan de commande (2, 2a, 2b, 2c, 2d, 2e, 2f, 2g).

**13.** Procédé selon la revendication 12, dans lequel un poids de rayonnement ($w_i$) est déterminé pour chaque rayon (3, 3a, 3b, 3c, 3d, 3e, 3f), les poids de rayonnement définissant les rapports relatifs des doses de rayonnement partielles ($D_i$) de différents rayons (3, 3a, 3b, 3c, 3d, 3e, 3f) les unes par rapport aux autres.

**14.** Procédé selon la revendication 13, dans lequel, pour corriger la dose de rayonnement totale, le poids de rayonnement de tous les rayons (3, 3a, 3b, 3c, 3d, 3e, 3f) affectés à un second plan de commande (2, 2a, 2b, 2c, 2d, 2e, 2f, 2g) est au total égal à un.

**15.** Procédé selon l'une des revendications précédentes, dans lequel la somme des doses de rayonnement partielles locales ($D_i$) de tous les rayons (3, 3a, 3b, 3c, 3d, 3e, 3f), à chaque position à l'intérieur du volume cible (4), est égale à la dose de rayonnement totale.

**16.** Procédé selon l'une des revendications précédentes, dans lequel les seconds plans de commande (2, 2a, 2b, 2c, 2d, 2e, 2f, 2g) présentent une épaisseur finie et dans lequel les rayons divisés en deux par un second plan de commande garantissent, grâce à une évolution locale de la dose de rayonnement partielle locale ($D_i$) à l'intérieur du second plan de commande (2, 2a, 2b, 2c, 2d, 2e, 2f, 2g), une transition progressive de la dose de rayonnement

partielle locale ($D_i$).

**17.** Procédé selon l'une des revendications précédentes, dans lequel la dose de rayonnement partielle locale ($D_i$) pour un rayon (3, 3a, 3b, 3c, 3d, 3e, 3f) affecté à un premier plan de commande (1, 1a, 1b) est non nulle sur les différents côtés du premier plan de commande (1, 1a, 1b), et dans lequel la dose de rayonnement partielle locale pour un rayon (3, 3a, 3b, 3c, 3d, 3e, 3f) affecté à un second plan de commande (2, 2a, 2b, 2c, 2d, 2e, 2f, 2g) est nulle sur précisément un des côtés du second plan de commande (2, 2a, 2b, 2c, 2d, 2e, 2f, 2g).

**18.** Procédé selon l'une des revendications précédentes, dans lequel les rayons (3, 3a, 3b, 3c, 3d, 3e, 3f) sont **caractérisés par** un point de départ, le point de départ d'un rayon désignant la position à laquelle un rayon (3, 3a, 3b, 3c, 3d, 3e, 3f) est généré.

**19.** Procédé selon la revendication 18, dans lequel deux rayons divisés en deux par un second plan de commande ont des points de départs sensiblement identiques.

**20.** Procédé selon l'une des revendications précédentes, dans lequel les seconds plans de commande (2, 2a, 2b, 2c, 2d, 2e, 2f, 2g) sont déterminés de telle sorte que les rayons présentant une dose de rayonnement partielle ($D_i$) dans un volume cible (4), lequel est plus grand que le volume cible (4) maximum auquel le rayon (3, 3a, 3b, 3c, 3d, 3e, 3f) peut accéder, sont divisés en deux par un second plan de commande.

**21.** Procédé selon l'une des revendications précédentes, dans lequel les premiers plans de commande (1, 1a, 1b) désignent des plans « patch » d'un plan d'irradiation et les seconds plans de commande (2, 2a, 2b, 2c, 2d, 2e, 2f, 2g) désignent des plans « split » d'un plan d'irradiation.

**22.** Installation de radiothérapie, comprenant un système de planification de traitement (208), un dispositif de traitement informatique (205) et un dispositif générateur de rayons (201),
le système de planification de traitement (208), dans le but de calculer des doses de rayonnement partielles locales ($D_i$) pour appliquer une dose de rayonnement totale ($D_{pr}$) dans un volume cible (4) avec une pluralité de rayons (3, 3a, 3b, 3c, 3d, 3e, 3f), étant configuré pour effectuer les étapes suivantes :

- détermination d'au moins un premier plan de commande (1, 1a, 1b) pour commander le dosage des rayons, chacun du au moins un premier plan de commande (1, 1a, 1b) divisant le volume cible (4) en deux sous-volumes,
- pour le côté antérieur (10, 10a, 10b, 10c, 10d, 10e) et le côté postérieur (11, 11a, 11b, 11c, 11d, 11e) de chacun du au moins un plan de commande : affectation d'au moins un rayon,
- pour chaque premier plan de commande (1, 1a, 1b) : détermination d'une dose de rayonnement totale de sous-volume ($D_o$, $D_r$) pour chacun des deux sous-volumes sous forme d'une fraction ($F_o$, $F_r$) de la dose de rayonnement totale ($D_{pr}$),
- détermination d'au moins un second plan de commande (2, 2a, 2b, 2c, 2d, 2e, 2f, 2g) pour commander le positionnement des rayons, chacun du au moins un second plan de commande (2, 2a, 2b, 2c, 2d, 2e, 2f, 2g) divisant le volume cible (4) en deux sous-volumes,
- affectation d'au moins un rayon (3, 3a, 3b, 3c, 3d, 3e, 3f) à chacun du au moins un second plan de commande (2, 2a, 2b, 2c, 2d, 2e, 2f, 2g),

un rayon (3, 3a, 3b, 3c, 3d, 3e, 3f) affecté à un second plan de commande (2, 2a, 2b, 2c, 2d, 2e, 2f, 2g) étant divisé en deux par le second plan de commande respectif, de telle sorte que la dose de rayonnement partielle locale (Di) des deux rayons (3, 3a, 3b, 3c, 3d, 3e, 3f) ainsi obtenus est à chaque fois non nulle dans des sous-volumes différents définis par le second plan de commande respectif,
le dispositif de traitement informatique (205) étant configuré pour effectuer l'étape suivante :

- pour au moins un côté d'un premier plan de commande : calcul isolé des doses de rayonnement partielles locales respectives ($D_i$) de tous les rayons affectés à ce premier plan de commande (1, 1a, 1b), de telle sorte que la somme des doses de rayonnement partielles locales des rayons affectés au côté tourné vers le sous-volume respectif du premier plan de commande respectif (1, 1a, 1b), donne la dose de rayonnement totale de sous-volume respective ($D_o$, $D_r$) et que la somme des doses de rayonnement partielles locales du reste des rayons (3, 3a, 3b, 3c, 3d, 3e, 3f) affectés à ce premier plan de commande (1, 1a, 1b), donne la différence entre la dose de rayonnement totale de sous-volume respective ($D_o$, $D_r$) et la dose de rayonnement totale ($D_{pr}$),

le dispositif générateur de rayons (201) étant configuré pour appliquer des rayons (3, 3a, 3b, 3c, 3d, 3e, 3f) avec la dose de rayonnement partielle locale ($D_i$) calculée par le dispositif de traitement informatique (205).

**23.** Installation de radiothérapie selon la revendication 22, laquelle est configurée pour mettre en oeuvre un procédé selon l'une des revendications 2 à 11.

**24.** Installation de radiothérapie, comprenant un système de planification de traitement (208), un dispositif de traitement informatique (205) et un dispositif générateur de rayons (201),
le système de planification de traitement (208), dans le but de calculer des doses de rayonnement partielles locales ($D_i$) pour appliquer une dose de rayonnement totale ($D_{pr}$) dans un volume cible (4) avec une pluralité de rayons, étant configuré pour effectuer les étapes suivantes :

- détermination d'au moins un second plan de commande (2, 2a, 2b, 2c, 2d, 2e, 2f, 2g) pour commander le positionnement des rayons, chacun du au moins un second plan de commande (2, 2a, 2b, 2c, 2d, 2e, 2f, 2g) divisant le volume cible (4) en deux sous-volumes,
- affectation d'au moins un rayon (3, 3a, 3b, 3c, 3d, 3e, 3f) à chacun du au moins un second plan de commande (2, 2a, 2b, 2c, 2d, 2e, 2f, 2g),

un rayon (3, 3a, 3b, 3c, 3d, 3e, 3f) affecté à un second plan de commande (2, 2a, 2b, 2c, 2d, 2e, 2f, 2g) étant divisé en deux par le second plan de commande respectif, de telle sorte que la dose de rayonnement partielle locale (Di) des deux rayons (3, 3a, 3b, 3c, 3d, 3e, 3f) ainsi obtenus est à chaque fois non nulle dans des sous-volumes différents définis par le second plan de commande respectif,
le système de planification de traitement (208) étant configuré pour effectuer l'étape suivante : détermination de rayons (3, 3a, 3b, 3c, 3d, 3e, 3f) sans affectation à des seconds plans de commande (2, 2a, 2b, 2c, 2d, 2e, 2f, 2g),
le dispositif de traitement informatique étant configuré pour effectuer les étapes suivantes :

- correction de la dose de rayonnement totale par soustraction des doses de rayonnement partielles locales des rayons (3, 3a, 3b, 3c, 3d, 3e, 3f) sans affectation de la dose de rayonnement totale,
- calcul des doses de rayonnement partielles locales (Di) pour tous les rayons affectés à un second plan de commande (2, 2a, 2b, 2c, 2d, 2e, 2f, 2g),

le dispositif générateur de rayons (201) étant configuré pour appliquer des rayons (3, 3a, 3b, 3c, 3d, 3e, 3f) avec la dose de rayonnement partielle locale (Di) calculée par le dispositif de traitement informatique (205).

**25.** Installation de radiothérapie selon la revendication 24, laquelle est configurée pour mettre en oeuvre un procédé selon l'une des revendications 13 à 21.

FIG 1

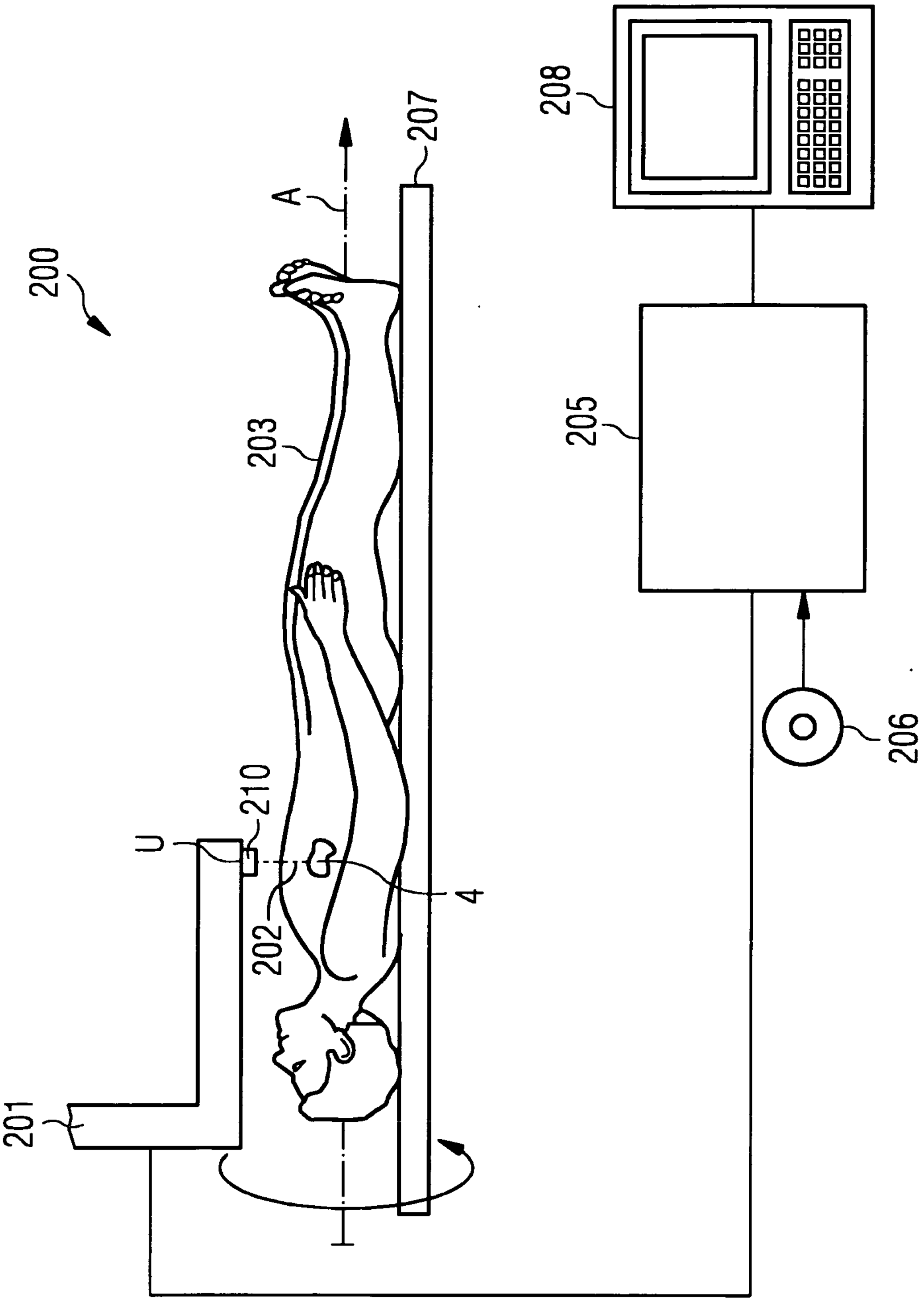

FIG 2

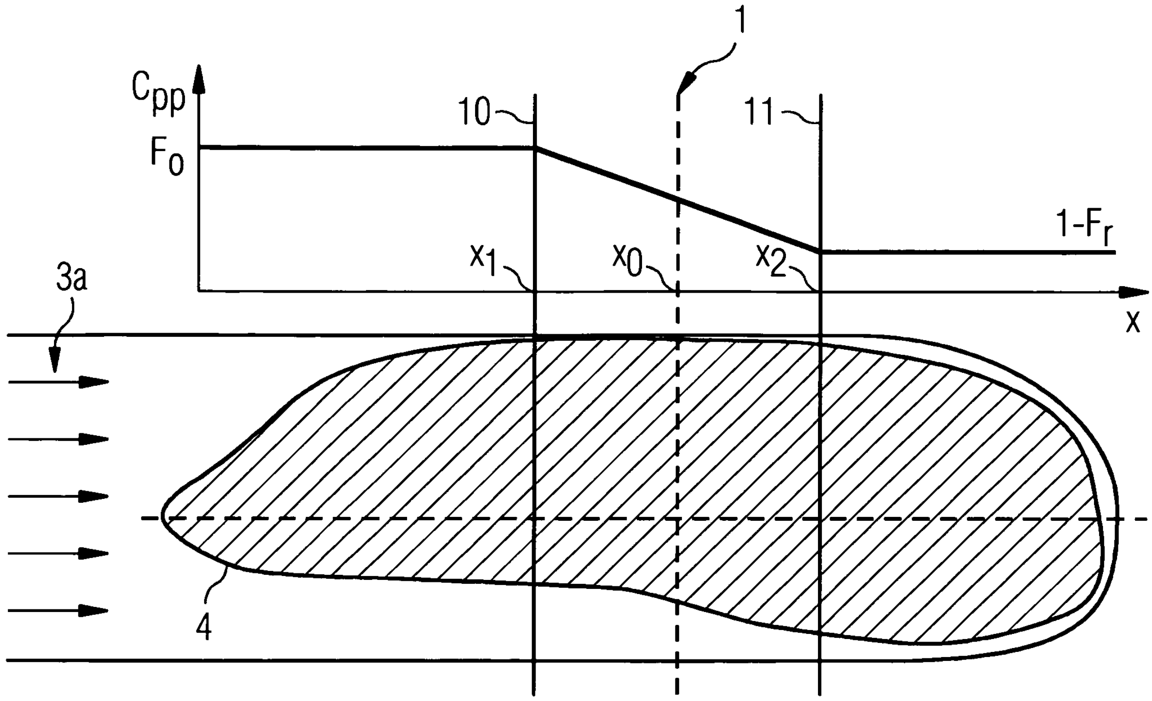

FIG 3

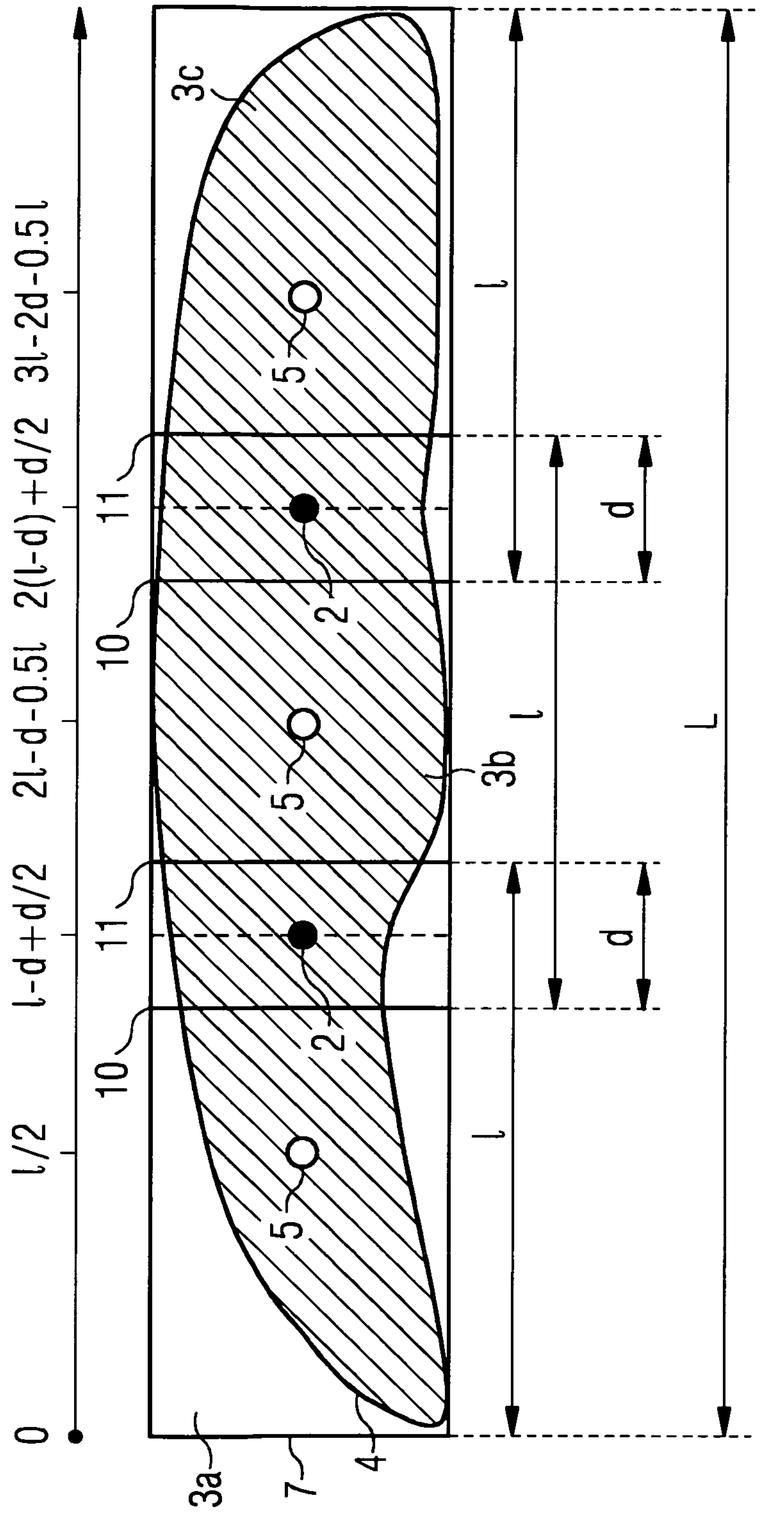

FIG 4

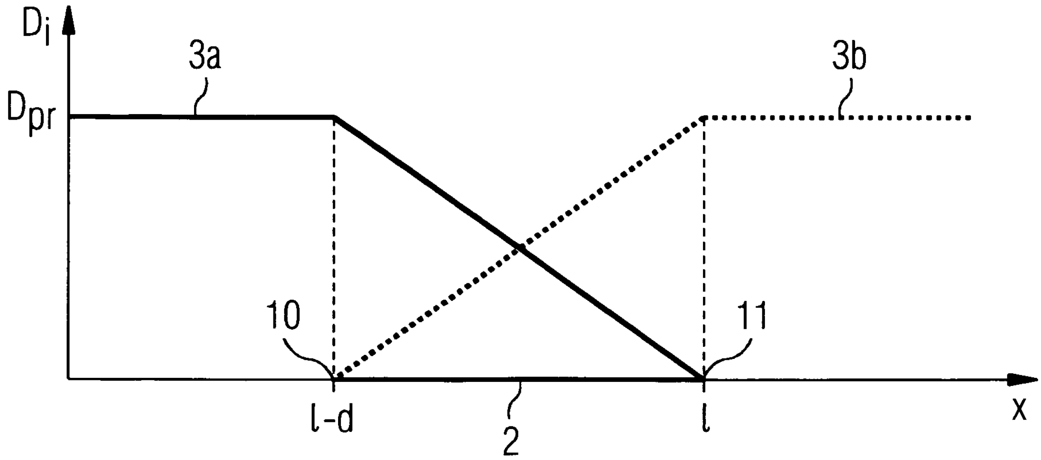


FIG 5

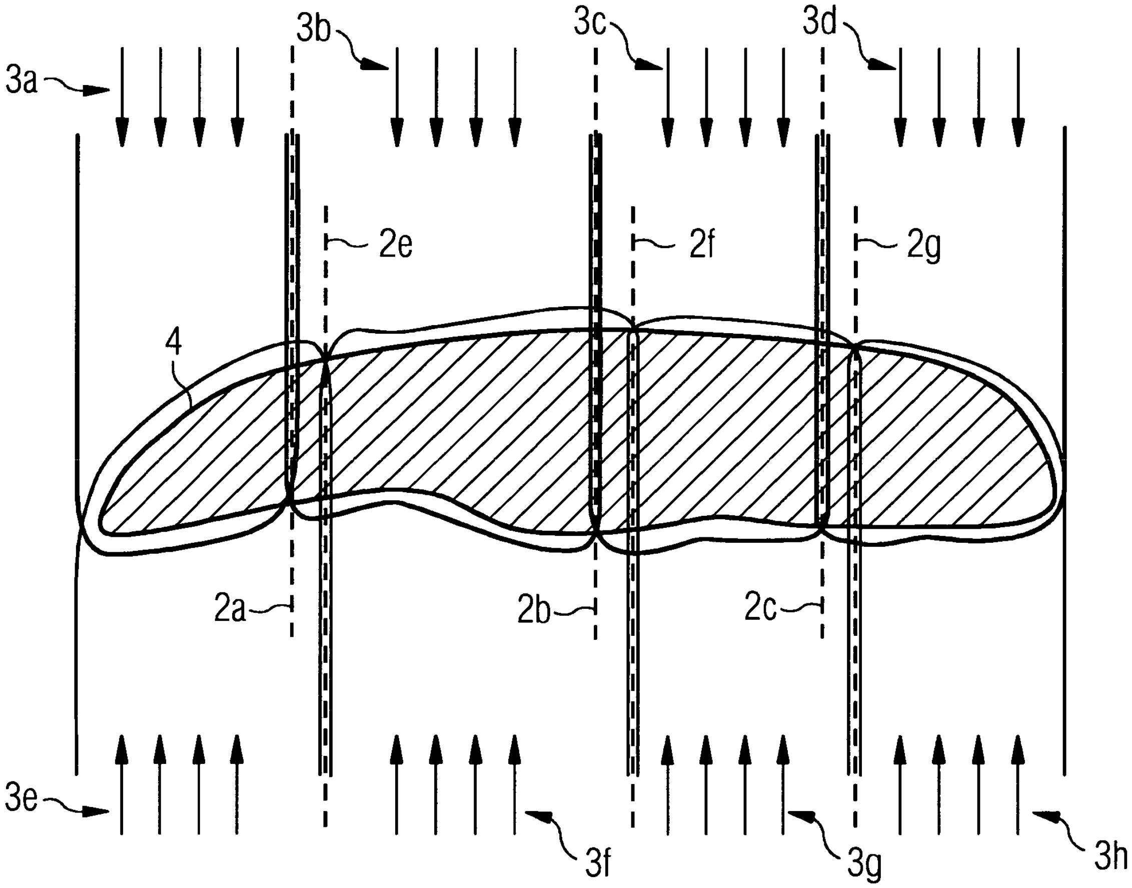

FIG 6

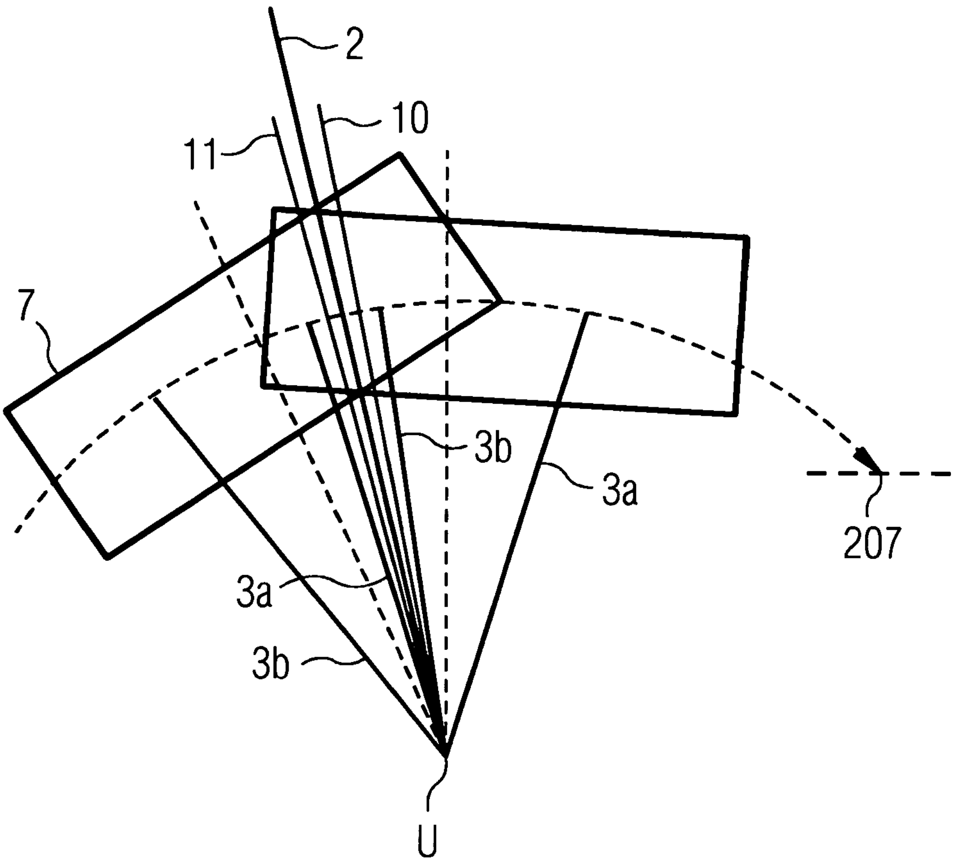

FIG 7

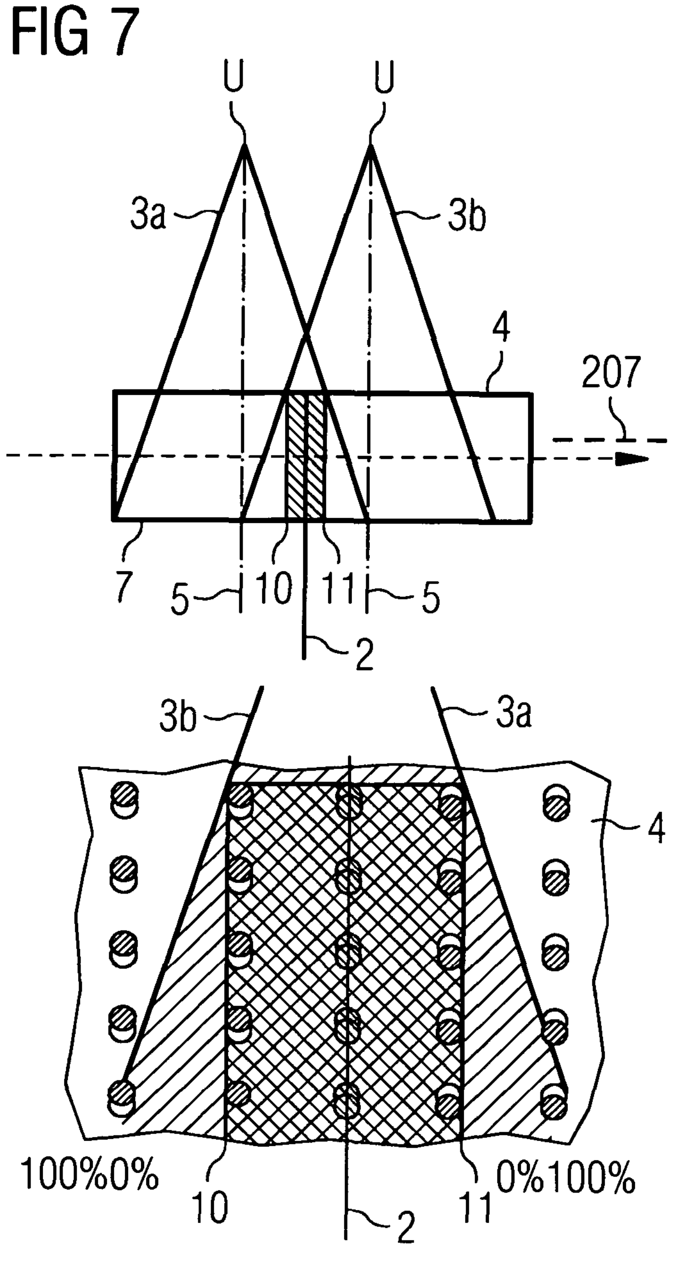

FIG 8

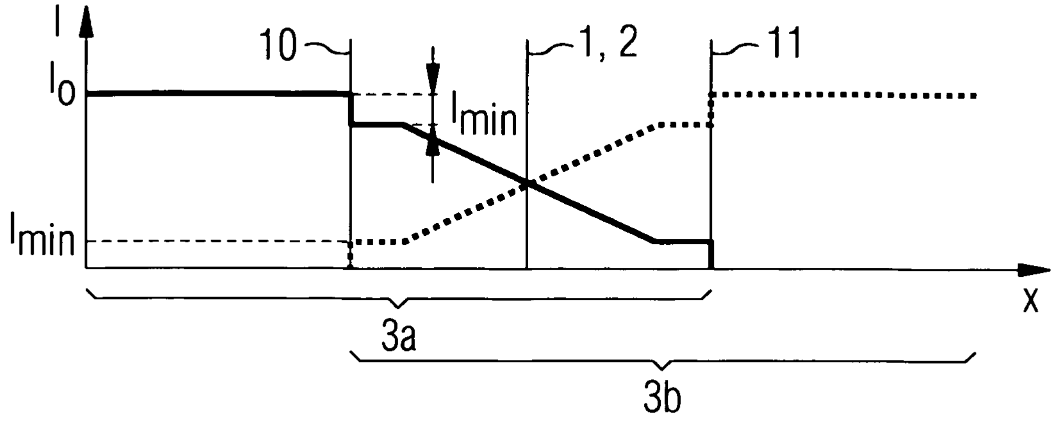


FIG 9

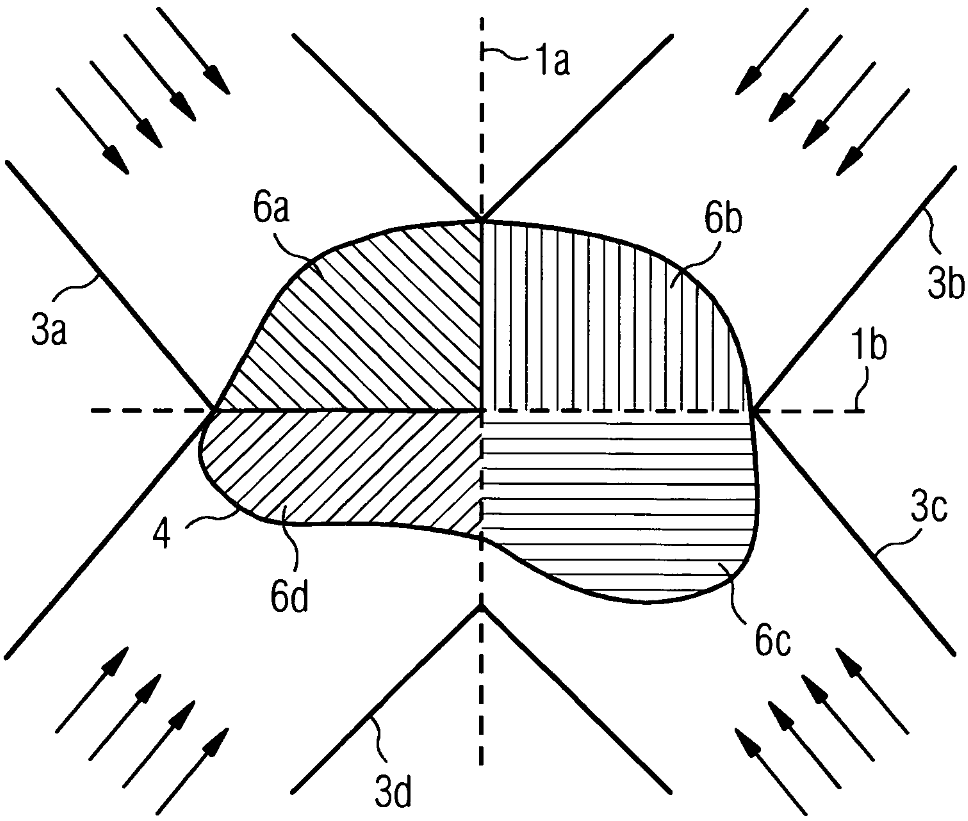

FIG 10

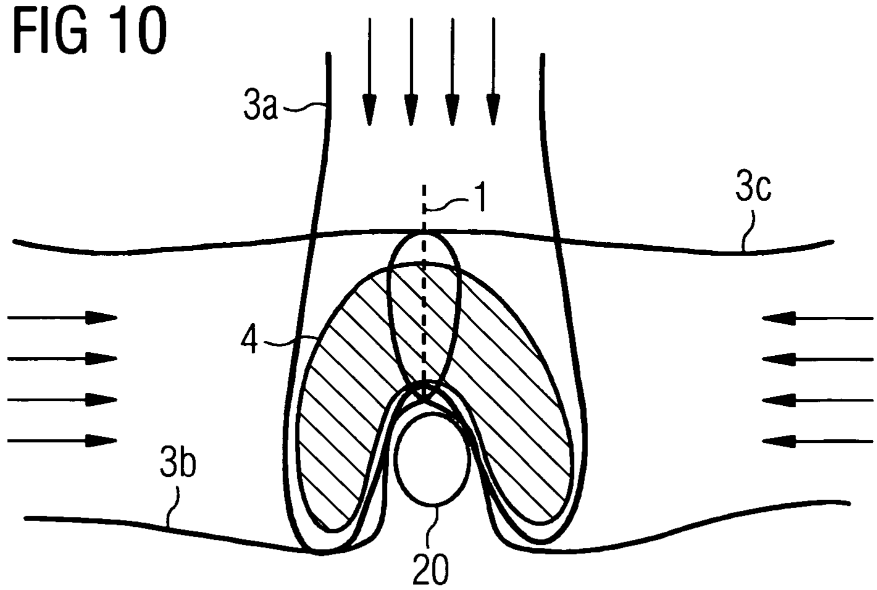


FIG 11

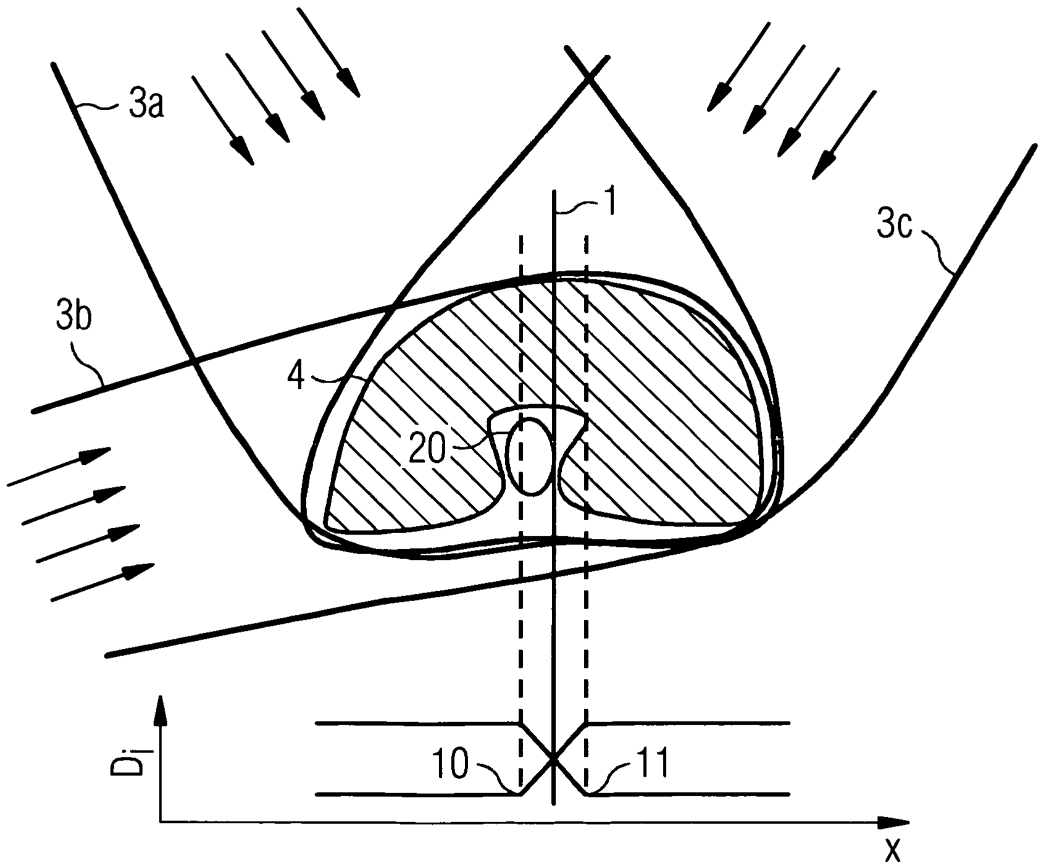

FIG 12

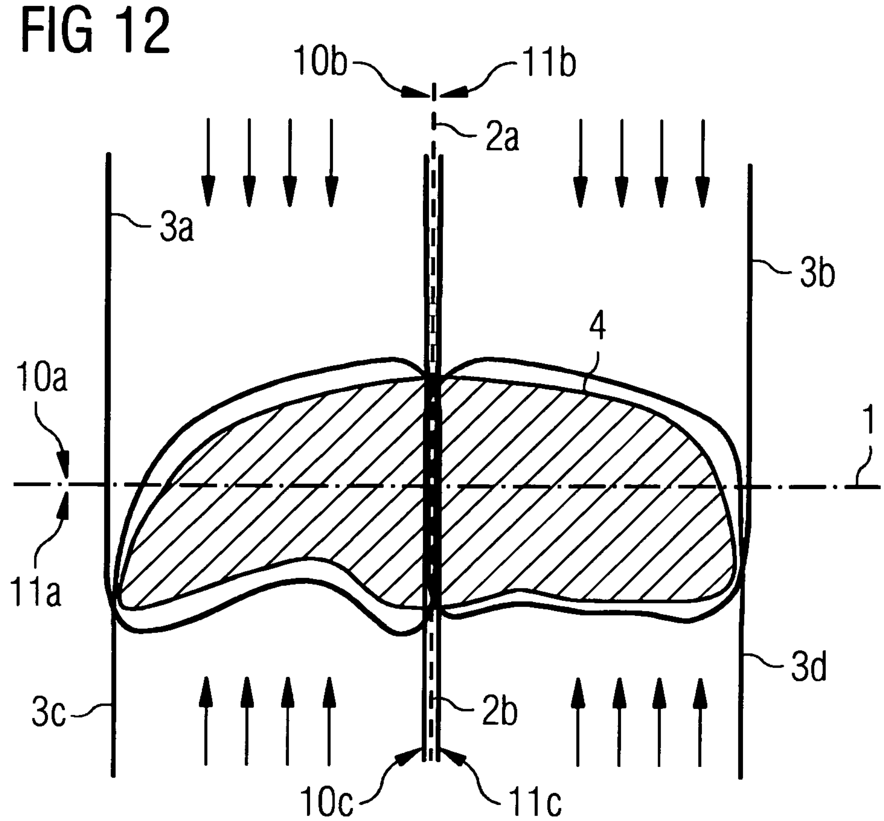
10b
11b
2a
3a
3b
4
10a
1
11a
3d
3c
2b
10c
11c

FIG 13

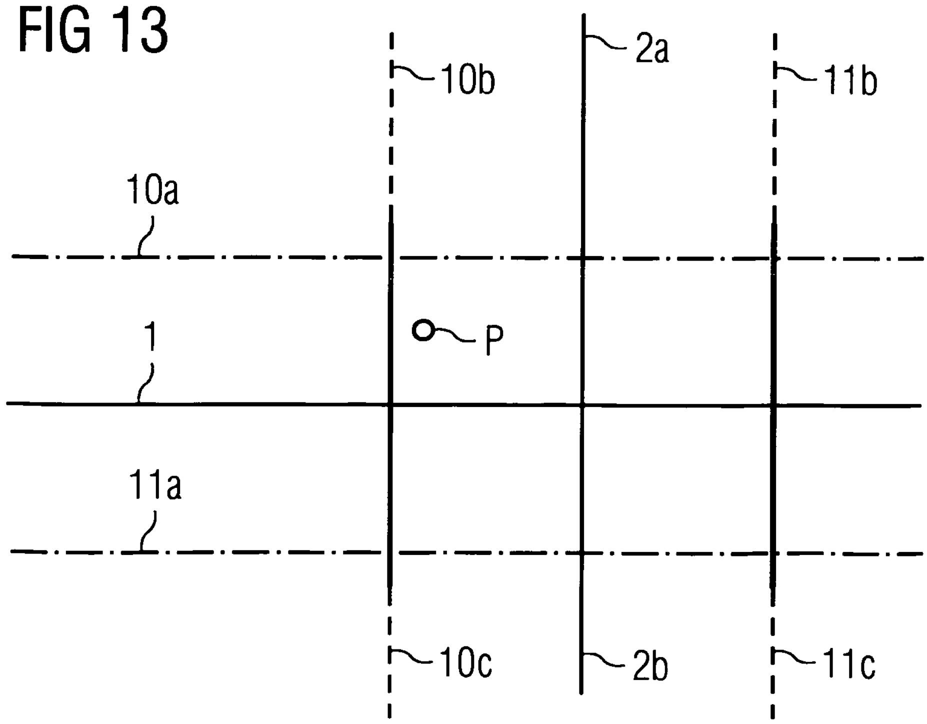
2a
10b
11b
10a
P
1
11a
10c
2b
11c

FIG 14

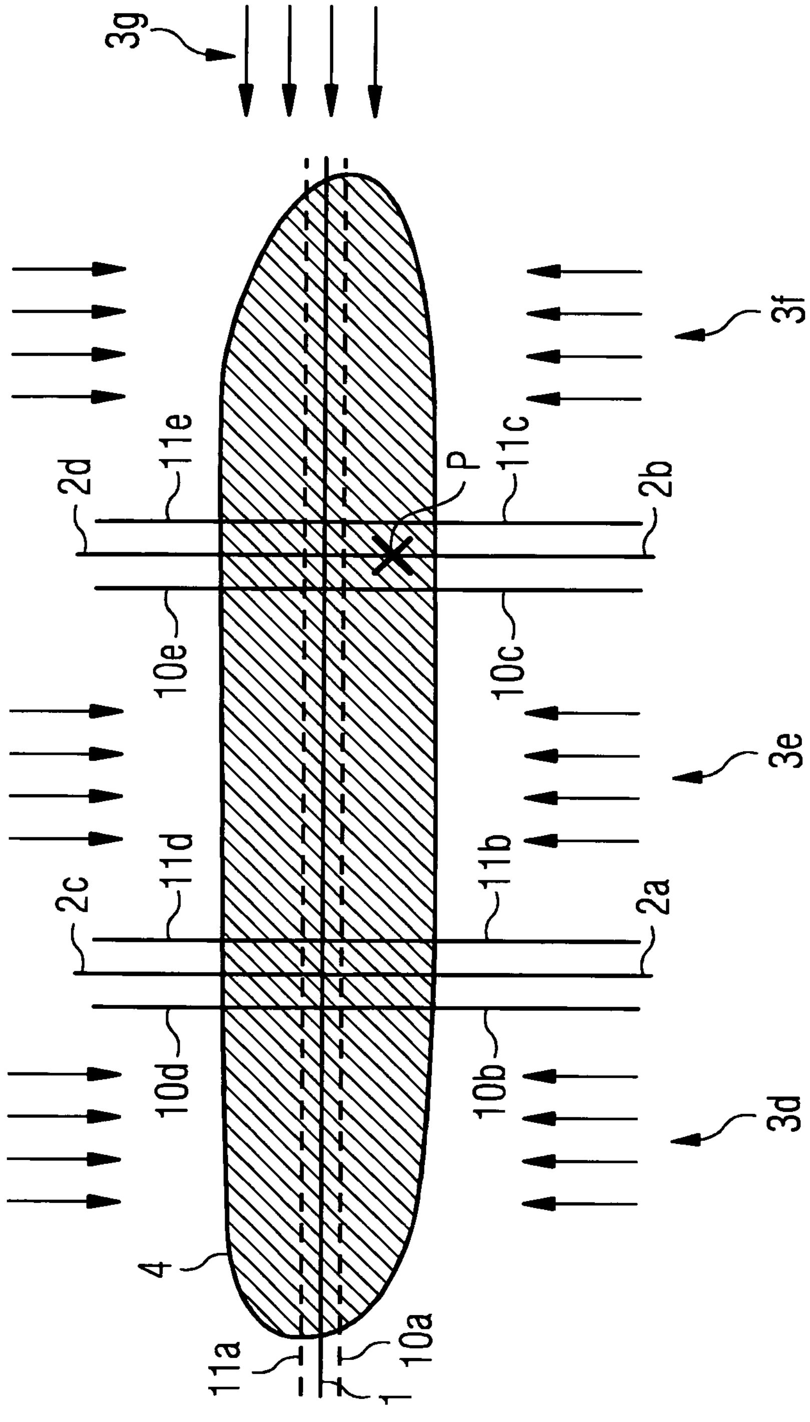

3g
3c
3f
2d
11e
P
11c
2b
10e
10c
3b
3e
2c
11d
11b
2a
10d
10b
3a
3d
4
11a
1
10a

FIG 15A

FIG 15

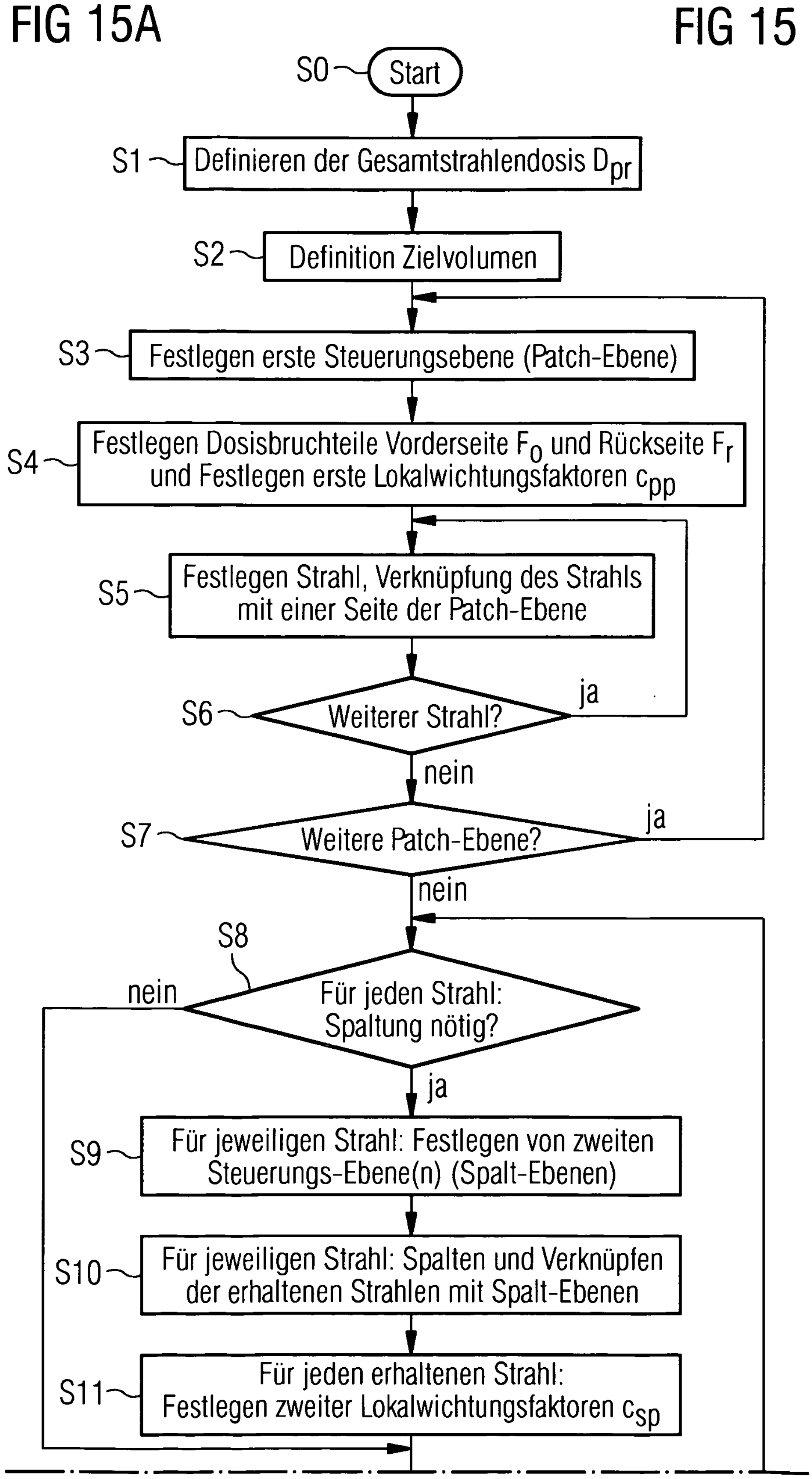


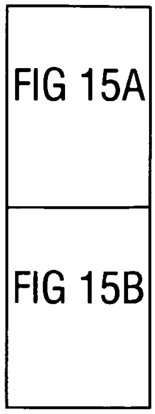

FIG 15B

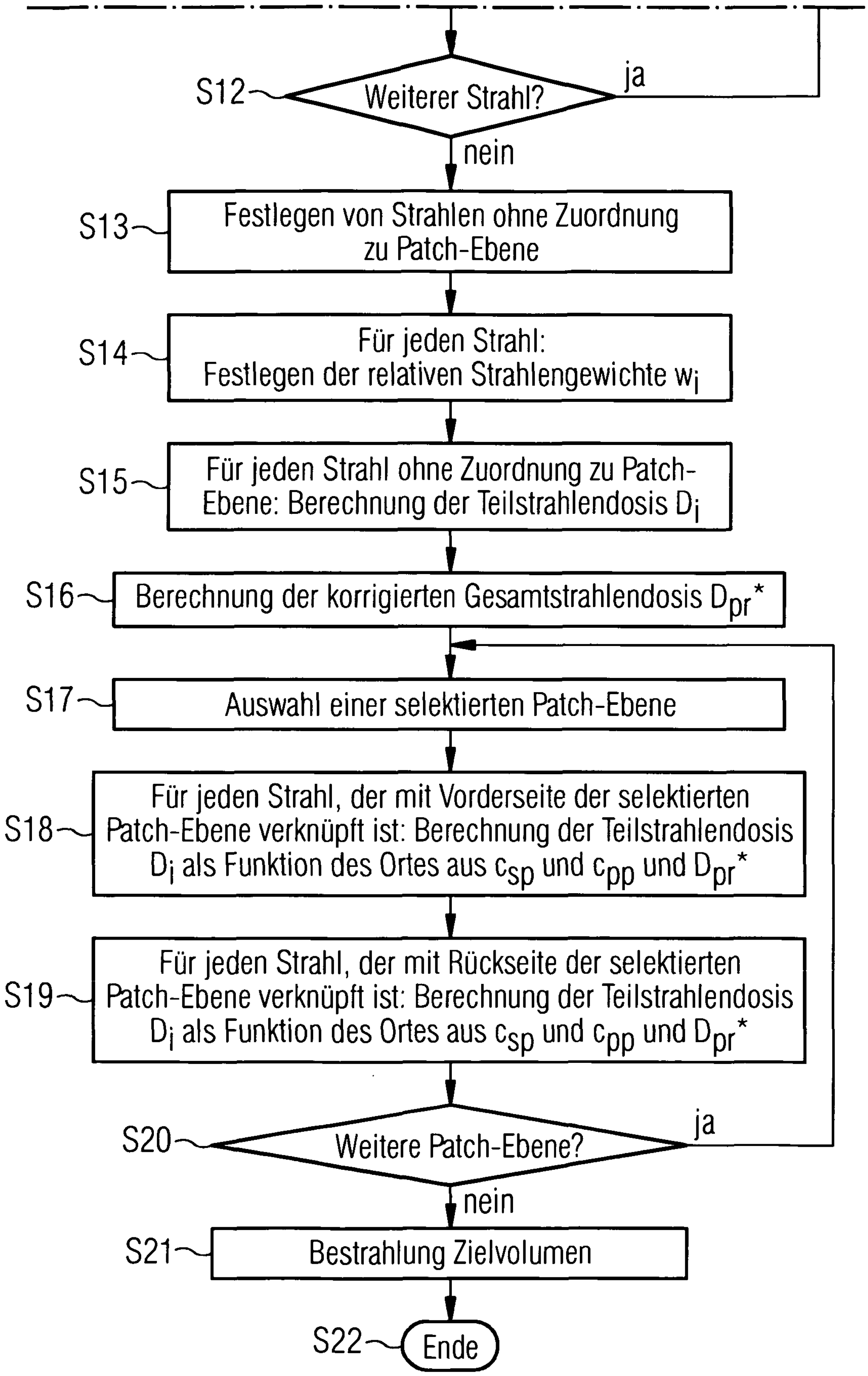
S12
Weiterer Strahl?
ja
nein
S13
Festlegen von Strahlen ohne Zuordnung zu Patch-Ebene
S14
Für jeden Strahl: Festlegen der relativen Strahlengewichte $w_i$
S15
Für jeden Strahl ohne Zuordnung zu Patch-Ebene: Berechnung der Teilstrahlendosis $D_i$
S16
Berechnung der korrigierten Gesamtstrahlendosis $D_{pr}^*$
S17
Auswahl einer selektierten Patch-Ebene
S18
Für jeden Strahl, der mit Vorderseite der selektierten Patch-Ebene verknüpft ist: Berechnung der Teilstrahlendosis $D_i$ als Funktion des Ortes aus $c_{sp}$ und $c_{pp}$ und $D_{pr}^*$
S19
Für jeden Strahl, der mit Rückseite der selektierten Patch-Ebene verknüpft ist: Berechnung der Teilstrahlendosis $D_i$ als Funktion des Ortes aus $c_{sp}$ und $c_{pp}$ und $D_{pr}^*$
S20
Weitere Patch-Ebene?
ja
nein
S21
Bestrahlung Zielvolumen
S22
Ende

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **Q. WU et al.** *Phys. Med. Biol.,* 2000, vol. 45, 1731-1740
- **E.B. HUG et al.** *Int. J. Radiat. Onkol. Biol. Phys.,* 2000, vol. 47, 979